(19) Europäisches Patentamt
European Patent Office
Office européen des brevets

(11) EP 0 647 629 B1

# (12) FASCICULE DE BREVET EUROPEEN

(45) Date de publication et mention de la délivrance du brevet:
**07.07.1999 Bulletin 1999/27**

(51) Int Cl.⁶: **C07D 231/14**, A61K 31/415, C07D 401/04, C07C 243/22, C07C 255/66

(21) Numéro de dépôt: **94402263.1**

(22) Date de dépôt: **10.10.1994**

## (54) 1-Naphtylpyrazole-3-carboxamides substitués actifs sur la neurotensine, leur préparation, les compositions pharmaceutiques en contenant

Neurotensin-aktive substituierte 1-Naphthylpyrazol-3-carboxamide, ihre Herstellung und sie enthaltende pharmazeutische Zusammensetzungen

Neurotensin active substituted 1-naphthylpyrazole-3-carboxamides, their preparation and pharmaceutical compositions containing them

(84) Etats contractants désignés:
**AT BE CH DE DK ES FR GB GR IE IT LI LU MC NL PT SE**
Etats d'extension désignés:
**LT SI**

(30) Priorité: **12.10.1993 FR 9312136**

(43) Date de publication de la demande:
**12.04.1995 Bulletin 1995/15**

(73) Titulaire: **SANOFI**
**75013 Paris (FR)**

(72) Inventeurs:
- **Labeeuw, Bernard**
  **F-34080 Montpellier (FR)**
- **Gully, Danielle**
  **F-31600 Saubens (FR)**
- **JeanJean, Francis**
  **F-34270 Valflaunes (FR)**
- **Molimard, Jean-Claude**
  **F-34980 Saint Gely du Fesc (FR)**
- **Boigegrain, Robert**
  **F-34820 Assas (FR)**

(74) Mandataire: **Gillard, Marie-Louise**
**Cabinet Beau de Loménie**
**158, rue de l'Université**
**75340 Paris Cédex 07 (FR)**

(56) Documents cités:
**EP-A- 0 418 845** **EP-A- 0 477 049**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 647 629 B1

## Description

La présente invention concerne de nouveaux 1-naphtylpyrazole-3-carboxamides substitués ayant une grande affinité pour le récepteur humain de la neurotensine, un procédé pour leur préparation et des compositions pharmaceutiques les contenant en tant que principes actifs.

Les premiers médicaments potentiels de synthèse, non peptidiques, capables de se lier aux récepteurs de la neurotensine ont été décrits dans EP-0477049. Il s'agit d'amides de l'acide pyrazole-3-carboxylique, diversement substitués avec des acides aminés, qui déplacent la neurotensine iodée de son récepteur, à des doses inférieures à la micromole, sur des membranes de cerveau de cobaye. Cette série a conduit à la mise au point d'un composé, l'acide 2-[1-(7-chloro-4-quinolyl)-5-(2,6-diméthoxyphényl)pyrazol-3-ylcarbonylamino]-adamantane-2-carboxylique, SR 48692, doté d'une activité antagoniste de la neurotensine puissante et sélective (D. Gully et al., Proc. Natl. Acad. Sci. USA, 1993, 90, 65-69).

La caractéristique de la série de produits décrite dans EP-0477049 est la présence, en position 1 du cycle pyrazole, notamment d'un groupe phényle, naphtyle, 4-quinolyle, substitués ou non substitués. Plus particulièrement, le SR 48692 possède, en position 1 du pyrazole, un groupe 7-chloro-4-quinolyle. Les produits décrits dans ce document ayant un groupe 1-naphtyle ou 4-chloro-1-naphtyle en position 1 du cycle pyrazole ont une affinité extrêmement élevée pour le récepteur de la neurotensine du cobaye car leur $IC_{50}$ est de l'ordre de 1 à 10 nanomoles, alors que leur affinité pour le récepteur humain est inférieure car leur $IC_{50}$ est de 10 à 100 nmoles.

Il a été maintenant trouvé qu'en substituant la position 4 du groupe naphtyle des composés 1-naphtylpyrazole-3-carboxamides avec des groupes particuliers, on augmente l'affinité pour le récepteur de la neurotensine et plus particulièrement, on augmente l'affinité pour le récepteur humain de la neurotensine.

Ainsi, la présente invention concerne, selon l'un de ses aspects, des nouveaux 1-naphtylpyrazole-3-carboxamides substitués de formule :

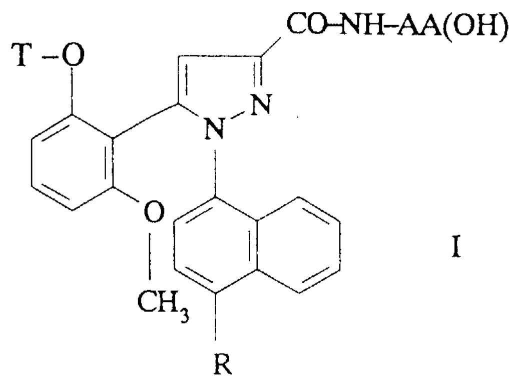

I

dans laquelle:

- R représente un groupe choisi parmi :
  $-CN$, $-C(NH_2) = N\text{-}OH$, $-C(NR_4R_5) = NR_6$, $-CONR_1R_2$, $-CON(R_7)(CH_2)_pNR_1R_2$, $-CON(R_7)(CH_2)_qCN$, $-CON(R_7)(CH_2)_qC(NR_{14}R_{15}) = N\text{-}R_{16}$, $-CH_2CN$, $-CH_2CONR_1R_2$, $-CH_2CON(R_7)(CH_2)_pNR_1R_2$, $-CH_2CON(R_7)(CH_2)_qCN$, $-CH_2COOR_7$, $-O(CH_2)_nNR_1R_2$, $-O(CH_2)_nCONR_1R_2$, $-O(CH_2)_nCOOR_7$, $-O(CH_2)_nSO_2NR_1R_2$, $-N(R7)COR_3$, $N(R_7)CO(CH_2)_nNR_1R_2$, $-N(R_7)CO(CH_2)_nNHCOR_3$, $-N(R_7)SO_2R_8$, $-N(R_7)CONR_9R_{10}$, $-CH_2N(R_7)COR_3$, $-CH_2N(R_7)SO_2R_8$, $-CH_2CH_2NR_{11}R_{12}$, $-CH_2CH_2N(R_7)COR_3$, $-CH_2CH_2N(R_7)SO_2R_8$, $-SO_2NR_1R_2$, $-SO_2N(R_7)(CH_2)_nNR_1R_2$, $-CH_2CON(R_7)(CH_2)_qC(NR_{14}R_{15}) = NR_{16}$ $-N(R_7)CO(CH_2)_qCN$, $-N(R_7)CO(CH_2)_qC(NR_{14}R_{15}) = NR_{16}$, $-SO_2N(R_7)(CH_2)_qCN$, $-SO_2N(R_7)(CH_2)_qC(NR_{14}R_{15}) = NR_{16}$ ;
- ou bien R, lié à l'atome de carbone en position 5 du radical naphtyle, forme un groupe :

$$-CON(R_{13})CO-$$

- p = 2 à 6 ;
- n = 1 à 6 ;
- q = 1 à 5 ;
- $R_1$ et $R_2$, représentent chacun indépendamment un hydrogène ou un $(C_1\text{-}C_4)$alkyle ; ou $R_1$ et $R_2$ ensemble avec l'atome d'azote auquel ils sont liés représentent un hétérocycle choisi parmi : la pyrrolidine, la pipéridine, la pipérazine substituée en position 4 par $R_7$, la morpholine ou la thiomorpholine;

- $R_3$ représente l'hydrogène ; un $(C_1\text{-}C_8)$alkyle ; un $(C_3\text{-}C_8)$cycloalkyle ; un phényle ; un pipéridyle ;
- $R_4$ et $R_5$ représentent chacun indépendamment un hydrogène ou un $(C_1\text{-}C_4)$alkyle ;
- $R_6$ représente un $(C_1\text{-}C_4)$alkyle ;
- $R_7$ représente un hydrogène ou un $(C_1\text{-}C_4)$alkyle ;
- $R_8$ représente un $(C_1\text{-}C_4)$alkyle ;
- $R_9$ et $R_{10}$ représentent chacun indépendamment un hydrogène ou un $(C_1\text{-}C_4)$alkyle ; $R_{10}$ peut de plus représenter un groupe $-(CH_2)_nNR_1R_2$ ;
- ou $R_9$ et $R_{10}$ ensemble avec l'atome d'azote auquel ils sont liés représentent un hétérocycle choisi parmi : la pyrrolidine, la pipéridine, la pipérazine substituée en position 4 par $R_7$, la morpholine ou la thiomorpholine ;
- $R_{11}$ et $R_{12}$ représentent chacun indépendamment un hydrogène ou un $(C_1\text{-}C_4)$alkyle ; ou $R_{11}$ et $R_{12}$ ensemble avec l'atome d'azote auquel ils sont liés représentent la pyrrolidine ou la pipéridine ;
- $R_{13}$ représente un hydrogène ; un groupe $-(CH_2)_nNR_1R_2$; un groupe $-NHCOR_3$ ;
- $R_{14}$ et $R_{15}$ représentent chacun indépendamment un hydrogène ou un $(C_1\text{-}C_4)$alkyle ;
- $R_{16}$ représente un hydrogène ; $R_{16}$ peut de plus représenter un $(C_1\text{-}C_4)$alkyle lorsque $R_{14}$ représente un hydrogène et $R_{15}$ représente un $(C_1\text{-}C_4)$alkyle ;
- ou $R_{14}$ et $R_{16}$ ensemble représentent un groupe éthylène ou un groupe triméthylène et $R_{15}$ représente un hydrogène ou un $(C_1\text{-}C_4)$alkyle ;
- T représente l'hydrogène; un $(C_1\text{-}C_4)$alkyle ; un allyle ; un $(C_3\text{-}C_8)$cycloalkyle ; un $(C_3\text{-}C_8)$cycloalkylméthyle ; un méthoxyéthyle ;
- le groupe -NH-AA(OH) représente le résidu d'un acide aminé :

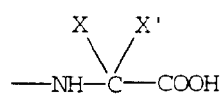

où X est l'hydrogène et X' est l'hydrogène, un $(C_1\text{-}C_5)$alkyle ou un radical carbocyclique non aromatique en $C_3\text{-}C_{15}$ ; ou bien X et X', ensemble avec l'atome de carbone auquel ils sont liés, forment un carbocycle non aromatique en $C_3\text{-}C_{15}$ ; et leurs sels.

Avantageusement, l'invention concerne les composés de formule I dans laquelle :

- R représente un groupe choisi parmi :
  $-CN$, $-C(NH_2) = N\text{-}OH$, $-CONR_1R_2$, $-CON(R_7)(CH_2)_pNR_1R_2$, $-O(CH_2)_nNR_1R_2$, $-O(CH_2)_nCONR_1R_2$, $-O(CH_2)_nCOOR_7$, $-O(CH_2)_nSO_2NR_1R_2$, $-NHCOR_3$, $-NHCO(CH_2)_nNR_1R_2$, $-CH_2CONR_1R_2$, $-CH_2CON(R_7)(CH_2)_pNR_1R_2$, $-CH_2\ COOR_7$, $-CH_2NHCOR_3$, $-SO_2NR_1R_2$, $-NHSO_2R_8$,
  $-SO_2N(R_7)(CH_2)_nNR_1R_2$,

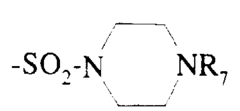

- p = 2 à 6 ;
- n = 1 à 6 ;
- $R_1$, et $R_2$, représentent chacun indépendamment un hydrogène ou un $(C_1\text{-}C_4)$alkyle ; ou $R_1$, et $R_2$ ensemble avec l'atome d'azote auquel ils sont liés représentent un hétérocycle choisi parmi pyrrolidine, pipéridine, pipérazine, morpholine ou thiomorpholine ;
- $R_3$ représente l'hydrogène ; un $(C_1\text{-}C_8)$alkyle, un $(C_3\text{-}C_8)$cycloalkyle, un phényle :
- $R_7$ représente un hydrogène ou un $(C_1\text{-}C_4)$alkyle ;
- $R_8$ représente un $(C_1\text{-}C_4)$alkyle ;
- T représente l'hydrogène, un alkyle en $C_1\text{-}C_4$, un allyle, un cycloalkyle en $C_3\text{-}C_8$, un $(C_3\text{-}C_8)$ cycloalkylméthyle, un méthoxyéthyle,
- le groupe -NH-AA(OH) représente le résidu d'un acide aminé :

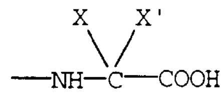

où X est l'hydrogène et X' est l'hydrogène, un alkyle en $C_1$-$C_5$ ou un radical carbocyclique non aromatique en $C_3$-$C_{15}$, ou bien X et X', ensemble avec l'atome de carbone auquel ils sont liés, forment un carbocycle non aromatique en $C_3$-$C_{15}$;

et leurs sels.

Selon la présente invention par alkyle en $C_1$-$C_4$ ou respectivement en $C_1$-$C_5$ ou respectivement en $C_1$-$C_8$, on entend un alkyle droit ou ramifié en $C_1$-$C_4$ ou respectivement en $C_1$-$C_5$ ou respectivement en $C_1$-$C_8$.

Les radicaux carbocycliques non aromatiques en $C_3$-$C_{15}$ comprennent les radicaux mono- ou poly-cycliques, condensés ou pontés, saturés ou insaturés, éventuellement terpéniques. Ces radicaux sont éventuellement mono- ou poly-substitués par un alkyle en $C_1$-$C_4$.

Les radicaux monocycliques incluent les cycloalkyles en $C_3$-$C_{15}$ par exemple cyclopropyle, cyclopentyle, cyclohexyle, cycloheptyle, cyclooctyle, cyclododécyle.

Dans le résidu de l'acide aminé ci-dessus lorsque X et X' ensemble avec l'atome de carbone auquel ils sont liés forment un carbocycle non aromatique en $C_3$-$C_{15}$, ledit carbocycle est tel que défini pour les radicaux correspondants ci-dessus.

Parmi les carbocycles non aromatiques polycycliques, l'adamantane est le préféré, le radical correspondant pouvant être le 1-adamantyle lorsque X est l'hydrogène ou le 2-adamantylidène lorsque X et X' ensemble avec l'atome de carbone auquel ils sont liés forment un carbocycle.

Parmi les carbocycles non aromatiques monocycliques, le cyclopentane et le cyclohexane sont particulièrement préférés.

Dans la formule I, R représente avantageusement un groupe choisi parmi : $-CONR_1R_2$, $-CH_2CONR_1R_2$ et $-O(CH_2)_nCONR_1R_2$, les substituants $R_1$ et $R_2$ étant de préférence l'hydrogène et n étant de préférence 1 ; $-N(R_7)COR_3$, le substituant $R_3$ étant notamment un ($C_1$-$C_8$)alkyle, de préférence un méthyle et $R_7$ étant de préférence l'hydrogène; $-CON(R_7)(CH_2)_pNR_1R_2$, le substituant $R_7$ étant de préférence un hydrogène ou un méthyle, $R_1$ et $R_2$ étant de préférence tous les deux méthyle et p étant de préférence 2, 3 ou 4 ; $-SO_2N(R_7)(CH_2)_nNR_1R_2$, le substituant $R_7$ étant de préférence hydrogène ou méthyle, $R_1$ et $R_2$ étant de préférence tous les deux méthyle et n étant de préférence 2,3 ou 4 ; et $-CON(R_7)(CH_2)_qC(NR_{14}R_{15}) = NR_{16}$, le substituant $R_7$ étant de préférence un hydrogène ou un méthyle, $R_{14}$ et $R_{16}$ étant de préférence tous les deux méthyle, $R_{15}$ étant de préférence un hydrogène et q étant de préférence 2 ou 3. Encore plus préférés, sont les composés de formule I dans lesquels R est tel que défini ci-dessus et dans laquelle en même temps T représente l'hydrogène ou un groupe méthyle ou cyclopropylméthyle. On préfère particulièrement les composés de formule I dans laquelle R et T sont tels que définis ci-dessus et dans laquelle en même temps le groupe AA(OH) représente un radical 2-carboxyadamant-2-yle ou $\alpha$-carboxycyclohexylméthyle, ces deux radicaux représentant, avec le groupe NH adjacent, les résidus N-terminaux des acides 2-aminoadamantane-2-carboxylique et a-aminocyclohexaneacétique (cyclohexylglycine), respectivement.

Les 1-naphtylpyrazole-3-carboxamides substitués préférés selon la présente invention sont ceux de formule I dans laquelle :

- R représente un groupe aminocarbonyle ; aminocarbonylméthyle ; acétamido ; N-(3-N',N'-diméthylaminopropyl) aminosulfonyle ; N-méthyl-N-(3-N',N'-diméthylaminopropyl)aminosulfonyle ; carbamoylméthyloxy ; N-(3-N',N'-diméthylaminopropyl)aminocarbonyle ; N-(2-N',N'-diméthylaminoéthyl)aminocarbonyle; N-méthyl-N-(3-N',N'-diméthylaminopropyl)aminocarbonyle ; N-méthyl-N-(2-N',N'-diméthylaminoéthyl)aminocarbonyle; N-méthyl-N-[2-$N^1$-méthyl-$N^2$-méthylamidino)éthyl]carbamoyle;
- T représente un groupe méthyle ou cyclopropylméthyle ; et
- le groupe -NH-AA-(OH) représente le résidu de l'acide 2-aminoadamantane-2-carboxylique ou (S)$\alpha$-aminocyclohexaneacétique ;

et leurs sels.

De façon toute particulière, on préfère les composés de formule :

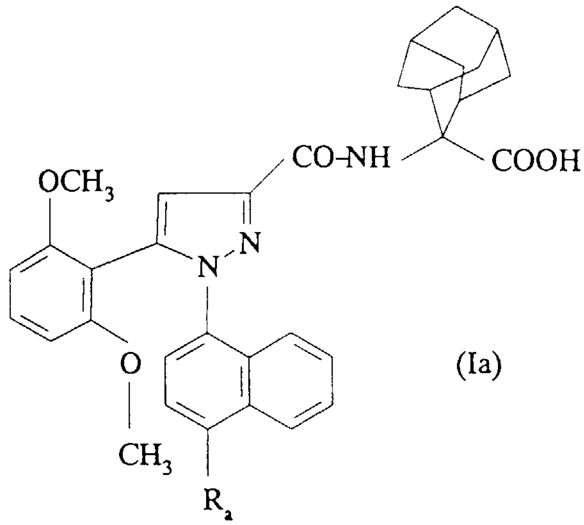

(Ia)

dans laquelle :

- $R_a$ représente un groupe N-(3-N',N'-diméthylaminopropyl)aminocarbonyle ou N-(2-N',N'-diméthylaminoéthyl)aminocarbonyle;

et leurs sels.

Les sels des composés de l'invention sont ceux avec des métaux alcalins, de préférence le sodium ou le potassium, des métaux alcalino-terreux, de préférence le calcium et avec des bases organiques comme la diéthylamine, la trométhamine, la méglumine (N-méthyl-D-glucamine), la lysine, l'arginine, l'histidine ou la diéthanolamine.

Les sels des composés de formule I selon la présente invention comprennent également ceux avec des acides minéraux ou organiques qui permettent une séparation ou une cristallisation convenable des composés de formule I, tels que l'acide picrique, l'acide oxalique ou un acide optiquement actif, par exemple un acide mandélique ou un acide camphosulfonique, et ceux qui forment des sels pharmaceutiquement acceptables tels que le chlorhydrate, l'hydrogénosulfate, le dihydrogénophosphate, le méthanesulfonate, le maléate, le fumarate, le 2-naphtalène sulfonate, l'iséthionate.

Lorsque les composés I incluent un carbone asymétrique, les énantiomères font partie de l'invention.

Lorsque le groupe -NH(AA)OH représente le résidu d'un aminoacide cycloaliphatique, les groupes amino ou aminométhyle peuvent être en position endo ou en position exo par rapport au système cyclique ; dans les deux cas, les composés de formule I font partie de l'invention.

Selon un autre de ses aspects, la présente invention concerne un procédé pour la préparation des 1-naphtylpyrazole-3-carboxamides substitués de formule I et de leurs sels, caractérisé en ce que :

1) l'on traite un dérivé fonctionnel d'un acide 1-naphtylpyrazole-3-carboxylique de formule :

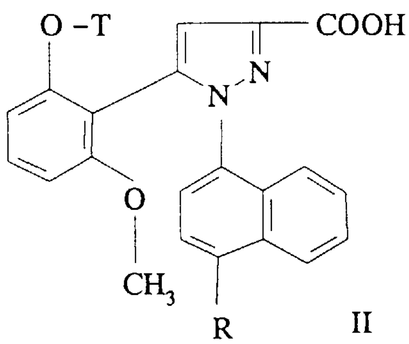

II

ou

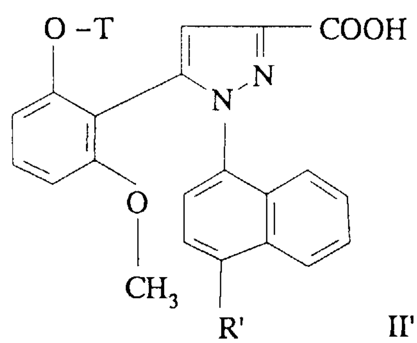

II'

dans laquelle T et R ont les significations données ci-dessus pour le composé de formule I et R' représente un

précurseur de R choisi parmi les groupes nitro, amino, hydroxy, sulfo, chlorosulfonyle, carboxy, avec un acide aminé, éventuellement protégé par les groupements protecteurs habituels en synthèse peptidique, de formule:

H-HN-AA(OH) III

dans laquelle -NH-AA(OH) est tel que défini ci-dessus pour le composé de formule I ;
2) le cas échéant, on soumet le composé ainsi obtenu de formule:

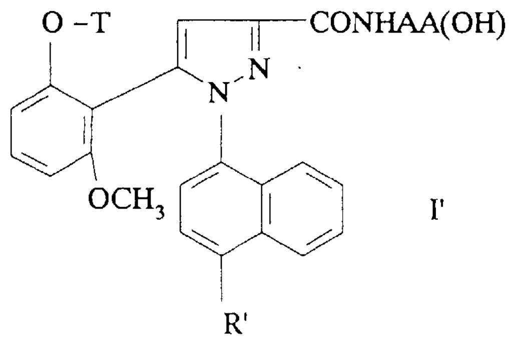

I'

à un traitement ultérieur approprié pour transformer le substituant R', précurseur de R, en le substituant R ;
3) éventuellement, on déprotège le composé ainsi obtenu à l'étape 1) ou à l'étape 2) pour conduire à l'acide libre de formule I correspondant ;
4) le cas échéant, on prépare un sel du composé I ainsi obtenu.

Comme dérivé fonctionnel de l'acide 1-naphtylpyrazole-3-carboxylique substitué de formule II ou II', on peut utiliser le chlorure d'acide, l'anhydride, un anhydride mixte, un ester alkylique en $C_1$-$C_4$, un ester activé, par exemple l'ester de p-nitrophényle, ou l'acide libre opportunément activé, par exemple, avec le N,N-dicyclohexylcarbodiimide ou avec l'hexafluorophosphate de benzotriazol-1-yloxytris-(diméthylamino)-phosphonium (BOP).

Les acides aminés de formule III peuvent être utilisés soit tels quels, soit après protection préalable du groupe carboxyle par des groupements protecteurs habituels en synthèse peptidique comme décrit par exemple dans Protective Groups in Organic Chemistry, Ed. J.F.W. McOmie, Plenum Press, 1973, page 183, ou dans Protective Groups in Organic Synthesis, II Ed. J.F.W. Greene et P.G.M. Wuts, John Wiley & Sons, 1991, page 224.

Pour cette protection, le groupe carboxylique de l'acide aminé III peut être tout simplement estérifié, par exemple sous forme d'ester méthylique, isobutylique ou tert-butylique, le groupe estérifiant étant ensuite éliminé par saponification ou réduction. La protection par estérification ne peut être utilisée que lorsque le groupe R ou R' ne contient pas, lui aussi, soit un groupe ester qui doit être conservé, comme au cas où, par exemple, R représenterait un groupe $O(CH_2)_nCOOR_7$ ou $CH_2COOR_7$ avec $R_7$ = alkyle, soit, de toute façon, un groupe susceptible d'être affecté pendant le déblocage du groupe ester. La protection du groupe carboxylique de l'acide aminé III peut également être effectuée par silylation, par exemple avec le bis(triméthylsilyl)acétamide, ladite protection pouvant être effectuée *in situ*. L'ester silylique du composé I est ensuite facilement décomposé pendant l'isolement du produit final par simple acidification.

Ainsi dans l'étape 1) du procédé on peut faire réagir le chlorure d'un acide 1-naphtylpyrazole-3-carboxylique, obtenu par réaction du chlorure de thionyle ou du chlorure d'oxalyle sur un acide de formule II ou II', avec un acide aminé de formule III, dans un solvant tel que l'acétonitrile, le THF, le DMF ou le DCM, sous une atmosphère inerte, à la température ambiante, pendant un temps compris entre quelques heures et quelques jours, en présence d'une base telle que la pyridine, la soude ou la triéthylamine.

Une variante de l'étape 1) consiste à préparer le chlorure d'acide ou l'anhydride mixte d'un acide 1-naphtylpyrazole-3-carboxylique par réaction du chloroformiate d'isobutyle ou d'éthyle avec un acide de formule II ou II', en présence d'une base telle que la triéthylamine, et à le faire réagir avec un dérivé de N,O-bis-triméthylsilyle d'un acide aminé de formule III, obtenu par réaction du bis(triméthylsilyl)acétamide ou de la 1,3-bis(triméthylsilyl)urée avec un acide aminé de formule III, dans des solvants tels que l'acétonitrile, le DCM, sous atmosphère inerte, à la température ambiante, pendant un temps compris entre 1 jour et quelques jours.

Une autre variante au mode opératoire de l'étape 1) consiste à faire réagir l'anhydride mixte d'un acide 1-naphtylpyrazole-3-carboxylique de formule II ou II' avec un acide aminé de formule III, dans un solvant tel que le

DCM, sous une atmosphère inerte, à la température ambiante, pendant un temps compris entre 1 jour et quelques jours, en présence d'une base telle que la triéthylamine.

Lorsque le composé de formule I présente une fonction basique et est obtenu sous forme de base libre, la salification est effectuée par traitement avec l'acide choisi, dans un solvant organique. Par traitement de la base libre dissoute par exemple dans un alcool, tel que le méthanol, avec une solution de l'acide choisi, dans le même solvant ou dans un autre solvant, tel que l'éther diéthylique, on obtient le sel correspondant qui est isolé selon les techniques classiques. Ainsi, on prépare par exemple le chlorhydrate, le bromhydrate, le sulfate, l'hydrogénosulfate, le dihydrogénophosphate, le méthanesulfonate, le méthylsulfate, l'oxalate, le maléate, le fumarate, le 2-naphtalènesulfonate, l'iséthionate.

Lorsque le composé de formule I présente une fonction basique et est isolé sous forme d'un de ses sels, par exemple le chlorhydrate ou l'oxalate, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Lorsque le produit de formule I est obtenu sous forme acide, il peut être transformé en un sel métallique, notamment alcalin, tel que le sel de sodium, ou alcalino-terreux, tel que le sel de calcium, selon les procédés classiques.

Les acides 1-naphtylpyrazole-3-carboxyliques substitués de formule :

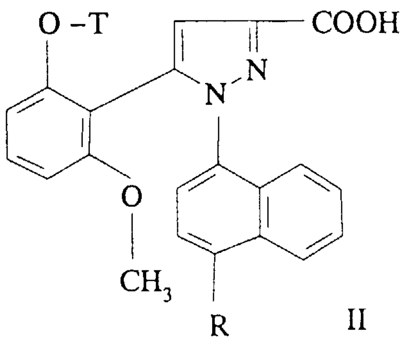
II

ou

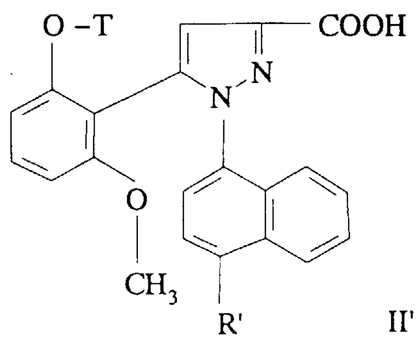
II'

dans laquelle T et R ont les définitions données ci-dessus pour les composés I et R' représente un précurseur de R choisi parmi les groupes nitro, amino, hydroxy, sulfo, chlorosulfonyle, carboxy, ainsi que leurs dérivés fonctionnels de la fonction acide sont des intermédiaires clés dans la préparation des composés de formule I. Lorsque R' est autre que carboxy, les composés de formules II et II' sont nouveaux et ils constituent un aspect ultérieur de la présente invention.

Les acides de formules II et II', les chlorures des acides de formules II et II', les esters alkyliques en $C_1$-$C_4$ des acides de formules II et II', qui peuvent être également des précurseurs desdits acides (notamment les esters méthylique, éthylique et t-butylique) et l'anhydride mixte des acides de formules II et II' avec le chloroformiate d'isobutyle ou d'éthyle sont des produits intermédiaires particulièrement préférés.

Le procédé de préparation des composés II ou II' via les esters IIa ou II'a est représenté par le schéma suivant :

SCHEMA 1

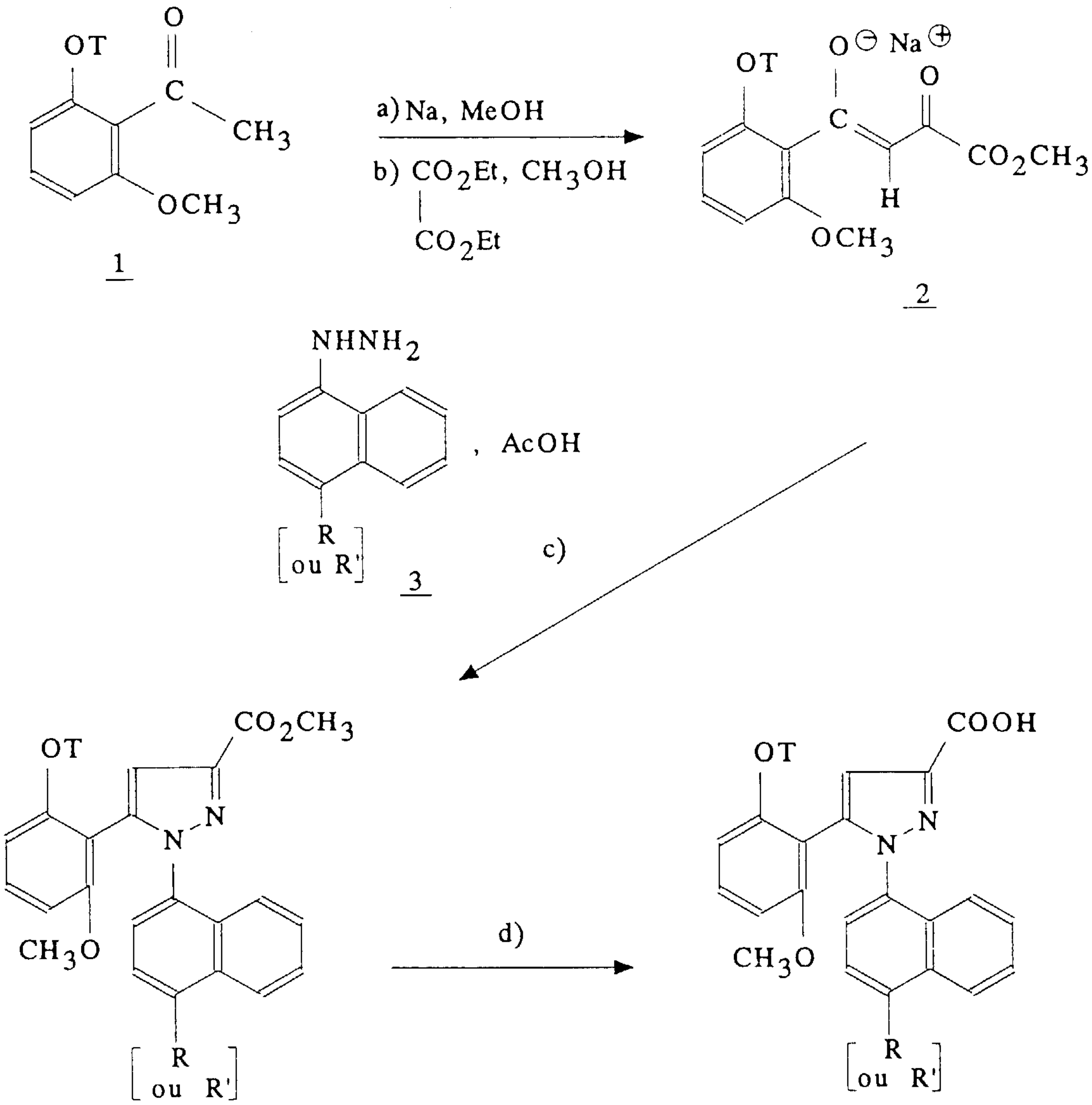


1 2 3

IIa ou IIa' II ou II'

Dans la première étape a) on fait réagir une base forte, telle que le méthylate de sodium sur une cétone de formule 1 dans laquelle T est tel que défini précédemment, puis on fait agir (étape b)) une quantité équimolaire d'oxalate d'éthyle dans un alcanol comme par exemple le méthanol, selon L. Claisen, Ber., 1909, 42, 59. Après précipitation dans un éther, tel que l'éther éthylique ou l'éther isopropylique, les énolates de sodium 2 sont séparés par filtration. On peut également préparer un énolate de lithium selon W.V. Murray et al., J. Heterocyclic Chem., 1989, 26, 1389.

L'énolate métallique 2 ainsi préparé et un excès de dérivé de naphtylhydrazine 3 ou d'un sel de celui-ci sont alors chauffés au reflux de l'acide acétique (étape c)) pour obtenir les esters IIa ou II'a.

Par saponification des esters IIa ou II'a, par action d'un agent alcalin comme par exemple l'hydroxyde de potassium, l'hydroxyde de sodium ou l'hydroxyde de lithium puis acidification, on obtient les acides II ou II' (étape d)).

De façon particulière, on prépare un composé de formule II dans laquelle R, lié à l'atome de carbone en position 5 du radical naphtyle forme un groupe -CO-N($R_{13}$)CO- avec $R_{13}$ = -$NHCOR_3$, en utilisant le procédé illustré dans le schéma 2 suivant :

SCHEMA 2

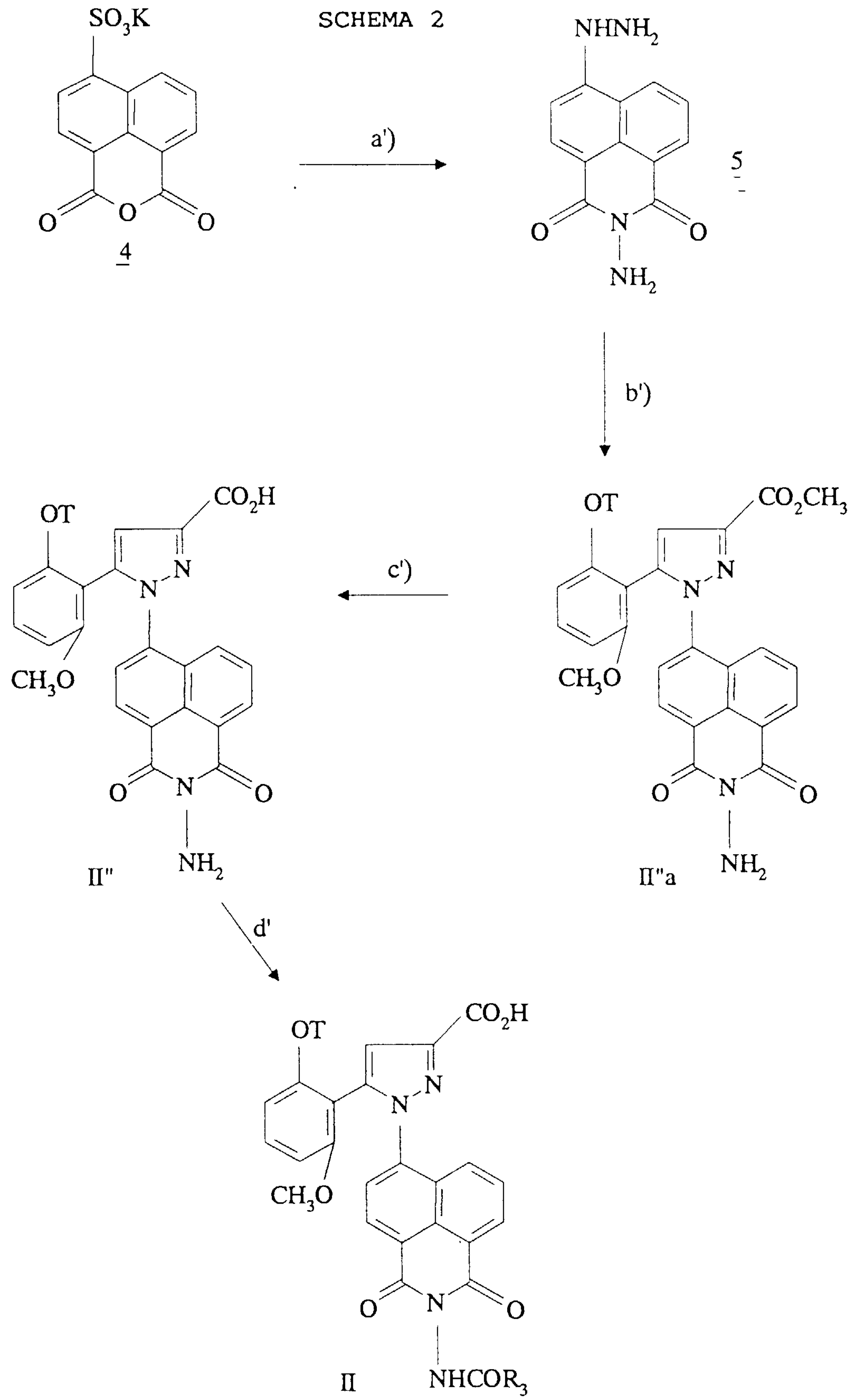


4

5

II"

II"a

II

Dans la première étape a') on fait réagir le sel de potassium de l'anhydride 4-sulfo-1,8-naphtalique avec l'hydrazine monohydrate en chauffant à la température de 120°C pendant 36 heures. Après précipitation dans l'eau,

le composé 5 est séparé par filtration. La réaction de l'hydrazine 5 avec l'énolate métallique 2 (étape b') selon le procédé décrit dans l'étape c) du SCHEMA 1 permet d'obtenir les esters II"a. Par saponification des esters II"a (étape c') selon le procédé décrit dans l'étape d) du SCHEMA 1, on obtient les acides II". Par réaction des acides II" avec des chlorures d'acides ou des anhydrides appropriés on obtient les composés II attendus.

Parmi les composés de formule 3 certains sont nouveaux et constituent un objet ultérieur de la présente invention.

Ainsi les composés de formule :

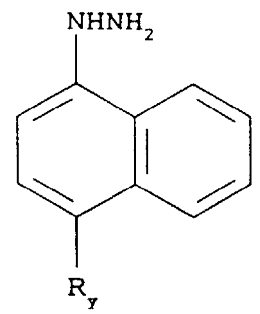

3'

dans laquelle :

$R_y$ représente un groupe cyano ou carboxyméthyle ; et leurs sels sont nouveaux et font partie de l'invention.

Les dérivés de la naphtylhydrazine (3) portant un substituant R ou un substituant R' peuvent être préparés par diazotation de la naphtylamine correspondante en présence du nitrite de sodium, puis réduction du sel de diazonium par exemple par action du chlorure stanneux. Les naphtylamines substituées sont connues ou préparées par des méthodes connues.

Les dérivés de la naphtylhydrazine dans lesquels R, relié avec l'atome de carbone en position 5 du radical naphtyle, forme un groupe $-CON(R_{13})CO-$ avec $R_{13}$ = H ou $-(CH_2)_nNR_1R_2$, se préparent en utilisant le procédé illustré dans le SCHEMA 3 suivant:

SCHEMA 3

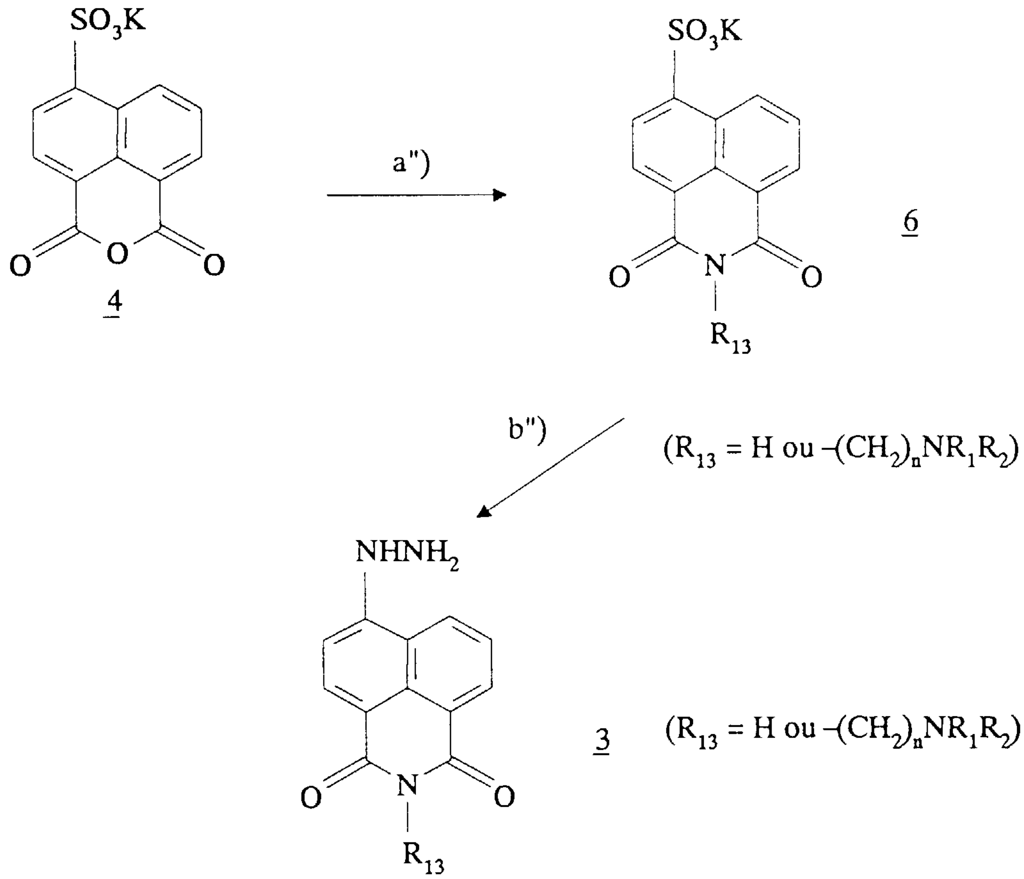


4 6 3

La réaction du composé 4 avec une amine de formule $H_2NR_{13}$ dans laquelle $R_{13}$ est l'hydrogène ou un groupe $-(CH_2)_nNR_1R_2$ permet d'obtenir les composés 6 (étape a"). Par chauffage des composés 6 avec l'hydrazine monohydrate en solution aqueuse on obtient les hydrazines 3 attendues (étape b").

La transformation d'un composé de formule I' ou respectivement de formule II' ou de formule II'a dans lequel le groupe naphtyle est substitué par R' en un composé de formule I ou respectivement de formule II ou de formule IIa dans lequel le groupe naphtyle est substitué par R est effectuée par des méthodes classiques bien connues de l'homme de l'art.

Par exemple lorsque R' = $SO_3H$ on prépare un composé II'a dans lequel R' = $SO_2Cl$ puis on le transforme en un autre composé IIa dans lequel R est un groupe aminosulfonyle, éventuellement substitué, par action d'une amine appropriée de formule $NHR_1R_2$, $HN(R_7)$ $(CH_2)_nNR_1R_2$ ou $HN(R_7)(CH_2)_qCN$ dans lesquelles $R_1$, $R_2$, $R_7$, n et q ont les définitions données ci-dessus pour le composé de formule I.

Les composés de formule IIa dans lesquels R représente un groupe carboxyméthyle permettent de préparer des composés de formule IIa dans lesquels R représente un groupe $-CH_2CONR_1R_2$ ou respectivement un groupe $-CH_2CON(R_7)(CH_2)_pNR_1R_2$ ou un groupe $-CH_2CON(R_7)(CH_2)_qCN$, par réaction du chlorure d'acide, préparé intermédiairement, avec une amine $HNR_1R_2$ ou respectivement avec une diamine $HN(R_7)(CH_2)_pNR_1R_2$ ou avec un composé aminé $HN(R_7)(CH_2)_qCN$.

Par réaction des composés de formule I dans lesquels R représente un groupe $-CH_2CONH_2$, avec du péroxyde de sodium, on obtient les composés de formule I dans lesquels R représente un groupe carboxyméthyle. Lors de la préparation des composés de formule I dans lesquels R représente un groupe $-CH_2CONH_2$ à partir des composés de formule II dans lesquels R représente un groupe $CH_2CONH_2$, on obtient également les composés de formule I dans lesquels R représente un groupe $-CH_2CN$ que l'on sépare par chromatographie. Par réduction des composés de formule I dans lesquels R représente un groupe $-CH_2CN$, par exemple par hydrogénation en présence d'un catalyseur tel que le nickel de Raney®, on obtient les composés de formule I dans lesquels R représente un groupe $-CH_2CH_2NH_2$. Ces derniers composés permettent de préparer des composés de formule I dans lesquels R représente un groupe $-CH_2CH_2NR_1R_2$, un groupe $-CH_2CH_2N(R_7)COR_3$ ou un groupe $-CH_2CH_2N(R_7)SO_2R_8$ par des méthodes connues de

l'homme de l'art.

Les composés de formule II'a, ou respectivement les composés de formule II' ou les composés de formule I', dans lesquels R' représente un groupe nitro peuvent être transformés en composés de formule II'a, ou respectivement de formule II' ou de formule I', dans lesquels R' est un groupe amino; puis par des méthodes connues, on prépare les composés de formule IIa ou respectivement de formule II ou de formule I, dans lesquels R représente un groupe $-N(R_7)COR_3$, un groupe $-N(R_7)CO(CH_2)_nNR_1R_2$, un groupe $-N(R_7)CO(CH_2)_nNHCOR_3$, un groupe $-N(R_7)SO_2R_8$, un groupe $-N(R_7)CONR_9R_{10}$ ou un groupe $-N(R_7)CO(CH_2)_qCN$.

Les composés de formule II'a, ou respectivement, de formule II', ou de formule I', dans lesquels R' est un groupe amino permettent également de préparer des composés de formule II'a ou respectivement de formule II' ou de formule I', dans lesquels R' représente un groupe hydroxyle ; puis, par des méthodes connues, on prépare les composés de formule IIa ou respectivement de formule II ou de formule I dans lesquels R représente un groupe $-O(CH_2)_nNR_1R_2$, un groupe $O(CH_2)_nCONR_1R_2$, un groupe $-O(CH_2)_nCOOR_7$ ou un groupe $-O(CH_2)_nSO_2NR_1R_2$.

Les composés de formule I dans lesquels R est un groupe cyano permettent de préparer, par réaction avec du péroxyde d'hydrogène en présence d'une base telle que la soude, les composés de formule I dans lesquels R est un groupe carbamoyle. De la même façon on obtient les composés de formule II dans lesquels R est un groupe carbamoyle à partir des composés de formule IIa dans lesquels R est un groupe cyano.

Les composés de formule I dans lesquels R est un groupe cyano permettent également de préparer, par réaction avec l'hydroxylamine en présence d'une base telle que le carbonate de potassium, les composés de formule I dans lesquels R représente un groupe $-C(NH_2) = NOH$.

Par réduction des composés de formule IIa ou respectivement de formule II ou de formule I dans lesquels R représente un groupe cyano, par exemple par hydrogénation en présence d'un catalyseur tel que l'oxyde de platine, puis réaction avec un chlorure d'acide ou un anhydride approprié ou respectivement avec un chlorure de sulfonyle, on obtient les composés de formule IIa ou respectivement de formule II ou de formule I dans lesquels R représente un groupe $-CH_2NHCOR_3$ ou respectivement $-CH_2NHSO_2R_8$. De même on obtient les composés de formule IIa ou respectivement de formule II ou de formule I dans lesquels R représente un groupe $-CH_2N(R_7)COR_3$ ou un groupe $-CH_2N(R_7)SO_2R_8$ avec $R_7$ autre que l'hydrogène, en effectuant une réaction d'alkylation sur l'amine obtenue intermédiairement.

Les composés de formule II'a dans lesquels R' représente un groupe carboxy permettent de préparer les composés de formule IIa dans lesquels R représente un groupe $-CONR_1R_2$ ou respectivement un groupe $-CON(R_7)(CH_2)_pNR_1R_2$ ou un groupe $-CON(R_7)(CH_2)_qCN$ par réaction du chlorure d'acide, préparé intermédiairement, avec une amine $HNR_1R_2$ ou respectivement avec une amine $HN(R_7)(CH_2)_pNR_1R_2$ ou avec une amine $HN(R_7)(CH_2)_qCN$.

La réaction des composés de formule I dans lesquels R représente un groupe $-CON(R_7)(CH_2)_qCN$ ou un groupe $-CH_2CON(R_7)(CH_2)_qCN$ ou un groupe $-N(R_7)CO(CH_2)_qCN$ ou un groupe $-SO_2N(R_7)(CH_2)_qCN$ avec de l'acide chlorhydrique en solution alcoolique, permet d'obtenir intermédiairement l'imidate correspondant.

Si l'on fait réagir l'imidate avec une quantité équimolaire d'amine $HNR_{14}R_{15}$ on obtient les composés de formule I dans lesquels R représente :

un groupe $-CON(R_7)(CH_2)_qC(NR_{14}R_{15}) = NR_{16}$ ou
un groupe $-CH_2CON(R_7)(CH_2)_qC(NR_{14}R_{15})=NR_{16}$, ou
un groupe $-N(R_7)CO(CH_2)_qC(NR_{14}R_{15})=NR_{16}$ ou
un groupe $-SO_2N(R_7)(CH_2)_qC(NR_{14}R_{15})=NR_{16}$ avec $R_{16}$ = H.

Si l'on fait réagir l'imidate avec un excès d'amine $HNR_{14}R_{15}$ on obtient les composés de formule I dans lesquels R représente :

un groupe $-CON(R_7)(CH_2)_qC(NR_{14}R_{15}) = N-R_{16}$ ou
un groupe $-CH_2CON(R_7)(CH_2)_qC(NR_{14}R_{15})=NR_{16}$, ou
un groupe $-N(R_7)CO(CH_2)_qC(NR_{14}R_{15})=NR_{16}$ ou
un groupe $-SO_2N(R_7)(CH_2)_qC(NR_{14}R_{15})=NR_{16}$ avec $R_{16}$ autre que l'hydrogène.

Si l'on fait réagir l'imidate avec de l'éthylènediamine non substitué ou N-substitué par un $(C_1-C_4)$alkyle ou avec du 1,3-diaminopropane non substitué ou N-substitué par un $(C_1-C_4)$alkyle, on obtient les composés de formule I dans lesquels R représente :

un groupe $-CON(R_7)(CH_2)_qC(NR_{14}R_{15})=N-R_{16}$ ou
un groupe $-CH_2CON(R_7)(CH_2)_qC(NR_{14}R_{15})=NR_{16}$ ou
un groupe $-N(R_7)CO(CH_2)_qC(NR_{14}R_{15})=NR_{16}$ ou
un groupe $-SO_2N(R_7)(CH_2)_qC(NR_{14}R_{15})=NR_{16}$ dans lesquels $R_{14}$ et $R_{16}$ ensemble constituent un groupe éthylène

ou triméthylène et $R_{15}$ représente un hydrogène ou un $(C_1-C_4)$alkyle.

Les composés de formule I dans lesquels R représente un groupe carbamoyle monosubstitué sur l'azote, permettent de préparer, par réaction avec du pentachlorure de phosphore puis réaction avec une amine $HNR_4R_5$, les composés de formule I dans lesquels R représente un groupe $-C(NR_4R_5) = N-R_6$.

Les acides aminés de formule III incluent par exemple la glycine, l'alanine, la leucine, la norleucine, l'isoleucine, la valine, la 1-adamantylglycine, la 2-adamantylglycine, la cyclopropylglycine, la cyclopentylglycine, la cyclohexylglycine, la cycloheptylglycine, l'acide 1-aminocyclopropanecarboxylique, l'acide 1-aminocyclobutanecarboxylique, l'acide 1-aminocyclopentanecarboxylique, l'acide 1-aminocyclohexanecarboxylique, l'acide 1-aminocycloheptanecarboxylique, l'acide 1-amino-4-méthylcyclohexanecarboxylique, l'acide 2-aminoadamantane-2-carboxylique, l'acide 2-aminobicyclo[3.2.1]octane-2-carboxylique, l'acide 9-aminobicyclo[3.3.1]nonane-9-carboxylique, l'acide 2-aminobicyclo[2.2.1]heptane-2-carboxylique (ou 2-aminonorbornane-2-carboxylique).

Les acides aminés de formule III sont des produits commerciaux ou peuvent être très facilement préparés selon les méthodes classiques. Notamment les aminoacides (III) non commerciaux sont préparés selon la synthèse de Strecker, Ann, 1850, 75, 27 ou selon la synthèse de H.T. Bucherer et al., J. Pract. Chem., 1934, 141, 5, suivie d'une hydrolyse pour conduire aux aminoacides ; par exemple, l'acide 2-aminoadamantane-2-carboxylique est préparé selon H.T. Nagasawa et al., J. Med. Chem., 1973, 16, (7), 823.

Les acides α-amino-1-adamantylacétique et α-amino-2-adamantylacétique sont préparés selon B. Gaspert et al., Croatica Chemica Acta, 1976, 48 (2), 169-178.

L'acide 2-aminonorbornane-2-carboxylique est préparé selon H.S. Tager et al., J. Am. Chem. Soc., 1972, 94, 968.

Les acides α-aminocycloalkyl carboxyliques sont préparés selon J.W. Tsang et al., J. Med. Chem., 1984, 27, 1663.

Les cyclopentylglycines R et S sont préparées selon la demande de brevet européen EP 477049.

Les cyclohexylglycines R et S sont préparées selon Rudman et al., J. Am. Chem. Soc., 1952, 74, 551.

On peut également préparer les cyclohexylglycines R et S par hydrogénation catalytique des phénylglycines R et S.

On peut aussi préparer les acides α-aminocycloalkylcarboxyliques de configuration R ou S par hydrolyse enzymatique, stéréospécifique, des dérivés N-acétylés racémiques correspondant, selon J. Hill et al., J. Org. Chem., 1965, 1321.

Les composés de formule I et leurs sels possèdent une très grande affinité pour les récepteurs humains de la neurotensine, dans les tests décrits dans la publication de D. Gully et al. citée ci-dessus. Plus particulièrement, par rapport aux dérivés 1-naphtyl et 4-chloro-1-naphtyl décrits dans EP 0477049, qui ont une $IC_{50}$ égale ou supérieure à 100 nM, les composés de l'invention possèdent une $IC_{50}$ nettement inférieure, allant de 1 nM jusqu'à 50 nM. Particulièrement intéressants sont les produits de formule I dans laquelle T est méthyle et R est aminocarbonyle ; aminocarbonylméthyle ; acétamido ; N-méthyl-N-(3-N',N'-diméthylaminopropyl)aminosulfonyle ; carbamoylméthyloxy ; N-(3-N',N'-diméthylaminopropyl)aminocarbonyle ; N-(2-N', N'-diméthylaminoéthyl)aminocarbonyle. Ces composés sont donc même plus actifs que le SR 48692, ce qui est inattendu si on se réfère à l'activité des 1-naphtylpyrazole-3-carboxamides décrits dans EP 0477049.

Les composés de formule I et leurs sels ont été étudiés *in vivo.* En opérant selon la technique décrite par M. Poncelet *et al.* dans Naunyn Schmiedberg's Arch. Pharmacol., 1994, 60, 349-357, on observe que les composés selon l'invention, administrés par voie orale, antagonisent les rotations contralatérales induites par injection intrastriatale unilatérale de neurotensine chez la souris.

Par ailleurs, en opérant selon la technique décrite par D. Nisato *et al.* dans Life Sciences, 1994, 54, 7, 95-100, on constate que les composés selon l'invention, administrés par voie intraveineuse, inhibent l'augmentation de la pression artérielle induite par injection intra-veineuse de neurotensine chez le cobaye anesthésié.

Les composés décrits dans le brevet EP 0477 049 présentent sur ces tests une activité inférieure à celle des composés selon la présente invention.

Les composés de formule I et leurs sels pharmaceutiquement acceptables sont peu toxiques ; notamment, leur toxicité aiguë est compatible avec leur utilisation comme médicament. Pour une telle utilisation, on administre aux mammifères une quantité efficace d'un composé de formule I, ou d'un de ses sels pharmaceutiquement acceptables, pour le traitement des pathologies neurotensine-dépendantes. Ainsi lesdits composés peuvent être utilisés pour le traitement de désordres neuropsychiatriques en particulier ceux qui sont liés à un dysfonctionnement des systèmes dopaminergiques, par exemple les psychoses, plus particulièrement la schizophrénie et les maladies du mouvement comme la maladie de Parkinson, (D.R. Handrich et al., Brain Research, 1982, 231, 216-221 et C.B. Nemeroff, Biological Psychiatry, 1980, 15 (2), 283-302). Ils peuvent être utiles pour diagnostiquer et/ou traiter les maladies cancéreuses, par exemple les méningiomes humains non accessibles chirurgicalement (P. Mailleux, Peptides, 1990, 11, 1245-1253), les cancers de la prostate (I. Sehgal *et al.*, Proc. Nat. Acad. Sci., 1994, 91, 4673-4677), les cancers du poumon à

petites cellules (T. Sethi *et al.*, Cancer Res., 1991, 51, 3621-3623). Ils peuvent être utilisés dans le traitement de désordres gastrointestinaux moteurs, sécrétoires, ulcéreux et/ou tumoraux (Revue de A. Shulkes dans "Gut Peptides : Biochemistry and Physiology, Ed. J. Waish and G.J. Dockray, 1994"). Ainsi les composés I selon l'invention peuvent être utiles dans le traitement d'affections telles que : syndrome du colon irritable, diarrhée, colites, ulcères, tumeurs du tractus gastro-intestinal, dyspepsie, pancréatite, oesophagite. Les composés selon l'invention peuvent être indiqués dans le cas de troubles cardiovasculaires, et également dans le cas de pathologies associées à une libération d'histamine telles que les processus inflammatoires (D.E. Cochrane *et al.*, Faseb J., 1994, 8, 7, 1195). Les composés de la présente invention peuvent également présenter un intérêt dans l'analgésie en agissant sur les effets de la morphine (M.O. Urban, J. Pharm. Exp. Ther., 1993, 265, 2, 580-586).

Ainsi, la présente invention a pour objet, selon un autre de ses aspects, des compositions pharmaceutiques contenant, comme principes actifs, les composés de formule I ou leurs sels éventuels pharmaceutiquement acceptables.

Dans les compositions pharmaceutiques de la présente invention pour l'administration orale, sublinguale, sous-cutanée, intramusculaire, intraveineuse, transdermique ou rectale, les principes actifs peuvent être administrés, sous formes unitaires d'administration, en mélange ou avec des supports pharmaceutiques classiques aux animaux et aux être humains. Les formes unitaires d'administration appropriées comprennent les formes par voie orale, telles que les comprimés, les gélules, les poudres, les granules et les solutions ou suspensions orales, les formes d'administration sublinguale et buccale, les formes d'administration sous-cutanée, intramusculaire ou intraveineuse et les formes d'administration rectale.

Afin d'obtenir l'effet désiré, la dose de principe actif peut varier entre 0,5 et 1000 mg par jour, de préférence entre 2 et 500 mg.

Chaque dose unitaire peut contenir de 0,5 à 250 mg de principe actif, de préférence de 1 à 125 mg, en combinaison avec un support pharmaceutique. Cette dose unitaire peut être administrée 1 à 4 fois par jour.

Lorsqu'on prépare une composition solide sous forme de comprimés, on mélange le principe actif avec un véhicule pharmaceutique, tel que la gélatine, l'amidon, le lactose, le stéarate de magnésium, le talc, la gomme arabique, ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une administation rectale on recourt à des suppositoires qui sont préparés avec des liants fondant à la température rectale, par exemple du beurre de cacao ou des polyéthylèneglycols.

Pour une administration parentérale, on utilise des suspensions aqueuses, des solution salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Pour améliorer la solubilité des produits de l'invention, les composés de formule I ou leurs sels pharmaceutiquement acceptables peuvent être également présentés sous forme de complexes avec des cyclodextrines.

Dans la description et dans les exemples, les abréviations suivantes sont utilisées.

MeOH : méthanol
EtOH : éthanol
Ether : éther éthylique
Ether iso : éther isopropylique
AcOEt : acétate d'éthyle
MeCN : acétonitrile
DCM : dichlorométhane
DMF : diméthylformamide
DMSO : diméthylsulfoxide
THF : tétrahydrofurane
HCl : acide chlorhydrique

AcOH : acide acétique
TFA : acide trifluoroacétique
$H_2SO_4$ : acide sulfurique
NaOH : soude
KOH : potasse
$NH_4OH$ : ammoniaque
$Na_2SO_4$ : sulfate de sodium
$P_2O_5$ : anhydride phosphorique
Me, MeO : méthyle, méthoxy
Et : éthyle
F : point de fusion
TA : température ambiante
Silice H : gel de silice 60 H commercialisé par MERCK (DARMSTAD).
RMN : résonnance magnétique nucléaire
s : singulet
se : singulet élargi
d : doublet
t : triplet
qd : quadruplet
qt : quintuplet
m : massif
mt : multiplet

Préparation 1

Sel de sodium du 4-(2,6-diméthoxyphényl)-4-oxydo-2-oxobut-3-énoate de méthyle.

On ajoute lentement une solution de 100 g de 2,6-diméthoxyacétophénone et 7,5 ml d'oxalate d'éthyle dans 520 ml de MeOH anhydre à une solution de méthylate de sodium préparée à partir de 12,7 g de sodium et 285 ml de MeOH anhydre. On chauffe à reflux pendant 7 heures et laisse une nuit à TA. On verse le mélange réactionnel sur 2 litres d'éther iso et laisse 15 minutes sous agitation. On obtient le produit attendu par filtration, lavage à l'éther iso et séchage sous vide, m = 120 g, F = 178°C.

PREPARATIONS DES HYDRAZINES 3

Préparation 2.1

Chlorhydrate de 1-hydrazino-4-nitronaphtalène.

A une suspension refroidie à -5°C, de 5,2 g de 4-nitro-1-naphtylamine dans 150 ml d'HCl concentré, 100 ml d'HCl 1N et 150 ml d'AcOH, on ajoute une solution de 1,9 g de nitrite de sodium dans 10 ml d'eau. On laisse 1 heure 15 sous agitation, à une température comprise entre -5°C et 0°C. On refroidit à -15°C et ajoute très lentement une solution de 12,5 g de chlorure stanneux dihydrate dans 30 ml d'HCl concentré. On laisse remonter la température à TA et maintient 2 heures 30 sous agitation. On filtre le milieu réactionnel, puis reprend le solide par de l'eau et filtre à nouveau. On obtient 5,9 g du produit attendu.

Préparation 2.2

4-cyano-1-hydrazinonaphtalène.

A une solution, refroidie à 0°C, de 8,35 g de 4-cyano-1-naphtylamine dans 180 ml d'HCl 1N on ajoute une solution de 4,09 g de nitrite de sodium dans 30 ml d'eau. On laisse 1 heure 15 sous agitation à 0°C. On refroidit à -10°C et ajoute lentement une solution de 41,75 g de chlorure stanneux dihydrate dans 42 ml d'HCl concentré. On agite 1 heure en laissant remonter la température à TA. On filtre le milieu réactionnel. On met le résidu en suspension dans l'eau et ajoute 20 ml de NaOH concentré. On obtient le produit attendu après filtration, rinçages à l'eau puis séchage sous vide, m = 8,6 g.

Préparation 2.3

Chlorhydrate de l'acide 4-hydrazinonaphtalène-1-sulfonique.

A une suspension de 22,33 g de l'acide 4-aminonaphtalène-1-sulfonique dans 125 ml d'eau, on ajoute 4 g

de NaOH. On refroidit le mélange à 0°C et ajoute 2 ml de NaOH concentrée, puis 7,5 g de nitrite de sodium et 125 g de glace pour maintenir la température à 0°C. On verse la suspension ainsi obtenue sur 75 ml d'HCl concentré préalablement refroidi à 0°C et laisse 2 heures 15 sous agitation à cette température. On ajoute lentement le mélange réactionnel sur une solution de 55 g de chlorure stanneux dihydrate dans 50 ml d'HCl concentré et 25 ml d'eau, préalablement refroidie à -10°C. Après une nuit, on obtient le produit attendu par filtration, rinçage par HCl 1N et à l'eau, m = 23,96 g.

Préparation 2.4

Chlorhydrate de l'acide 4-hydrazinonaphtalène-1-acétique.

A) Chlorhydrate de l'acide 4-aminonaphtalène-1-acétique.

Ce composé se prépare selon la méthode décrite par Y. Ogata et coll., J. Org. Chem., 1951, 16, 1588.

B) Chlorhydrate de l'acide 4-hydrazinonaphtalène-1-acétique.

On mélange à -5°C, 3,18 g du composé obtenu à l'étape précédente avec 30 ml d'HCl concentré et 30 ml d'AcOH. On ajoute rapidement une solution de 1 g de nitrite de sodium dans 15 ml d'eau et laisse 2 heures sous agitation à une température comprise entre -5°C et +4°C. On ajoute ensuite une solution de 14,2 g de chlorure stanneux dihydrate dans 19 ml d'HCl concentré et laisse 30 minutes sous agitation à 0°C. On laisse remonter la température à TA et laisse 1 heure sous agitation. On filtre le milieu réactionnel et agite les cristaux recueillis, 1 heure, dans MeCN. On obtient le produit attendu après filtration, m = 2,4 g, F = 180°C.

Préparation 2.5

Chlorhydrate de l'acide 4-hydrazinonaphtalène-1-carboxylique.

A) Méthanesulfonate de l'acide 4-aminonaphtalène-1-carboxylique.

A une solution refroidie à -15°C de 5 g de 4-bromo-1-naphtylamine dans 90 ml d'éther on ajoute, sous atmosphère d'argon, 2,8 ml d'une solution 1,6 M de n-butyllithium dans l'hexane et laisse 1 heure sous agitation à -15°C. Puis on ajoute une solution de 5 g de 1,2-bis(chlorodiméthylsilyl)éthane dans 50 ml d'éther, laisse 30 minutes sous agitation à -10°C et laisse remonter la température à TA. On refroidit à -5°C, ajoute 15 ml d'une solution 1,6 M de n-butyllithium dans l'hexane et laisse 1 heure sous agitation à 0°C. Puis on fait barboter dans le mélange réactionnel un courant de dioxyde de carbone, pendant 2 heures, à une température comprise entre 0 et 5°C. On ajoute ensuite 2,86 ml de chlorotriméthylsilane et laisse 30 minutes sous agitation. Puis on ajoute de l'eau, extrait à l'AcOEt, sèche la phase organique sur $Na_2SO_4$ et évapore sous vide le solvant. On dissout le résidu dans l'acétone, ajoute 1,46 ml d'acide méthanesulfonique, essore le précipité formé et le lave à l'éther. On obtient 4 g du produit attendu, F = 180°C (déc).

On obtient également ce composé en suivant le mode opératoire décrit ci-après.

A') Acide 4-aminonaphtalène-1-carboxylique.

On chauffe à reflux pendant une nuit un mélange de 32 g de 4-cyano-1-naphtylamine dans 400 ml d'une solution à 50 % de KOH dans l'eau. Après refroidissement, on ajoute 800 ml d'eau au mélange réactionnel et filtre un insoluble. On refroidit le filtrat à +5°C et acidifie à pH = 5 par ajout d'HCl concentré. On essore le précipité formé et après séchage on obtient 35 g du produit attendu.

On obtient également ce composé en suivant les deux étapes du procédé décrit ci-après.

A'') Acide 4-nitronaphtalène-1-carboxylique.

On prépare ce composé selon J. Am. Chem. Soc., 1929, 51, 1831-1836, à partir de l'anhydride 4-nitro-1,8-naphtalique.

B'') Chlorhydrate de l'acide 4-aminonaphtalène-1-carboxylique.

On hydrogène, dans un appareil de Parr, sous une pression de 8 bars un mélange de 35 g du composé préparé à l'étape A'', 1 litre de MeOH, 200 ml de DMF et en présence de nickel de Raney®. Après 4 heures, on filtre le catalyseur et évapore sous vide le filtrat. On reprend le résidu dans l'eau, laisse une nuit sous agitation et essore le précipité. On dissout le précipité dans une solution saturée d'acide chlorhydrique dans le MeOH et ajoute de l'éther jusqu'à précipitation. Après essorage puis séchage, on obtient 21,4 g du produit attendu.

B) Chlorhydrate de l'acide 4-hydrazinonaphtalène-1-carboxylique.

On mélange à 0°C, 2,4 g de chlorhydrate de l'acide 4-aminonaphtalène-1-carboxylique avec 80 ml d'HCl concentré. On ajoute une solution de 0,89 g de nitrite de sodium dans 19 ml d'eau et laisse 2 heures sous agitation à 2-3°C. On refroidit à -10°C et ajoute lentement une solution de 9,7 g de chlorure stanneux dihydrate dans 90 ml d'HCl concentré. On laisse remonter la température à TA et maintient 30 minutes sous agitation. On ajoute 200 ml d'eau, laisse 30 minutes sous agitation et essore le solide formé. On reprend le solide dans MeCN, essore,

lave à l'éther et sèche. On obtient 2,2 g du produit attendu, F = 190°C.

On obtient également ce composé en suivant les trois étapes du procédé décrit ci-après :

A"') N-*tert*-butoxycarbonyl-4-bromo-1-naphtylamine.

On chauffe à reflux pendant 20 heures un mélange de 5g de 4-bromo-1-naphtylamine, 6,4g de di-*tert*-butyldicarbonate dans 120ml de *tert*-butanol. On verse le mélange réactionnel sur 250ml d'eau, essore le précipité formé et lave à l'eau. On dissout le précipité dans du DCM, sèche la phase organique sur $Na_2SO_4$ et évapore sous vide le solvant. On obtient 6,9g du produit attendu, F= 134°C.

B"') Acide 4-*tert*-butoxycarbonylaminonaphtalène-1-carboxylique.

A une solution refroidie à -10°C de 5g du composé obtenu à l'étape précédente dans 100 ml d'éther, on ajoute, sous atmosphère d'azote, 21,3ml d'une solution 1,6 M de n-butyllithium dans l'hexane et laisse 1 heure et 30 minutes sous agitation à 0°C. On refroidit à -10°C et fait barboter dans le mélange réactionnel un courant de dioxyde de carbone, pendant 15 minutes. Puis on ajoute 250ml d'eau et après décantation on lave la phase aqueuse à l'éther. On acidifie la phase aqueuse à pH=6 par ajout d'AcOH, extrait au DCM, sèche la phase organique sur $Na_2SO_4$ et évapore sous vide le solvant. On obtient 2,7g du produit attendu, F= 214°C.

C"') Méthanesulfonate de l'acide 4-aminonaphtalène-1-carboxylique.

A une suspension de 7g du composé obtenu à l'étape précédente dans 70ml de DCM, on ajoute, goutte à goutte et à TA, une solution de 15,8ml d'acide méthanesulfonique dans 50ml de DCM. Après 45 minutes d'agitation à TA, on ajoute au mélange réactionnel 150ml d'éther et essore le précipité formé. Après lavage du précipité à l'éther et séchage, on obtient 6,5g du produit attendu, F = 196°C(déc.).

<u>Préparation 2.6</u>

Chlorhydrate de 4-hydrazino-1,8-naphtalimide

A) Sel de potassium de 4-sulfo-1,8-naphtalimide.

On laisse 2 heures sous agitation à 60-70°C un mélange de 25 g de sel de potassium de l'anhydride 4-sulfo-1,8-naphtalique et 300 ml d'une solution aqueuse à 30 % d'ammoniaque. Après une nuit à TA, on essore le précipité formé, lave à l'eau puis à l'EtOH. On obtient 21 g du produit attendu, F > 300°C.

B) Chlorhydrate de 4-hydrazino-1,8-naphtalimide.

On chauffe pendant 4 jours à 80°C un mélange de 7 g du composé obtenu à l'étape précédente, 3,5 ml d'hydrazine monohydrate et 100 ml d'eau. Après refroidissement, on acidifie à pH = 1 par ajout d'HCl 1N, essore le précipité formé. On obtient 3,5 g du produit attendu après trituration dans le mélange EtOH/éther puis essorage, F = 278°C.

<u>Préparation 2.7</u>

N-amino-4-hydrazino-1,8-naphtalimide (composé 5)

On chauffe à 120°C pendant 1,5 jours un mélange de 25 g du sel de potassium de l'anhydride 4-sulfo 4-sulfo-1,8-naphtalique et 50 ml d'hydrazine monohydrate. Après refroidissement, on ajoute de l'eau au mélange réactionnel, essore le précipité formé et sèche. On obtient 15,7 g du produit attendu, F = 260°C.

PREPARATIONS DES ESTERS IIa,.II'a ET II"a.

<u>Préparation 3.1</u>

Ester méthylique de l'acide 5-(2,6-diméthoxyphényl)-1-(4-nitronapht-1-yl)-pyrazole-3-carboxylique.

(II'a : R' = $NO_2$ ; T = Me).

On chauffe pendant 5 heures 30 à reflux un mélange de 10 g du composé obtenu à la Préparation 2.1, 13,5 g de composé obtenu à la Préparation 1, et 200 ml d'AcOH. Après filtration de l'insoluble, on verse le filtrat sur 2 litres d'un mélange d'eau et de glace. On filtre le précipité obtenu et agite ce dernier dans 300 ml d'éther iso. On obtient 14,8 g du produit attendu après filtration, F = 180°C.

<u>Préparation 3.2</u>

Ester méthylique de l'acide 1-(4-cyanonapht-1-yl)-5-(2,6-diméthoxyphényl)pyrazole-3-carboxylique.

(IIa: R = CN, T = Me).

On chauffe pendant 5 heures 15 à reflux un mélange de 8,6 g du composé obtenu à la Préparation 2.2, 13,5 g de composé obtenu à la Préparation 1 et 85 ml d'AcOH. Après une nuit à TA, on verse le mélange réactionnel sur

un mélange d'eau et de glace. On filtre le précipité obtenu et lave à l'eau. On chromatographie sur silice en éluant par DCM puis DCM/AcOEt (98/2, v/v). On obtient 5,38 g du produit attendu, F = 165°C.

Préparation 3.3

Ester méthylique de l'acide 5-(2,6-diméthoxyphényl)-1-(4-sulfonapht-1-yl)pyrazole-3-carboxylique.
(II'a : R' = $SO_3H$, T = Me).
On chauffe pendant 2 heures à reflux un mélange de 22,9 g du composé obtenu à la Préparation 2.3, 21,65 g de composé obtenu à la Préparation 1 et 150 ml d'AcOH. Après refroidissement, on verse le milieu réactionnel sur un mélange d'eau et de glace et filtre le précipité obtenu. On refroidit le filtrat et ajuste le pH à 5-6 par ajout de NaOH concentré. On filtre le précipité obtenu et lave à l'eau. On obtient 28,66 g du produit attendu, F = 236°C.

Préparation 3.4

Ester méthylique de l'acide 1-[4-(carboxyméthyl)napht-1-yl]-5-(2,6-diméthoxyphényl)pyrazole-3-carboxylique.
(IIa : R = $-CH_2COOH$; T = Me).
On chauffe pendant 2 heures 30 à 60-70°C un mélange de 2,4 g du composé obtenu à la Préparation 2.4, 2,8 g du composé obtenu à la Préparation 1 et 50 ml d'AcOH. Après refroidissement, on ajoute de l'eau dans le milieu et sépare l'huile visqueuse formée. On dissout cette huile dans de l'EtOH et l'additionne lentement à la solution aqueuse. On agite 1 heure et filtre le précipité obtenu. On obtient 2,8 g du produit attendu, F = 200°C.

Préparation 3.5

Ester méthylique de l'acide 5-(2,6-diméthoxyphényl)-1-[4-éthanecarboxamido) napht-1-yl]pyrazole-3-carboxylique.
(IIa : R = NHCOEt ; T = Me).
On hydrogène à 80°C, à la pression atmosphérique, pendant 33 heures un mélange de 0,87 g du composé obtenu à la Préparation 3.1, 0,3 g de Palladium sur charbon à 10 % et 5 ml d'anhydride propionique dans 5 ml de DMF. Puis on ajoute 0,5 ml de pyridine et laisse une nuit sous agitation à TA. On filtre sur Célite ®, rince au MeOH et évapore sous vide. On chromatographie sur silice H en éluant par le mélange toluène/AcOEt (70/30 ; v/v). On obtient 0,45 g du produit attendu, F = 110°C.

Préparation 3.6

Ester méthylique de l'acide 1-[4-(acétylaminométhyl)napht-1-yl]-5-(2,6-diméthoxyphényl)pyrazole-3-carboxylique.
(IIa : R = $-CH_2NHCOMe$ ; T = Me).
On hydrogène, pendant 12 heures à 60°C, puis 8 heures à 100°C, à la pression atmosphérique, un mélange de 2 g du composé obtenu à la Préparation 3.2, 30 ml d'anhydride acétique et 0,2 g d'oxyde de platine. Après filtration sur Célite ®, lavage à l'AcOEt, on ajoute de l'eau au filtrat. On extrait à l'AcOEt, sèche sur sulfate de sodium et évapore sous vide le solvant. On obtient le produit attendu après chromatographie sur silice H en éluant par un gradient du mélange DCM/AcOEt (98/2 ; v/v) jusqu'à (60/40 ; v/v).On obtient 0,9 g du produit attendu.
Spectre de RMN à 200 MHz dans DMSO.

1,95 ppm : s : 3H
3,5 ppm : se : 6H
3,9 ppm : s : 3H
4,75 ppm : d : 2H
6,55 ppm : d : 2H
6,95 ppm: s : 1H
7,15 à 8,25 ppm : m : 7H
8,5 ppm : t : 1H

Préparation 3.7

Ester méthylique de l'acide 5-(2,6-diméthoxyphényl)-1-[4-(diméthylaminosulfonyl)napht-1-yl]pyrazole-3-carboxylique.

(IIa : R = $-SO_2N(Me)_2$ ; T = Me)

A) Ester méthylique de l'acide 1-[4-chlorosulfonyl)napht-1-yl]-5-(2,6-diméthoxyphényl)pyrazole-3-carboxylique.

(II'a : R' = $-SO_2Cl$; T = Me).

On agite à TA pendant 5 heures, un mélange de 5 g du composé obtenu à la Préparation 3.3, 3,21 g de pentachlorophosphore dans 100 ml de DCM. On évapore sous vide à siccité et chauffe le résidu à 110°C pendant 1 heure. On abandonne une nuit à TA. On reprend plusieurs fois le résidu par du toluène, du DCM, du 1,2-dichloroéthane, suivi à chaque fois par une évaporation sous vide. On obtient 5,9 g du produit attendu qui est utilisé tel quel à l'étape suivante.

B) Ester méthylique de l'acide 5-(2,6-diméthoxyphényl)-1-[4-(diméthylaminosulfonyl)napht-1-yl]pyrazole-3-carboxylique.

(IIa : R = $-SO_2N(Me)_2$, T = Me).

On introduit, pendant 45 minutes, de la diméthylamine gaz à une solution de 5,9 g du composé obtenu à l'étape précédente dans 100 ml de DCM auquel on ajoute du 1,2-dichloroéthane et du DMF jusqu'à dissolution du composé. Puis on laisse sous agitation à TA pendant 3 heures 30. Après filtration d'un insoluble, on évapore sous vide le filtrat. On extrait le résidu à l'AcOEt, lave à l'eau, sèche sur sulfate de sodium et évapore sous vide le solvant. On obtient 2,67 g du produit attendu.

Spectre de RMN à 200 MHz dans DMSO

2,65 ppm : s : 6H
3,35 ppm : se : 6H
6,45 ppm : d : 2H
6,95 ppm : s : 1H
7,15 ppm : t : 1H
7,3 à 8,8 ppm : m : 6H

Préparation 3.8

Ester méthylique de l'acide 5-(2,6-diméthoxyphényl)-1-{4-[N-méthyl-N-(3-N',N'-diméthylaminopropyl)aminosulfonyl]napht-1-yl}pyrazole-3-carboxylique.

(IIa : R = $-SO_2N(Me)(CH_2)_3N(Me)_2$ ; T = Me).

On dissout à reflux 2,34 g du composé obtenu à l'étape A de la Préparation 3.7 dans 50 ml de toluène, refroidit à TA et ajoute 1,4 ml de N,N,N'-triméthyl-1,3-propanediamine. Puis on agite à TA pendant 2 heures 45 et ajoute 0,7 ml de N,N,N'-triméthyl-1,3-propanediamine. Après 45 minutes d'agitation à TA, on filtre un insoluble et évapore sous vide le filtrat. On extrait le résidu à l'AcOEt, lave à l'eau, sèche sur sulfate de sodium et évapore sous vide le solvant. On chromatographie sur silice en éluant avec le mélange DCM/MeOH (100/6 ; v/v). On obtient 0,82 g du produit attendu, F = 87°C.

Préparation 3.9

Ester méthylique de l'acide 1-[4-(carbamoylméthyl)napht-1-yl]-5-(2,6-diméthoxyphényl)pyrazole-3-carboxylique.

(IIa : R = $-CH_2CONH_2$ ; T = Me).

On chauffe à 40°C pendant 3 heures un mélange de 1,4 g du composé obtenu à la Préparation 3.4, 3 ml de chlorure de thionyle dans 30ml de DCM ; puis on évapore sous vide à siccité. On ajoute une solution du chlorure d'acide préparé ci-dessus dans du THF à 20 ml d'une solution 0,4N d'ammoniac dans le THF. On laisse une nuit sous agitation à TA et évapore sous vide le mélange réactionnel. On reprend le résidu dans l'eau et filtre le précipité formé. On obtient le produit attendu que l'on utilise tel quel à la Préparation 4.8.

Préparation 3.10

Ester méthylique de l'acide 1-(4-aminonapht-1-yl)-5-(2,6-diméthoxyphényl) pyrazole-3-carboxylique.

(II'a: R' = $NH_2$ ; T = Me)

On hydrogène à TA, pendant 6 jours, à la pression atmosphérique un mélange de 7,5 g du composé préparé à la Préparation 3.1, 0,75 g du nickel de Raney ® dans 200 ml de MeOH. On filtre le mélange réactionnel sur Célite ®, lave par 100 ml de DMF et évapore sous vide les solvants. On obtient 0,7 g du produit attendu après cristallisation dans l'éther iso, F = 246°C.

Préparation 3.11

Ester méthylique de l'acide 5-(2,6-diméthoxyphényl)-1-(4-hydroxynapht-1-yl)pyrazole-3-carboxylique.

(II'a : R' = OH ; T = Me)

A un mélange, refroidi à 0°C, de 2,5 g du composé préparé à la Préparation 3.10 dans 50 ml d'$H_2SO_4$ à 35 %, on ajoute une solution de 0,475 g de nitrite de sodium dans 25 ml d'eau. On laisse 1 heure 30 sous agitation à 3°C et obtient 1,3 g de sel de diazonium après filtration des cristaux formés, puis séchage. On ajoute le sel de diazonium ainsi préparé à une solution de 130 g de nitrate de cuivre dans 800 ml d'eau et laisse 30 minutes sous agitation. On ajoute ensuite 1 g de sulfate de fer et laisse 2 heures sous agitation. Après filtration du milieu réactionnel, on reprend le résidu dans du MeOH et laisse sous agitation en présence de noir animal. On filtre sur Célite ® et évapore sous vide le filtrat. On obtient 0,69 g du produit attendu après cristallisation dans l'EtOH, F = 240°C.

Préparation 3.12

Ester méthylique de l'acide 5-(2,6-diméthoxyphényl)-1-[4-(3-N,N-diméthylaminopropoxy)napht-1-yl]pyrazole-3-carboxylique.

(IIa : R = $-O(CH_2)_3N(Me)_2$ ; T = Me)

On mélange 0,44 g du composé préparé à la Préparation 3.11, 0,19 ml d'une solution d'hydroxyde de césium à 50 % dans l'eau et 1 ml de MeOH puis on évapore à siccité. On reprend le résidu dans 5 ml de DMF et ajoute 0,48 g de chlorhydrate de 3-chloro-N,N-diméthylpropylamine puis 1,44 g de carbonate de potassium. On chauffe 3 heures à 60°C. Après refroidissement, on ajoute de l'eau, extrait à l'AcOEt, sèche sur sulfate de sodium et évapore sous vide le solvant. On chromatographie sur silice en éluant par le mélange DCM/MeOH/$NH_4OH$ (100/5/0,5 v/v/v). On obtient 0,25 g du produit attendu.

Spectre de RMN à 200 MHz dans DMSO.

1,9 ppm : qt : 2H
2,1 ppm : s : 6H
2,4 ppm : mt : 2H
3,35 ppm : s : 6H
3,7 ppm : s : 3H
4,1 ppm : t : 2H
6,35 ppm : d: 2H
6,8 ppm : s : 1H
6,95 à 8,1 ppm : m : 7H

Préparation 3.13

Ester méthylique de l'acide 1-[4-(carbamoylméthoxy)napht-1-yl]-5-(2,6-diméthoxyphényl)pyrazole-3-carboxylique.

(IIa : R = $-OCH_2CONH_2$, T = Me).

On mélange 0,39 g du composé préparé à la Préparation 3.11, 0,17 ml d'une solution d'hydroxyde de césium à 50 % dans l'eau et 1 ml de MeOH puis on évapore à siccité. On reprend le résidu dans 5 ml de DMF et ajoute 0,193 g de 2-bromoacétamide. On chauffe 2 heures à 60°C. Après refroidissement, on ajoute de l'eau, extrait à l'AcOEt, sèche sur sulfate de sodium et évapore sous vide le solvant. On obtient 0,28 g du produit attendu que l'on utilise tel quel à la Préparation 4.10.

Préparation 3.14

Ester méthylique de l'acide 1-(4-carboxynapht-1-yl)-5-(2,6-diméthoxyphényl) pyrazole-3-carboxylique.

(II'a : R' = -COOH; T = Me).

On chauffe à reflux pendant 3 heures un mélange de 2,2 g du composé obtenu à la Préparation 2,5, 2,65 g du composé obtenu à la Préparation 1 et 200 ml d'AcOH. Après refroidissement, on ajoute 700 ml d'eau et essore le précipité formé. On reprend le précipité dans du 1,4-dioxane et évapore sous vide le solvant. Après séchage, on obtient 2,75 g du produit attendu, F = 240°C.

Préparation 3.15

Ester méthylique de l'acide 5-(2,6-diméthoxyphényl)-1-[4-[N-[3-(N',N'-diméthylamino)propyl]carbamoyl]napht-1-yl]pyrazole-3-carboxylique.

(IIa : R = $-CONH(CH_2)_3N(Me)_2$ ; T = Me).

On laisse 2 heures sous agitation un mélange de 0,5 g du composé obtenu à la Préparation 3.14 et 5 ml de chlorure de thionyle puis on concentre sous vide. On reprend le chlorure d'acide ainsi obtenu dans 5 ml de DCM puis on ajoute, goutte à goutte, cette solution à une solution de 0,165 ml de N,N-diméthyl-1,3-propanediamine, 0,172 ml de triéthylamine dans 10 ml de DCM, et laisse une nuit sous agitation à TA. On ajoute de l'eau au mélange réactionnel, après décantation, sèche la phase organique sur $Na_2SO_4$ et évapore sous vide le solvant. On obtient 0,52 g du produit attendu.

Spectre de RMN à 200 MHz dans DMSO

1,8 à 2,0 ppm : mt : 2H
2,8 ppm : s : 6H
3,05 à 3,40 ppm : 2t : 4H
3,4 ppm : s : 6H
6,5 ppm : d: 2H
7,0 ppm : s : 1H
7,1 à 7,2 ppm : t : 1H
7,4 ppm : d : 1H
7,45 à 7,70 ppm : m : 4H
8,20 ppm : mt : 1H

<u>Préparation 3.16</u>

Ester méthylique de l'acide 5-(2,6-diméthoxyphényl)-1-[4-[N-[2-(N',N'-diméthylamino)éthyl]carbamoyl]napht-1-yl]pyrazole-3-carboxylique.

(IIa : R = $-CONH(CH_2)_2N(Me)_2$ ; T = Me).

On prépare ce composé selon le mode opératoire décrit à la Préparation 3.15 à partir de 0,5 g du composé obtenu à la Préparation 3.14, 5 ml de chlorure de thionyle puis 0,155 ml de N,N-diméthyléthylènediamine et 0,196 ml de triéthylamine. On obtient 0,6 g du produit attendu.

Spectre de RMN à 200 MHz dans DMSO

2,8 ppm : s : 6H
3,2 à 3,8 ppm : m : 10H
3,9 ppm : s : 3H
6,8 ppm : d : 2H
7,0 ppm : s : 1H
7,2 ppm : t : 1H
7,4 ppm : d : 1H
7,45 à 7,70 ppm : m : 3H
7,8 ppm : d : 1H
8,3 ppm : m : 1H
10,2 ppm : se : 1H

<u>Préparation 3.17</u>

Ester méthylique de l'acide 1[4-[N-(cyanométhyl)carbamoyl]napht-1-yl]-5-(2,6-diméthoxyphényl)pyrazole-3-carboxylique.

(IIa: R = $-CONHCH_2CN$ ; T = Me).

On prépare ce composé selon le mode opératoire décrit à la Préparation 3.15 à partir de 1,22 g du composé obtenu à la Préparation 3.14, 12 ml de chlorure de thionyle puis 0,269 g de chlorhydrate d'aminoacétonitrile et 0,8 ml de triéthylamine. On obtient 0,77 g du produit attendu après cristallisation dans l'EtOH, F = 138-140°C.

<u>Préparation 3.18</u>

Ester méthylique de l'acide 1-[4-[N-(2-cyanoéthyl)-N-méthylcarbamoyl]napht-1-yl]-5-(2,6-diméthoxyphényl) pyrazole-3-carboxylique.

(IIa : R = $-CON(Me)CH_2CH_2CN$ ; T = Me).

On prépare ce composé selon le mode opératoire décrit à la Préparation 3.15 à partir de 10 g du composé

obtenu à la Préparation 3.14, 15 ml de chlorure de thionyle puis 2,4 ml de 3-méthylaminopropionitrile et 3,3 ml de triéthylamine. On obtient 12 g du produit attendu.

Spectre de RMN à 200 MHz dans DMSO

2,6 ppm : s : 3H
2,8 ppm : t : 2H
3,3 ppm : s : 6H
3,9 ppm : m : 5H
6,4 ppm : d : 2H
6,9 ppm : s : 1H
7,1 ppm : t : 1H
7,2 à 7,8 ppm : m : 6H

Préparation 3.19

Ester méthylique de l'acide 5-(2,6-diméthoxyphényl)-1-[4-(N-méthylcarbamoyl) napht-1-yl)pyrazole-3-carboxylique.

(IIa : R = -CONHMe ; T = Me).

On chauffe à 40°C pendant 2 heures un mélange de 4 g du composé obtenu à la Préparation 3.14, 20 ml de chlorure de thionyle dans 20 ml de DCM puis concentre sous vide. On reprend le chlorure d'acide ainsi obtenu dans 40 ml de DCM et ajoute, goutte à goutte, à une solution de 4 ml d'une solution aqueuse à 40 % de méthylamine dans 80 ml de MeOH, préalablement refroidie à 5°C. On laisse une nuit sous agitation à TA, concentre sous vide, reprend le résidu à l'eau et essore le précipité formé. Après séchage, on obtient le produit attendu que l'on utilise tel quel à la Préparation 4.15.

Préparation 3.20

Ester méthylique de l'acide 5-(2,6-diméthoxyphényl)-1-[4-(6-acétamidohexanoyl amino)napth-1-yl]pyrazole-3-carboxylique.

(IIa : R = $-NHCO(CH_2)_5NHCOMe$ ; T = Me).

A) Chlorure de l'acide 6-acétamidohexanoïque.
On laisse 24 heures sous agitation à TA un mélange de 0,37 g d'acide 6-acétamidohexanoïque et 2,5 ml de chlorure de thionyle. On concentre sous vide et utilise tel quel le chlorure d'acide ainsi obtenu à l'étape suivante.
B) Ester méthylique de l'acide 5-(2,6-diméthoxyphényl)-1-[4-(6-acétamido hexanoylamino)napth-1-yl]pyrazole-3-carboxylique.

On laisse une nuit sous agitation à TA un mélange de 0,5 g du composé obtenu à la Préparation 3.10, 0,5 ml de bis(triméthylsilyl)acétamide dans 5 ml de MeCN. Puis on ajoute une solution du composé obtenu à l'étape précédente dans 5 ml de MeCN, puis 2 ml de triéthylamine. On laisse deux jours sous agitation à TA, ajoute 5 ml de MeOH et 5 ml d'eau, laisse 15 minutes sous agitation et concentre sous vide. On extrait le résidu avec 50 ml de DCM, lave la phase organique à l'eau, par une solution d'HCl 1N, sèche sur $Na_2SO_4$ et concentre sous vide le solvant. On obtient 0,7 g du produit attendu que l'on utilise tel quel à la Préparation 4.16.

Préparation 3.21

Ester méthylique de l'acide 5-(2,6-diméthoxyphényl)-1-[4-[N-méthyl-N-[3-(N',N'-diméthylamino)propyl]carbamoyl]napht-1-yl]pyrazole-3-carboxylique.

(IIa : R = $-CON(Me)(CH_2)_3N(Me)_2$ ; T = Me).

On prépare ce composé selon le mode opératoire décrit à la Préparation 3.15 à partir de 0,5 g du composé obtenu à la Préparation 3.14, 5 ml de chlorure de thionyle puis 0,120 ml de N,N,N'-triméthyl-1,3-propanediamine et 0,170 ml de triéthylamine. On obtient 0,43 g du produit attendu.

Préparation 3.22

Ester méthylique de l'acide 5-(2,6-diméthoxyphényl)-1-[4-[N-méthyl-N-[2-(N',N'-diméthylamino)éthyl]carbamoyl]napht-1-yl]pyrazole-3-carboxylique.

(IIa : R = $-CON(Me)(CH_2)_2N(Me)_2$ ; T = Me).

On prépare ce composé selon le mode opératoire décrit à la Préparation 3.15 à partir de 0,5 g du composé obtenu à la Préparation 3.14, 5 ml de chlorure de thionyle puis 0,179 ml de N,N,N'-triméthyléthylènediamine et 0,2 ml de triéthylamine. On obtient 0,4 g du produit attendu.

<u>Préparation 3.23</u>

Ester méthylique de l'acide 5-(2,6-diméthoxyphényl)-1-[1,3(2H)-dioxo-1*H*-benz[de]isoquinol-6-yl]pyrazole-3-carboxylique.

(IIa : 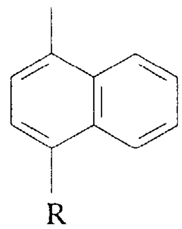 = 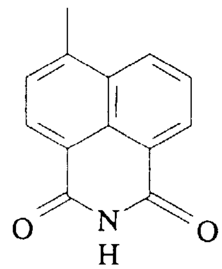 ; T = Me)

On chauffe à reflux pendant 3 heures un mélange de 2 g du composé obtenu à la Préparation 2.6, 2,4 g du composé obtenu à la Préparation 1 et 50 ml d'AcOH. Après refroidissement, on ajoute de l'eau et essore le précipité formé. On chromatographie sur silice le précipité en éluant par le mélange DCM/MeOH (100/2 ; v/v). On obtient 2,4 g du produit attendu, F = 138°C (déc.)

<u>Préparation 3.24</u>

Ester méthylique de l'acide 5-(2,6-diméthoxyphényl)-1-[2-amino-1,3(2*H*)-dioxo-1*H*-benz[de]isoquinol-6-yl] pyrazole-3-carboxylique.
(II"a T = Me).
On chauffe à 70°C pendant 2 heures un mélange de 3 g du composé obtenu à la Préparation 2.7, 3,6 g du composé obtenu à la Préparation 1 et 70 ml d'AcOH. On concentre sous vide, reprend le résidu à l'eau, essore le précipité formé et sèche. On chromatographie le précipité sur silice en éluant par le mélange DCM/AcOH (100/5 ; v/v). On obtient 3,3 g du produit attendu que l'on utilise tel quel à la Préparation 4.20.

PREPARATIONS DES ACIDES II, II'et II".

<u>Préparation 4.1</u>

Acide 5-(2,6-diméthoxyphényl)-1-(4-nitronapht-1-yl)pyrazole-3-carboxylique.
(II' R' = $-NO_2$ ; T = Me).
On chauffe pendant 1 heure 30 à 40°C un mélange de 14,8 g de l'ester obtenu à la Préparation 3.1, 5,8 g de KOH, 12 ml d'eau et 50 ml de MeOH. On évapore sous vide les solvants et reprend le résidu avec 250 ml d'eau. On acidifie à 10°C à pH 1 par ajout d'HCl 1N. On filtre le précipité formé et lave deux fois à l'eau. On obtient 14,64 g du produit attendu après recristallisation dans l'éther iso, F = 255°C.

<u>Préparation 4.2</u>

Acide 5-(2,6-diméthoxyphényl)-1-[4-(éthanecarboxamido)napht-1-yl]-pyrazole-3-carboxylique.
(II : R = -NHCOEt ; T = Me).
On agite à TA pendant 4 heures 30 un mélange de 0,35 g du composé obtenu à la Préparation 3.5, 0,07 g d'hydroxyde de lithium monohydrate dans 2 ml d'EtOH. On évapore sous vide, reprend le résidu dans 5 ml d'HCl 1N et laisse 1 heure sous agitation. On obtient 0,27 g du produit attendu après filtration et lavages à l'eau, F = 168°C.

<u>Préparation 4.3</u>

Acide 1-(4-cyanonapht-1-yl)-5-(2,6-diméthoxyphényl)pyrazole-3-carboxylique.
(II : R = -CN ; T = Me).
On agite une nuit à TA un mélange de 4,88 g de l'ester obtenu à la Préparation 3.2, 1,2 ml de NaOH concentrée

dans 500 ml de MeOH. On chauffe 3 heures à 40°C et concentre sous vide de moitié le milieu réactionnel. On ajoute 1,2 ml de NaOH concentré et chauffe 6 heures à 40°C. Après 48 heures à TA, on concentre en partie le milieu réactionnel et verse sur un mélange d'eau et de glace. On extrait à l'éther, acidifie la phase aqueuse à pH 4 par addition d'HCl 1, 2N, extrait à l'AcOEt, sèche sur sulfate de sodium et évapore sous vide le solvant. On obtient 4,6 g de produit attendu, F = 216°C.

Préparation 4.4

Acide 1-(4-carbamoylnapht-1-yl)-5-(2,6-diméthoxyphényl)pyrazole-3-carboxylique.
(II : R = $-CONH_2$ ; T = Me).
On chauffe pendant 1 heure 20 à reflux un mélange de 1,48 g du composé obtenu à la Préparation 3.2, 3 ml de NaOH 6N et 3 ml de peroxyde d'hydrogène (solution aqueuse à 33 %) dans 15 ml d'EtOH 95. Puis on agite à TA pendant une nuit. On filtre le précipité obtenu et rince à l'EtOH 95. A une suspension dans l'eau du solide recueilli, on ajoute HCl concentré jusqu'à pH 1. On obtient 0,92 g du produit attendu après filtration, lavages à l'eau, et sèchage, F = 305°C.

Préparation 4.5

Acide 1-[4-(acétylaminométhyl)napht-1-yl]-5-(2,6-diméthoxyphényl)pyrazole-3-carboxylique;
(II : R = $-CH_2NHCOMe$ ; T = Me).
On agite à TA pendant 4 jours un mélange de 0,8 g du composé obtenu à la Préparation 3.6, et 0,1 g d'hydroxyde de lithium monohydrate dans 12 ml de MeOH et 1,5 ml d'eau. On verse le mélange réactionnel sur un mélange d'eau et de glace et lave à l'éther. On acidifie la phase aqueuse à pH = 2 par addition d'HCl 1,2N. On obtient 0,58 g du produit attendu après filtration du précipité formé, lavage à l'eau et séchage sous vide sur $P_2O_5$, F = 252°C.

Préparation 4.6

Acide 5-(2,6-diméthoxyphényl)-1-[4-(diméthylaminosulfonyl)napht-1-yl]-pyrazole-3-carboxylique.
(II : R = $-SO_2N(Me)_2$ ; T = Me).
On ajoute à TA une solution de 0,6 g de KOH dans 0,5 ml d'eau à une solution de 2,15 g du composé obtenu à la Préparation 3.7 dans 15 ml de dioxane. On laisse sous agitation à TA pendant 1 heure puis on abandonne le milieu réactionnel pendant une nuit. On concentre en partie ledit milieu sous vide et laisse sous agitation à TA pendant 4 heures. On verse sur un mélange d'eau et de glace et lave à l'éther. On acidifie la phase aqueuse à pH = 4-5 par addition d'HCl 1,2N. On obtient 2 g du produit attendu après filtration du précipité formé, lavage à l'eau et séchage sous vide sur $P_2O_5$, F = 209°C.

Préparation 4.7

Acide 5-(2,6-diméthoxyphényl)-1-{4-[N-méthyl-N-(3-N',N'-diméthylaminopropyl)aminosulfonyl]napht-1-yl}pyrazole-3-carboxylique.
(II R = $-SO_2N(Me)(CH_2)_3N(Me)_2$; T = Me).
On ajoute à TA une solution de 0,2 g de KOH dans 0,5 ml d'eau à une solution de 0,82 g du composé obtenu à la Préparation 3.8 dans 16 ml de dioxane. On laisse sous agitation à TA pendant 1 jour puis verse sur un mélange d'eau et de glace. On lave à l'éther et acidifie la phase aqueuse à pH = 1 par addition d'HCl 1N. On extrait ensuite au DCM, sèche sur sulfate de sodium et évapore sous vide le solvant. On obtient 0,68 g du produit attendu après cristallisation dans l'éther, F = 176°C.

Préparation 4.8

Acide 1-[4-(carbamoylméthyl)napht-1-yl]-5-(2,6-diméthoxyphényl)pyrazole-3-carboxylique.
(II R = $-CH_2CONH_2$ ; T = Me).
On chauffe à reflux pendant 2 heures un mélange du composé obtenu à la Préparation 3.9, 0,35 g de KOH dans 10 ml d'EtOH 95. On évapore sous vide le mélange réactionnel et reprend le résidu par une solution d'HCl 1N. On obtient 1,3 g du produit attendu après filtration du précipité formé puis séchage, F = 262°C.

Préparation 4.9

Acide 5-(2,6-diméthoxyphényl)-1-[4-(3-N,N-diméthylaminopropoxy)napht-1-yl]pyrazole-3-carboxylique.

(II : R = $-O(CH_2)_3N(Me)_2$ ; T = Me)
On chauffe à 60°C pendant 2 heures un mélange de 0,25 g du composé obtenu à la Préparation 3.12, 0,025 g d'hydroxyde de lithium monohydrate dans 3 ml de MeOH et 0,4 ml d'eau. On évapore sous vide et neutralise le milieu aqueux à pH = 7 par addition d'HCl 1N. On obtient 0,19 g du produit attendu après filtration du précipité formé puis séchage et on l'utilise tel quel à l'EXEMPLE 16.

Préparation 4.10

Acide 1-[4-(carbamoylméthoxy)napht-1-yl]-5-(2,6-diméthoxyphényl)pyrazole-3-carboxylique.
(II : R = $-OCH_2CONH_2$ ; T = Me).
On chauffe à 60°C pendant 4 heures un mélange de 0,28 g du composé obtenu à la Préparation 3.13, 0,029 g d'hydroxyde de lithium monohydrate dans 3 ml de MeOH et 3 ml d'eau. On évapore sous vide, ajoute de l'eau et acidifie à pH 2 avec HCl N. On obtient 0,31 g du produit attendu après filtration du précipité formé puis séchage, F = 224°C.

Préparation 4.11

Acide 5-(2,6-diméthoxyphényl)-1-[4-[N-[3-(N',N'-diméthylamino)propyl] carbamoyl]napht-1-yl]pyrazole-3-carboxylique.
(II : R = $-CONH(CH_2)_3N(Me)_2$ ; T = Me).
On chauffe à reflux pendant 2 heures un mélange de 0,5 g du composé obtenu à la Préparation 3.15, 0,058 g d'hydroxyde de lithium monohydrate dans 15 ml de MeOH et 5 ml d'eau. On ajoute une solution d'HCl 1N jusqu'à pH = 6 et concentre sous vide. On reprend le résidu par une solution saturée de NaCl, ajoute du DCM et essore le solide formé. On reprend ce solide dans l'EtOH, filtre un insoluble et évapore sous vide le filtrat. On obtient 0,41 g du produit attendu.
Spectre de RMN à 200 MHz dans DMSO

1,5 à 1,7 ppm : qt : 2H
2,2 ppm : s : 6H
2,4 ppm : m : 2H
3,2 ppm : m : 2H
3,3 ppm : s : 6H
6,4 ppm : d : 2H
6,7 ppm : s : 1H
7,1 ppm : t : 1H
7,2 ppm : d : 1H
7,3 à 7,5 ppm : m : 4H
8,0 ppm : m : 1H
8,6 ppm : t : 1H

Préparation 4.12

Acide 5-(2,6-diméthoxyphényl)-1-[4-[N-[2-(N',N'-diméthylamino)éthyl]carbamoyl]napht-1-yl]pyrazole-3-carboxylique.
(II : R = $-CONH(CH_2)_2N(Me)_2$ ; T = Me).
On chauffe à 70°C pendant 2 heures un mélange de 0,6 g du composé obtenu à la Préparation 3.16, 0,088 g d'hydroxyde de lithium monohydrate dans 10 ml de MeOH et 10 ml d'eau. On concentre sous vide, reprend le résidu par une solution saturée de NaCl, ajoute une solution d'HCl 1N jusqu'à pH = 6,5, extrait au DCM, sèche la phase organique sur $Na_2SO_4$ et évapore sous vide le solvant. On obtient 0,44 g du produit attendu que l'on utilise tel quel à l'EXEMPLE 20.

Préparation 4.13

Acide 1-[4-[N-(cyanométhyl)carbamoyl]napht-1-yl]-5-(2,6-diméthoxyphényl)pyrazole-3-carboxylique.
(II : R = $-CONHCH_2CN$ ; T = Me).
On chauffe à reflux pendant 1 heure 30 minutes un mélange de 0,77 g du composé obtenu à la Préparation 3.17, 0,118 g d'hydroxyde de lithium monohydrate dans 15 ml de MeOH et 10 ml d'eau puis concentre sous vide. On reprend le résidu par une solution saturée de NaCl, extrait au DCM, sèche la phase organique sur $Na_2SO_4$ et évapore

sous vide le solvant. On obtient 0,55 g du produit attendu.

Spectre de RMN à 200 MHz dans DMSO

3,4 ppm : s : 6H
3,8 ppm : s : 2H
6,4 ppm : d : 2H
6,8 ppm : s : 1H
7,1 ppm : t : 1H
7,3 ppm : d : 1H
7,4 à 7,6 ppm : m : 4H
8,2 ppm : m : 1H
8,8 ppm : m : 1H

Préparation 4.14

Acide 1-[4-[N-(2-cyanoéthyl)-N-méthylcarbamoyl]napht-1-yl]-5-(2,6-diméthoxyphényl) pyrazole-3-carboxylique.

(II : R = $-CON(Me)CH_2CH_2CN$ ; T = Me).

On laisse une nuit sous agitation à TA un mélange de 8 g du composé obtenu à la Préparation 3.18, 0,77 g d'hydroxyde de lithium monohydrate dans 100 ml de MeOH et 100 ml d'eau. On dilue le mélange réactionnel par addition d'eau, puis ajoute une solution d'HCl 1N jusqu'à pH = 2 et essore le précipité formé. Après séchage, on obtient 7,36 g du produit attendu, F = 145°C.

Préparation 4.15

Acide 5-(2,6-diméthoxyphényl)-1-[4-(N-méthylcarbamoyl)napht-1-yl]-pyrazole-3-carboxylique.

(II : R = -CONHMe ; T = Me).

On chauffe à 50°C pendant 3 heures un mélange du composé obtenu à la Préparation 3.19, 0,69 g d'hydroxyde de lithium monohydrate dans 50 ml de MeOH et 50 ml d'eau. On ajoute une solution à 10 % d'HCl jusqu'à pH = 2 et essore le précipité formé. Après séchage, on recristallise le composé dans le MeOH et obtient 3,3 g du produit attendu, F > 260°C.

Spectre de RMN à 200 MHz dans DMSO

2,8 ppm : s : 3H
3,4 ppm : s : 6H
6,4 ppm : d: 2H
6,8 ppm : s : 1H
7,1 ppm : t : 1H
7,35 à 8,20 ppm : m : 6H
8,45 ppm : qd : 1H

Préparation 4.16

Acide 5-(2,6-diméthoxyphényl)-1-[4-(6-acétamidohexanoylamino)napht-1-yl] pyrazole-3-carboxylique.

(II : R = $-NHCO(CH_2)_5NHCOMe$ ; T = Me).

On laisse une nuit sous agitation à TA, un mélange de 0,7 g du composé obtenu à la Préparation 3.20, 0,18 g d'hydroxyde de lithium monohydrate dans 5 ml de 1,4-dioxane et 1 ml d'eau. On concentre sous vide, reprend le résidu dans 10 ml de MeOH et 5 ml d'eau et chauffe à 45°C pendant 2 heures dans un bain à ultrasons. On concentre sous vide, reprend le résidu dans 20 ml d'eau, lave avec 30 ml d'éther, acidifie la phase aqueuse à pH = 1 par ajout d'$H_2SO_4$ concentré, extrait deux fois par 500 ml d'AcOEt, sèche sur $Na_2SO_4$ et évapore sous vide. On obtient 0,4 g du produit attendu, F = 140°C.

Préparation 4.17

Acide 5-(2,6-diméthoxyphényl)-1-[4-[N-méthyl-N-[3-(N',N'-diméthylamino)propyl]carbamoyl]napht-1-yl]pyrazole-3-carboxylique.

(II : R = $-CON(Me)(CH_2)_3N(Me)_2$ ; T = Me).

On chauffe à reflux pendant 2 heures un mélange de 0,41 g du composé obtenu à la Préparation 3.21, 0,043

g d'hydroxyde de lithium monohydrate dans 15 ml de MeOH et 5 ml d'eau. On concentre sous vide, reprend le résidu par une solution saturée de NaCl, ajoute une solution d'HCl 1N jusqu'à pH = 6, extrait au DCM, sèche la phase organique sur $Na_2SO_4$ et évapore sous vide le solvant. On obtient 0,33 g du produit attendu.

Préparation 4.18

Acide 5-(2,6-diméthoxyphényl)-1-[4-[N-méthyl-N-[2-(N',N'-diméthylamino)éthyl]carbamoyl]napht-1-yl]pyrazole-3-carboxylique.

(II : R = $-CON(Me)(CH_2)_2N(Me)_2$ ; T = Me).

On chauffe à reflux pendant 2 heures un mélange de 0,38 g du composé obtenu à la Préparation 3.22, 0,052 g d'hydroxyde de lithium monohydrate dans 5 ml de MeOH et 5 ml d'eau. On concentre sous vide, reprend le résidu par une solution saturée de NaCl, ajoute une solution d'HCl 1N jusqu'à pH = 6,5, extrait au DCM, sèche sur $Na_2SO_4$ et évapore sous vide le solvant. On obtient 0,32 g du produit attendu.

Préparation 4.19

Acide 5-(2,6-diméthoxyphényl)-1-[1,3(2*H*)-dioxo-1*H*-benz[de]isoquinol-6-yl] pyrazole-3-carboxylique

(II : 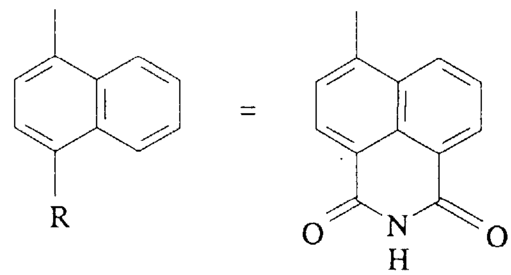 ; T = Me)

On laisse une nuit sous agitation à TA un mélange de 2,4 g du composé obtenu à la Préparation 3.23, 0,375 g d'hydroxyde de lithium monohydrate, 10 ml de MeOH et 10 ml d'eau puis on chauffe 2 heures à 60°C. Après refroidissement, on acidifie à pH = 2 par ajout d'HCl 1N et essore le précipité formé. On obtient 2,3 g du produit attendu, F = 190°C (déc.).

Préparation 4.20

Chlorhydrate de l'acide 5-(2,6-diméthoxyphényl)-1-[2-amino-1,3(2H)-dioxo-1*H*-benz[de]isoquinol-6-yl]pyrazole-3-carboxylique.

(II" T = Me).

On laisse une nuit sous agitation à TA un mélange de 3,3 g du composé obtenu à la Préparation 3.24, 0,7 g d'hydroxyde de lithium monohydrate, 15 ml de MeOH et 15 ml d'eau. On acidifie le mélange réactionnel à pH = 2 par ajout d'HCl 1N, essore le précipité formé et sèche. On obtient 2, 76 g du produit attendu, F = 180°C.

Préparation 4.21

Acide 5-(2,6-diméthoxyphényl)-1-[2-acétamido-1,3(2H)-dioxo-1*H*-benz[de]isoquinol-6-yl]pyrazole-3-carboxylique.

(II : 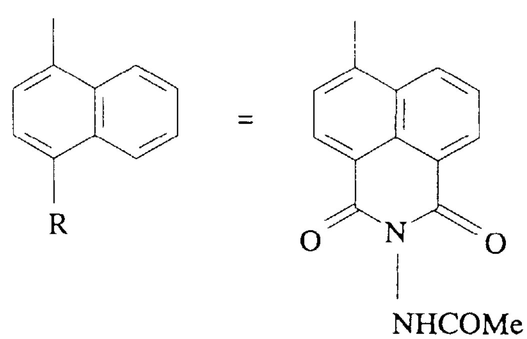 ; T = Me)

On laisse une nuit sous agitation à TA un mélange de 2,26 g du composé obtenu à la Préparation 4.20, 1,13 ml d'anhydride acétique, 0,407 g de bicarbonate de sodium et 75 ml d'AcOH. On ajoute de l'eau au mélange réactionnel, essore le précipité formé et sèche. On obtient 1,9 g du produit attendu, F = 250°C.

PREPARATION D'UN COMPOSE III.

Chlorhydrate de l'ester méthylique de la (S)-cyclohexylglycine.

A) (S)-N-*tert*-Butoxycarbonylcyclohexylglycine.

On hydrogène à TA, pendant 4 jours, sous 70 bars de pression un mélange de 10,7 g de (S)-N-*tert*Butoxycarbonylphénylglycine, 2 g de rhodium sur alumine à 5 % dans 100 ml de MeOH. On filtre le catalyseur sur Célite ® et évapore sous vide le filtrat. On obtient 11,1 g du produit attendu.

$\alpha_D^{20°}$ = + 3,5[c = 1; DMF]

B) Trifluoracétate de la (S)-cyclohexylglycine.

On ajoute rapidement 50 ml de TFA à une solution, refroidie à 0°C, de 11 g du composé obtenu à l'étape précédente dans 50 ml de DCM. On laisse 1 heure sous agitation à TA et évapore sous vide. On reprend le résidu dans l'éther iso et filtre le précipité formé. On obtient 7,6g du produit attendu, F > 260°C.

$\alpha_D^{20°}$ = +19,2 [c = 2; HCl 5N]

C) Chlorhydrate de l'ester méthylique de la (S)-cyclohexylglycine.

A une solution, refroidie à -10°C, de 1,2 g du composé obtenu à l'étape précédente dans 200 ml de MeOH, on ajoute, goutte à goutte, 10 ml de chlorure de thionyle. On laisse revenir à TA et chauffe 2 heures à reflux. On évapore à siccité et reprend le résidu dans du toluène puis évapore sous vide ; on répète deux fois cette opération. On obtient 1 g du produit attendu, F >260°C.

Spectre de RMN à 200 MHz dans DMSO.

0,8 à 2,1ppm : m : 11 H
2,6 ppm : DMSO
3,4 ppm : DHO
3,8 ppm : s : 3 H
3,95 ppm : d :1H
8,6 ppm : se : 3 H

EXEMPLE 1

Acide 2-[5-(2,6-diméthoxyphényl)-1-(4-formamidonapht-1-yl)pyrazol-3-yl-carbonylamino]adamantane-2-carboxylique.
(I : R = -NHCHO ; T = Me ; AA(OH) = 2-carboxyadamant-2-yl).

A) Chlorure de l'acide 5-(2,6-diméthoxyphényl)-1-(4-nitronapht-1-yl)pyrazole-3-carboxylique.

On chauffe pendant 4 heures à reflux une solution de 14,6 g de l'acide préparé à la Préparation 4.1 dans 100 ml de DCM et 30 ml de chlorure de thionyle. On évapore sous vide, reprend le résidu par du DCM puis évapore à nouveau. Le chlorure d'acide ainsi obtenu est utilisé tel quel à l'étape suivante.

B) Acide 2-[5-(2,6-diméthoxyphényl)-1-(4-nitronapht-1-yl)pyrazol-3-yl-carbonylamino]adamantane-2-carboxylique.

(I': R' = $-NO_2$ ; T = Me ; AA(OH) = 2-carboxyadamant-2-yl).

On ajoute à TA une solution de chlorure d'acide préparé à l'étape précédente dans 20 ml de DCM à une suspension de 6,8 g de l'acide 2-aminoadamantane-2-carboxylique dans 100 ml de DMF et 20 ml de pyridine. On laisse une nuit sous agitation à TA et évapore sous vide les solvants. On reprend le résidu par 200 ml d'HCl 1N et filtre le précipité formé. On obtient 10 g du produit attendu après deux recristallisations successives dans MeCN, F = 268°C.

C) Acide 2-[1-(4-aminonapht-1-yl)-5-(2,6-diméthoxyphényl)pyrazol-3-ylcarbonylamino]adamantane-2-carboxylique.

(I': R' = $-NH_2$ ; T = Me, AA(OH) = 2-carboxyadamant-2-yl).

On hydrogène à TA, pendant 4 heures, à la pression atmosphérique un mélange de 0,2 g du composé préparé à l'étape précédente, 0,05 g de Palladium sur charbon à 10 % dans 200 ml d'EtOH. On filtre le mélange réactionnel et évapore sous vide le solvant. On obtient 0,15 g du produit attendu après cristallisation dans l'éther iso et recristallisation dans le MeOH, F = 222°C.

D) Acide 2-[5-(2,6-diméthoxyphényl)-1-(4-formamidonapht-1-yl)pyrazol-3-ylcarbonylamino]adamantane-2-carboxylique.

On agite à TA pendant 1 heure un mélange de 0,2 g du composé préparé à l'étape précédente avec 1 ml d'acide formique (99-100 % ; d = 1,22) dans 2 ml d'anhydride acétique. On filtre le mélange réactionnel et lave le solide recueilli à l'éther iso. On obtient 0,17 g du produit attendu après séchage sous vide à 100°C, F = 270°C.

EXEMPLE 2

Acide 2-{5-(2,6-diméthoxyphényl)-1-[4-(méthylsulfonamido)napht-1-yl]pyrazol-3-ylcarbonylamino}adamantane-2-carboxylique.

(I: R = $-NHSO_2Me$ ; T = Me ; AA(OH) = 2-carboxyadamant-2-yl).

On agite à TA pendant 1 heure un mélange de 0,3 g du composé préparé à l'étape C de l'EXEMPLE 1, 0,3 g de chlorure de triméthylsilyle et 1,6 ml de triéthylamine dans 30 ml de THF. On ajoute 0,09 ml de chlorure de méthanesulfonyle et chauffe à reflux pendant 23 heures 30. On évapore sous vide, extrait au DCM, lave par HCl 1N, à l'eau, sèche sur sulfate de sodium et évapore sous vide. On chromatographie sur silice en éluant par du DCM puis par un mélange DCM/MeOH (97/3 ; v/v) jusqu'à (88/12; v/v). On obtient 0,15 g du produit attendu, F = 200°C (déc.).

EXEMPLE 3

Acide 2-[1-(4-acétamidonapht-1-yl)-5-(2,6-diméthoxyphényl)pyrazol-3-ylcarbonyl-amino]adamantane-2-carboxylique.

(I: R = -NHCOMe ; T = Me, AA(OH) = 2-carboxyadamant-2-yl).

A) 2'-[1-(4-acétamidonapht-1-yl)-5-(2,6-diméthoxyphényl)pyrazol-3-yl]spiro[adamantane-2,4'-(2'-oxazolin-5'-one)].

On agite à TA pendant 50 minutes un mélange de 0,2 g du composé préparé à l'étape C de l'EXEMPLE 1 avec 6 ml d'anhydride acétique. On évapore sous vide et reprend le résidu par une solution aqueuse à 5 % de carbonate de sodium. On filtre le précipité, lave à l'eau et sèche au dessicateur. On chromatographie sur silice H en éluant par un mélange DCM/AcOEt (95/5 ; v/v). On obtient 0,093 g du produit attendu après cristallisation dans l'éther iso, F = 213°C (déc.).

B) Acide 2-[1-(4-acétamidonapht-1-yl)-5-(2,6-diméthoxyphényl)pyrazol-3-ylcarbonyl amino]adamantane-2-carboxylique.

On abandonne à TA pendant 15 jours une solution de 0,09 g du composé préparé à l'étape précédente dans 2 ml de TFA et 2 ml de DCM. On évapore sous vide les solvants et reprend le résidu par de l'éther. On obtient 0,065 g du produit attendu après filtration et lavage à l'éther, F = 213°C (dec).

EXEMPLE 4

Acide 2-{5-(2,6-diméthoxyphényl)-1-[4-(éthanecarboxamido)napht-1-yl] pyrazol-3-ylcarbonylamino}adamantane-2-carboxylique.

(I: R = -NHCOEt ; T = Me ; AA(OH) = 2-carboxyadamant-2-yl).

On chauffe 2 heures 30 à reflux, sous atmosphère d'azote, une solution de 0,14 g de l'acide 2-aminoadamantane-2-carboxylique, 0,3 ml de bis(triméthylsilyl)acétamide dans 7 ml de MeCN. On conserve à TA. D'autre part, on ajoute 0,1 ml de chloroformiate d'isobutyle à une solution, refroidie à +5°C, de 0,31 g du composé préparé à la Préparation 4.2, 0,1 ml de triéthylamine dans 5 ml de DCM. On agite 3 heures à TA. On verse cette solution sur la solution du dérivé silylé ci-dessus et abandonne 4 jours à TA sous atmosphère d'azote. On acidifie à pH = 1 par ajout d'HCl 1N et extrait au DCM, sèche sur sulfate de sodium et évapore sous vide. On reprend le résidu dans du cyclohexane à reflux et après décantation, on extrait le produit à l'éther iso à reflux. On obtient 0,15 g du produit attendu après cristallisation en refroidissant à TA, F = 192°C.

EXEMPLE 5

Acide 2-[1-(4-cyanonapht-1-yl)-5-(2,6-diméthoxyphényl)pyrazol-3-ylcarbonylamino]adamantane-2-carboxylique.

(I : R = -CN ; T = Me ; AA(OH) = 2-carboxyadamant-2-yl).

A) Chlorure de l'acide 1-(4-cyanonapht-1-yl)-5-(2,6-diméthoxyphényl)pyrazole-3-carboxylique.

On prépare ce composé selon le mode opératoire décrit à l'étape A de l'EXEMPLE 1, à partir de 0,5 g du

composé obtenu à la Préparation 4.3 et 0,32 ml de chlorure de thionyle. On obtient 0,47 g de produit attendu et on l'utilise tel quel à l'étape suivante.

B) Acide 2-[1-(4-cyanonapht-1-yl)-5-(2,6-diméthoxyphényl)pyrazol-3-ylcarbonylamino]adamantane-2-carboxylique.

On prépare ce composé selon le mode opératoire décrit à l'étape B de l'EXEMPLE 1 à partir de 0,47 g du chlorure d'acide de l'étape précédente et de 0,25 g de l'acide 2-aminoadamantane-2-carboxylique. Après évaporation sous vide, on reprend le résidu au DCM et filtre le précipité formé. On chromatographie sur silice H en éluant par un mélange DCM/AcOEt/AcOH (95/4,5/0,5 ; v/v/v). On obtient 0,152 g du produit attendu après cristallisation dans l'éther, F = 290°C.

EXEMPLE 6

Acide 2-{1-[4-(hydroxyiminocarboxamido)napht-1-yl]-5-(2,6-diméthoxyphényl)pyrazol-3-ylcarbonylamino} adamantane-2-carboxylique.

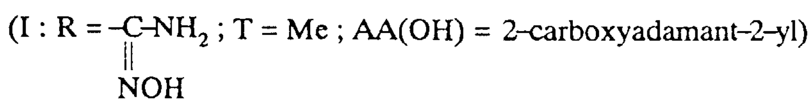

On ajoute une solution de 0,2 g de chlorhydrate d'hydroxylamine dans 10 ml de MeOH à une solution de 0,2 g du composé préparé à l'EXEMPLE 5 dans 20 ml d'EtOH; puis on introduit une solution de 0,2 g de carbonate de potassium dans 4 ml d'eau. On chauffe pendant 2 jours à reflux, puis concentre sous vide partiellement le mélange réactionnel. On obtient 0,15 g du produit attendu après ajout d'eau et filtration du précipité formé, F = 230°C.

EXEMPLE 7

Acide 2-[1-(4-carbamoylnapht-1-yl)-5-(2,6-diméthoxyphényl)pyrazol-3-ylcarbonylamino]adamantane-2-carboxylique.

(I : R = $-CONH_2$ ; T = Me ; AA(OH) = 2-carboxyadamant-2-yl).

On chauffe pendant 2 jours à reflux un mélange de 0,2 g du composé préparé à l'EXEMPLE 5, 0,4 ml de peroxyde d'hydrogène (solution aqueuse à 33 %), 0,4 ml de NaOH 6N dans 25 ml d'EtOH 95. Puis on ajoute 0,4 ml de peroxyde d'hydrogène, 0,4 ml de NaOH 6N au milieu réactionnel et poursuit le reflux pendant 1 jour. Après filtration, on dilue le filtrat à l'eau et extrait au DCM. On acidifie la phase aqueuse à pH 2 par addition d'HCl concentré. On filtre le précipité formé et lave à l'eau. On obtient 0,128 g du produit attendu après cristallisation dans l'éther, F = 287°C.

Le composé de l'EXEMPLE 7 peut également être obtenu en suivant le procédé décrit ci-après.

Acide 2-[1-(4-carbamoylnapht-1-yl)-5-(2,6-diméthoxyphényl)pyrazol-3-ylcarbonylamino]adamantane-2-carboxylique.

On prépare ce composé selon le mode opératoire décrit à l'EXEMPLE 4 à partir de 0,45 g du composé obtenu à la Préparation 4.4, et de 0,22 g de l'acide 2-aminoadamantane-2-carboxylique. On purifie le composé par chromatographie sur silice en éluant par un mélange DCM/MeOH/AcOH (100/4/0,5 ; v/v/v) puis cristallisation dans l'éther. On obtient 0,3 g du produit attendu, F = 292°C.

EXEMPLE 8

2-[1-(4-carbamoylnapht-1-yl)-5-(2,6-diméthoxyphényl)pyrazol-3-ylcarbonylamino]adamantane-2-carboxylate de sodium.

(I: : R = $-CONH_2$ ; T = Me ; AA(OH) = 2-carboxyadamant-2-yl, sel de sodium).

On ajoute 0,05 g du composé préparé à l'EXEMPLE 7 à une solution de 0,005 g de carbonate de sodium dans 0,3 ml d'eau et 3 ml de MeOH. On abandonne 48 heures à -18°C. Après évaporation sous vide à siccité, on triture le résidu dans 3 ml de propanol-2. On obtient 0,04 g du produit attendu après filtration et séchage sous vide sur $P_2O_5$, F = 335°C (déc.).

EXEMPLE 9

2-[1-(4-carbamoylnapht-1-yl)-5-(2,6-diméthoxyphényl)pyrazol-3-ylcarbonylamino]adamantane-2-carboxylate de N-méthyl-D-glucamine.

(I : R = $-CONH_2$ ; T = Me ; AA(OH) = 2-carboxyadamant-2-yl, sel de N-méthyl-D-glucamine).

On ajoute 0,05 g du composé préparé à l'EXEMPLE 7 à une solution de 0,017 g de N-méthyl-D-glucamine dans 4 ml de MeOH ; puis on rajoute 8 ml d'éther. On abandonne 3 jours à -18°C. On obtient 0,04 g du produit attendu après filtration des cristaux formés et séchage sous vide sur $P_2O_5$, F = 170-172°C.

EXEMPLE 10

Acide (2S)-2-[1-(4-carbamoylnapht-1-yl)-5-(2,6-diméthoxyphényl)pyrazol-3-ylcarbonylamino]-2-cyclohexylacétique.

(I: R = $-CONH_2$ ; T = Me ; AA(OH) = a-carboxycyclohexylméthyl).

A) Ester méthylique de l'acide (2S)-2-[1-(4-carbamoylnapht-1-yl)-5-(2,6-diméthoxyphényl)pyrazol-3-ylcarbonylamino]-2-cyclohexylacétique.

(I : R = $-CONH_2$ ; T = Me ; AA(OH) = ester méthylique de l'$\alpha$-carboxycyclohexylméthyl).

On agite à TA pendant 1 heure 30, sous atmosphère d'azote, un mélange de 0,45 g du composé obtenu à la Préparation 4.4, 0,15 ml de triéthylamine et 0,15 ml de chloroformiate d'isobutyle dans 10 ml de DCM. Puis on ajoute lentement une solution de 0,224 g du chlorhydrate de l'ester méthylique de la (S)-cyclohexylglycine, 0,15 ml de triéthylamine dans 5 ml de DCM. On laisse 5 jours sous agitation à TA. Après filtration d'un insoluble, on lave le filtrat par une solution d'HCl 1N, sèche sur sulfate de sodium et évapore sous vide. On obtient 0,37 g du produit attendu après recristallisation dans MeCN, F = 198°C.

B) Acide (2S)-2-[1-(4-carbamoylnapht-1-yl)-5-(2,6-diméthoxyphényl)pyrazol-3-ylcarbonylamino]-2-cyclohexylacétique.

On ajoute à TA une solution de 0,081 g de KOH dans 1 ml d'eau à une solution de 0,33 g du composé obtenu à l'étape précédente dans 5 ml de dioxane et laisse 5 heures sous agitation à TA. Puis on évapore sous vide le mélange réactionnel et reprend le résidu à l'eau. On acidifie à pH = 1 par addition d'une solution d'HCl 1N. On obtient 0,28 g du produit attendu après filtration, lavage à l'eau et séchage, F = 186°C.

$\alpha_D$ = +4° (c = 0,5 ; EtOH).

EXEMPLE 11

Acide 2-{1-[4-(acétylaminométhyl)napht-1-yl]-5-(2,6-diméthoxyphényl)pyrazol-3-ylcarbonylamino}adamantane-2-carboxylique.

(I : R = $-CH_2NHCOMe$ ; T = Me ; AA(OH) = 2-carboxyadamant-2-yl).

A) Chlorure de l'acide 1-[4-(acétylaminométhyl)napht-1-yl]-5-(2,6-diméthoxyphényl)pyrazole-3-carboxylique.

On prépare ce composé selon le mode opératoire décrit à l'étape A de l'EXEMPLE 1, à partir de 0,55 g du composé obtenu à la Préparation 4.5 et 0,31 ml de chlorure de thionyle. On utilise le produit obtenu tel quel à l'étape suivante.

B) Acide 2-{1-[4-(acétylaminométhyl)napht-1-yl]-5-(2,6-diméthoxyphényl)-pyrazol-3-ylcarbonylamino}adamantane-2-carboxylique.

On ajoute à TA, sous atmosphère d'azote, une solution du chlorure d'acide préparé à l'étape précédente dans 7 ml de DCM à un mélange de 0,29 g de l'acide 2-aminoadamantane-2-carboxylique et 15 ml de pyridine. On laisse 72 heures sous agitation à TA. Puis on filtre un insoluble et évapore sous vide le filtrat. On extrait le résidu au DCM, lave par un tampon pH = 2, sèche sur sulfate de sodium et évapore sous vide le solvant. Puis on chromatographie sur silice H en éluant avec le mélange DCM/MeOH (100/4 ; v/v). On obtient 0,15 g du produit attendu après cristallisation dans l'éther, F = 211°C.

EXEMPLE 12

Acide 2-{5-(2,6-diméthoxyphényl)-1-[4-(diméthylaminosulfonyl)napht-1-yl]pyrazol-3-ylcarbonylamino}adamantane-2-carboxylique.

(I : R = $-SO_2N(Me)_2$ ; T = Me ; AA(OH) = 2-carboxyadamant-2-yl).

A) Chlorure de l'acide 5-(2,6-diméthoxyphényl)-1-[4-(diméthylaminosulfonyl)-napht-1-yl]pyrazole-3-carboxylique.

On prépare ce composé selon le mode opératoire décrit à l'étape A de l'EXEMPLE 1, à partir de 1,46 g du composé obtenu à la Préparation 4.6 et 0,77 ml de chlorure de thionyle. On utilise immédiatement le produit obtenu à l'étape suivante.

B) Acide 2-{5-(2,6-diméthoxyphényl)-1-[4-(diméthylaminosulfonyl)napht-1-yl]-pyrazol-3-ylcarbonylamino}adamantane-2-carboxylique.

On prépare ce composé selon le mode opératoire décrit à l'étape B de l'EXEMPLE 11, à partir du composé obtenu à l'étape précédente et 0,7 g d'acide 2-aminoadamantane-2-carboxylique. On purifie le produit par chromatographie sur silice H en éluant avec le mélange DCM/AcOEt/AcOH (90/10/0,5 . v/v/v). On obtient 0,6 g du produit attendu après cristallisation dans l'hexane, F = 269°C.

EXEMPLE 13

Chlorhydrate de l'acide 2-{5-(2,6-diméthoxyphényl)-1-{4-[N-méthyl-N-(3-N',N'-diméthylaminopropyl)aminosulfonyl]napht-1-yl}pyrazol-3-ylcarbonylamino} adamantane-2-carboxylique.

(I: R = $-SO_2N(Me)(CH_2)_3N(Me)_2$; T = Me ; AA(OH) = 2-carboxyadamant-2-yl).

On prépare ce composé selon le mode opératoire décrit à l'EXEMPLE 4, à partir de 0,37 g du composé obtenu à la Préparation 4.7 et 0,13 g de l'acide 2-aminoadamantane-2-carboxylique. On purifie le produit par chromatographie sur silice H en éluant avec le mélange DCM/MeOH/AcOH (100/8/1 ; v/v/v). On obtient 0,11 g du produit attendu après cristallisation dans l'éther, F = 246°C (déc.).

Le composé de l'EXEMPLE 13 peut également être obtenu en suivant les 2 étapes du procédé décrit ci-après.

A') Chlorure de l'acide 5-(2,6-diméthoxyphényl)-1-{4-[N-méthyl-N-(3-N',N'-diméthylaminopropyl)aminosulfonyl] napht-1-yl}pyrazole-3-carboxylique.

On agite pendant 3 heures, sous atmosphère d'azote, à TA, un mélange de 0,2 g du composé obtenu à la Préparation 4.7 et 2 ml de chlorure de thionyle. Puis on rajoute 2 ml de chlorure de thionyle et poursuit l'agitation à TA pendant 1 heure 30. On évapore sous vide le mélange réactionnel, reprend le résidu par du DCM et évapore sous vide à nouveau. Le chlorure d'acide ainsi obtenu est utilisé tel quel à l'étape suivante.

B') Acide 2-{5-(2,6-diméthoxyphényl)-1-{4-[N-méthyl-N-(3-N',N'-diméthylaminopropyl)aminosulfonyl]napht-1-yl} pyrazol-3-ylcarbonylamino} adamantane-2-carboxylique.

On chauffe à reflux pendant 15 minutes, sous atmosphère d'azote, un mélange de 0,071 g d'acide 2-aminoadamantane-2-carboxylique, 0,36 ml de bis(triméthylsilyl) acétamide dans 10 ml de MeCN. Après refroidissement, on ajoute une solution du chlorure d'acide obtenu à l'étape précédente dans 10 ml de DCM et laisse 48 heures sous agitation à TA. On ajoute de l'eau, acidifie à pH = 2 par ajout d'HCl, 1,2N, extrait au DCM, sèche sur sulfate de sodium et évapore sous vide. On obtient 0,23 g du produit attendu.

EXEMPLE 14

Acide 2-{1-[4-(carbamoylméthyl)napht-1-yl]-5-(2,6-diméthoxyphényl)pyrazol-3-ylcarbonylamino}adamantane-2-carboxylique.

(I : R = $-CH_2CONH_2$ ; T = Me ; AA(OH) = 2-carboxyadamant-2-yl).

On prépare ce composé selon le mode opératoire décrit à l'EXEMPLE 4, à partir de 0,8 g du composé obtenu à la Préparation 4.8 et 0,4 g d'acide 2-aminoadamantane-2-carboxylique. Après évaporation du mélange réactionnel on ajoute sur le résidu une solution d'HCl 1N et de l'EtOH. Après agitation, on filtre le précipité formé et sèche ce dernier sous vide. Puis on chromatographie sur silice H en éluant avec le mélange DCM/MeOH/AcOH (100/4/0,5 ; v/v/v). On dissout le produit obtenu dans une solution de NaOH 1N et d'EtOH, acidifie à pH = 1 par addition d'HCl 1N, extrait au DCM, sèche sur sulfate de sodium et évapore sous vide le solvant. On obtient 0,53 g du produit attendu après cristallisation dans l'EtOH, F = 224°C.

EXEMPLE 15

Acide 2-{1-[4-(carboxyméthyl)napht-1-yl]-5-(2,6-diméthoxyphényl)pyrazol-3-ylcarbonylamino}adamantane-2-carboxylique.

(I: R = $-CH_2CO_2H$ ; T = Me ; AA(OH) = 2-carboxyadamant-2-yl).

On chauffe à 60°C pendant 1 jour un mélange de 0,17 g du composé obtenu à l'EXEMPLE 14, 0,135 g de peroxyde de sodium dans 5 ml d'eau et quelques gouttes de MeOH. On acidifie à pH = 1 par addition d'HCl 1N et laisse sous agitation. Après filtration, on obtient le produit attendu par cristallisation dans MeCN, m = 0,1 g, F = 267°C.

EXEMPLE 16

Acide 2-{5-(2,6-diméthoxyphényl)-1-[4-(3-N,N-diméthylaminopropoxy)napht-1-yl]pyrazol-3-ylcarbonylamino}adamantane-2-carboxylique.

(I : R = $-O(CH_2)_3N(Me)_2$ ; T = Me ; AA(OH) = 2-carboxyadamant-2-yl).

On prépare ce composé selon le mode opératoire décrit à l'EXEMPLE 4 à partir de 0,19 g du composé obtenu à la Préparation 4.9, et de 0,14 g de l'acide 2-aminoadamantane-2-carboxylique. On purifie le composé par chromatographie sur silice en éluant par un mélange DCM/MeOH/$NH_4OH$ (100/15/1 ; v/v/v). On obtient 0,04 g du produit attendu, F = 200°C.

EXEMPLE 17

Acide 2-{1-[4-(carbamoylméthoxy)napht-1-yl]-5-(2,6-diméthoxyphényl)-pyrazol-3-ylcarbonylamino}adamantane-2-carboxylique.
(I : R = $-OCH_2CONH_2$ ; T = Me ; AA(OH) = 2-carboxyadamant-2-yl).
On prépare ce composé selon le mode opératoire décrit à l'EXEMPLE 4, à partir de 0,31 g du composé obtenu à la Préparation 4.10 et de 0,27 g de l'acide 2-aminoadamantane-2-carboxylique. On purifie le composé par chromatographie sur silice H en éluant par le mélange DCM/MeOH/AcOH (100/1/0,5 ; v/v/v). On obtient 0,14 g de produit attendu, F = 200°C.

EXEMPLE 18

Acide 2-{5-(2,6-diméthoxyphényl)-1-[4-[N-[3-(N',N'-diméthylamino)propyl] carbamoyl]napht-1-yl]pyrazol-3-ylcarbonylamino}adamantane-2-carboxylique.
(I: : R = $-CONH(CH_2)_3N(Me)_2$ ; T = Me ; AA(OH) = 2-carboxyadamant-2-yl).
On chauffe à 35°C pendant 1 heure un mélange de 2 g du composé obtenu à la Préparation 4.11, 10 ml de chlorure de thionyle dans 30 ml de DCM. On concentre sous vide et utilise tel quel le chlorure d'acide ainsi obtenu. D'autre part, on chauffe à reflux pendant 1 heure un mélange de 1,2 g d'acide 2-aminoadamantane-2-carboxylique, 3 ml de bis(triméthylsilyl)acétamide, dans 70 ml de MeCN. Après refroidissement à TA, on ajoute une solution du chlorure d'acide préparé ci-dessus dans 50 ml de DCM puis 0,57 ml de triéthylamine et laisse 48 heures sous agitation à TA. On concentre sous vide le mélange réactionnel, reprend le résidu à l'eau, acidifie à pH = 2 par ajout d'HCl 1N, laisse 1 heure sous agitation et essore le précipité formé. On dissout le précipité dans l'eau chaude, ajoute une solution à 5 % de NaOH jusqu'à pH = 6, extrait au DCM, sèche la phase organique sur $Na_2SO_4$ et évapore sous vide le solvant. On obtient 0,48 g du produit attendu après cristallisation dans le propan-2-ol et recristallisation dans le MeOH, F = 244°C.
Spectre de RMN à 200 MHz dans DMSO.

1,4 à 2,1 ppm : m : 14H
2,2 ppm : s : 6H
2,4 ppm : t : 2H
2,55 ppm : m : 2H
3,4 ppm : qd : 2H
3,6 ppm : s : 6H
6,5 ppm : d : 2H
6,9 ppm : s : 1H
7,2 ppm : t : 1H
7,4 à 7,8 ppm : m : 6H
8,2 ppm : m : 1H
8,7 ppm : t : 1H

EXEMPLE 19

p-Toluènesulfonate de l'acide 2-{5-(2,6-diméthoxyphényl)-1-[4-[N-[3-(N',N'-diméthylamino)propyl]carbamoyl] napht-1-yl]pyrazol-3-ylcarbonylamino}adamantane-2-carboxylique.
A une solution, refroidie à +5°C, de 0,4 g du composé obtenu à la Préparation 4.11, 0,127 ml de triéthylamine dans 10 ml de DCM, on ajoute 0,130 ml de chloroformiate d'isobutyle et on laisse deux jours sous agitation à TA. D'autre part, on chauffe à reflux pendant 1 heure et 30 minutes un mélange de 0,231 g de l'acide 2-aminoadamantane-2-carboxylique, 0,87 ml de bis(triméthylsilyl)acétamide dans 15 ml de MeCN. Après refroidissement à TA, on ajoute la solution d'anhydride mixte préparée ci-dessus et laisse deux jours sous agitation à TA. On ajoute de l'eau au mélange réactionnel, acidifie à pH = 2 par ajout d'HCl 1N, laisse sous agitation et concentre sous vide. On reprend le résidu à l'eau, ajoute une solution à 5 % de NaOH jusqu'à pH = 6,5, extrait au DCM, filtre un insoluble, sèche le filtrat sur $Na_2SO_4$ et évapore sous vide le solvant. On obtient 0,23 g du produit brut. On dissout 0,11 g du produit ainsi obtenu dans un minimum de MeCN, ajoute 0,032 g d'acide p-toluènesulfonique monohydrate et ajoute de l'éther jusqu'à précipitation. On obtient 0,050 g du produit attendu après essorage et séchage.

Spectre de RMN à 200 MHz dans DMSO.

1,6 à 2,2 ppm : m : 14H
2,4 ppm : s : 3H
2,5 ppm : m : 2H
2,9 ppm : s : 6H
3,2 ppm : t : 2H
3,3 à 3,8 ppm : m : 8H
6,5 ppm : d : 2H
6,9 ppm : s : 1H
7,2 ppm : d : 3H
7,4 à 7,8 ppm : m : 7H
8,2 ppm : m : 1H
8,7 ppm : t : 1H

EXEMPLE 20

Acide 2-{5-(2,6-diméthoxyphényl)-1-[4-[N- [2-(N',N'-diméthylamino)éthyl]carbamoyl]napht-1-yl]pyrazol-3-yl-carbonylamino}adamantane-2-carboxylique.

(I : R = $-CONH(CH_2)_2N(Me)_2$ ; T = Me ; AA(OH) = 2-carboxyadamant-2-yl).

On chauffe à 35°C pendant 1 heure un mélange de 2 g du composé obtenu à la Préparation 4.12, 10 ml de chlorure de thionyle dans 30 ml de DCM. On concentre sous vide et utilise tel quel le chlorure d'acide ainsi obtenu. D'autre part, on chauffe à reflux pendant 1 heure un mélange de 1,2 g d'acide 2-aminoadamantane-2-carboxylique, 3 ml de bis(triméthylsilyl)acétamide dans 70 ml de MeCN. Après refroidissement à TA, on ajoute une solution du chlorure d'acide préparé ci-dessus dans 50 ml de DCM, puis 0,58 ml de triéthylamine et laisse une nuit sous agitation à TA. On concentre sous vide le mélange réactionnel, reprend le résidu à l'eau (cristallisation du produit), acidifie à pH = 2 par ajout d'HCl 1N et laisse 1 heure sous agitation. On essore le produit cristallisé et, après séchage, on chromatographie ce dernier sur silice H en éluant par le gradient du mélange DCM/MeOH/$H_2O$ (100/5/0,2 ; v/v/v) jusqu'à (100/10/0,75 ; v/v/v). On obtient 0,62 g du produit attendu après cristallisation dans l'acétone.

Spectre de RMN à 200 MHz dans DMSO.

1,4 à 2,2 ppm : m : 12H
2,6 ppm : m : 2H
2,8 ppm : s : 6H
3,3 ppm : t : 2H
3,45 ppm : s : 6H
3,7 ppm : t : 2H
6,4 ppm : d : 2H
6,9 ppm : s : 1H
7,2 ppm : t : 1H
7,4 ppm : d : 1H
7,6 ppm : m : 3H
7,7 ppm : d : 1H
8,2 ppm : m : 1H
8,4 ppm : t : 1H

On prépare également ce composé selon le mode opératoire décrit ci-après.

A une solution, refroidie à +5°C, de 0,44 g du composé obtenu à la Préparation 4.12, 0,140 ml de triéthylamine dans 10 ml de DCM, on ajoute 0,139 ml de chloroformiate d'isobutyle et laisse 3 jours sous agitation à TA.

D'autre part, on chauffe à reflux pendant 1 heure et 30 minutes un mélange de 0,321 g de l'acide 2-aminoadamantane-2-carboxylique, 0,49 ml de bis(triméthylsilyl)acétamide dans 20 ml de MeCN. Après refroidissement à TA, on ajoute la solution d'anhydride mixte préparée ci-dessus et laisse 3 jours sous agitation à TA. On ajoute une solution d'HCl 1N jusqu'à pH = 2, puis du MeOH et concentre sous vide. On reprend le résidu par une solution saturée de NaCl, extrait au DCM, sèche la phase organique sur $Na_2SO_4$ et évapore sous vide le solvant. On reprend le résidu à l'eau, ajoute une solution d'ammoniaque jusqu'à pH = 7-8, essore le précipité formé et sèche. On chromatographie le précipité sur silice H en éluant par le mélange DCM/MeOH/$H_2O$ (80/10/0,75 ; v/v/v) puis par le mélange DCM/MeOH/$H_2O$ (80/15/2 ; v/v/v). On obtient 0,04 g du produit attendu.

EXEMPLE 21

Acide 2-{1-[4-[N-(cyanométhyl)carbamoyl]napht-1-yl]-5-(2,6-diméthoxyphényl)pyrazol-3-ylcarbonylamino} adamantane-2-carboxylique.

(I : R = $-CONHCH_2CN$ ; T = Me ; AA(OH) = 2-carboxyadamant-2-yl).

A une solution de 0,55 g du composé obtenu à la Préparation 4.13, 0,176 ml de triéthylamine dans 10 ml de 1,4-dioxane, on ajoute 0,181 ml de chloroformiate d'isobutyle et laisse 6 jours sous agitation à TA. D'autre part, on chauffe à reflux pendant 1 heure et 30 minutes un mélange de 0,351 g d'acide 2-aminoadamantane-2-carboxylique, 0,106 ml de bis(triméthylsilyl)acétamide dans 20 ml de MeCN. Après refroidissement à TA, on ajoute la solution d'anhydride mixte préparée ci-dessus et laisse 3 jours sous agitation à TA. On ajoute ensuite une solution d'HCl 1N jusqu'à pH = 2 et laisse 2 heures sous agitation à TA. On concentre sous vide, reprend le résidu par une solution saturée de NaCl, extrait au DCM et filtre un insoluble. Après décantation, on sèche la phase organique sur $Na_2SO_4$ et évapore sous vide le solvant. On chromatographie le résidu sur silice H en éluant par le mélange DCM/MeOH/$H_2O$ (80/5/0,3 ; v/v/v). On obtient 0,080 g du produit attendu.

Spectre de RMN à 200 MHz dans DMSO.

1,4 à 2,3 ppm : m : 12H
2,6 ppm : m : 2H
3,5 ppm : s : 6H
4,0 ppm : s : 2H
6,4 ppm : d : 2H
6,9 ppm : s : 1H
7,2 ppm : t : 1H
7,4 à 7,8 ppm : 5H
8,4 ppm : m : 1H
8,8 ppm : t : 1H

EXEMPLE 22

Acide 2-{1-[4-[N-(2-cyanoéthyl)-N-méthylcarbamoyl]napht-1-yl]-5-(2,6-diméthoxyphényl)pyrazol-3-ylcarbonylamino}adamantane-2-carboxylique.

(I : R = $-CON(Me)CH_2CH_2CN$ ; T = Me ; AA(OH) = 2-carboxyadamant-2-yl).

On chauffe à 35°C pendant 2 heures un mélange de 4 g du composé obtenu à la Préparation 4.14, 40 ml de chlorure de thionyle dans 40 ml de DCM. On concentre sous vide et utilise tel quel le chlorure d'acide ainsi obtenu. D'autre part, on chauffe à reflux pendant 1 heure un mélange de 2,34 g d'acide 2-aminoadamantane-2-carboxylique, 6 ml de bis(triméthylsilyl)acétamide dans 150 ml de MeCN. Après refroidissement à TA, on ajoute une solution du chlorure d'acide préparé ci-dessus dans 100 ml de DCM, puis 1,12 ml de triéthylamine et laisse une nuit sous agitation à TA. On concentre sous vide, reprend le résidu à l'eau, ajoute une solution d'HCl 1N jusqu'à pH = 2, laisse sous agitation et essore le solide formé. Après séchage, on chromatographie le solide sur silice H en éluant par le mélange DCM/MeOH/$H_2O$ (100/10/1 ; v/v/v). On obtient le produit attendu après cristallisation dans l'acétone, F = 264°C.

EXEMPLE 23

Acide 2-{5-(2,6-diméthoxyphényl)-1-[4-[N-méthyl-N-[2-($N^1$-méthyl-$N^2$-méthylamidino)éthyl]carbamoyl] napht-1-yl]pyrazol-3-ylcarbonylamino}adamantane-2-carboxylique.

(I : R = $-CON(Me)CH_2CH_2C(=NMe)NHMe$; T = Me ; AA(OH) = 2-carboxyadamant-2-yl).

On dissout 1 g du composé obtenu à l'EXEMPLE 22 dans 30 ml d'EtOH et 10 ml d'éther puis on fait barboter pendant 2 heures de l'HCl gaz. On abandonne une nuit à +5°C et concentre sous vide. On reprend le résidu dans 50 ml d'EtOH et fait barboter, pendant 2 heures à TA, de la méthylamine gaz. On concentre sous vide, reprend le résidu dans un mélange EtOH/éther, filtre le chlorhydrate de méthylamine qui a cristallisé et évapore sous vide le filtrat. On reprend le résidu à l'eau, essore les cristaux formés et sèche. On obtient 0,7 g du produit attendu après recristallisation dans l'EtOH, F = 235°C.

Spectre de RMN à 200 MHz dans DMSO.

1,4 à 2,4 ppm : m : 12H
2,5 à 4,0 ppm : m : 21H
6,5 ppm : d : 2H
6,9 ppm : s : 1H

7,15 ppm : t : 1H
7,3 à 7,8 ppm : m : 6H

EXEMPLE 24

Acide 2-{5-(2,6-diméthoxyphényl)-1-[4-($N^1$-méthyl-$N^2$-méthylamidino)-napht-1-yl]pyrazol-3-ylcarbonylamino}adamantane-2-carboxylique.
(I : R = -C(=NMe)NHMe ; T = Me ; AA(OH) = 2-carboxyadamant-2-yl).

A) Acide 2-{5-(2,6-diméthoxyphényl)-1-[4-(N-méthylcarbamoyl)napht-1-yl] pyrazol-3-ylcarbonylamino}adamantane-2-carboxylique.

On prépare ce composé selon le mode opératoire décrit à l'EXEMPLE 22 à partir de 2 g du composé obtenu à la Préparation 4.15, 25 ml de chlorure de thionyle puis 1,39 g d'acide 2-aminoadamantane-2-carboxylique, 3,5 ml de bis(triméthylsilyl) acétamide et 0,64 ml de triéthylamine. On chromatographie sur silice H en éluant par le mélange DCM/MeOH/$H_2O$ (100/3/0,2 v/v/v) puis (100/5/0,4 ; v/v/v). On obtient 1,2 g du produit attendu, F = 170°C.

B) Acide 2-{5-(2,6-diméthoxyphényl)-1-[4-($N_1$-méthyl-$N_2$-méthylamidino)napht-1-yl] pyrazol-3-ylcarbonylamino} adamantane-2-carboxylique.

On chauffe à reflux pendant 20 minutes un mélange de 0,5 g du composé obtenu à l'étape précédente, 0,34 g de pentachlorure de phosphore dans 15 ml de toluène. Après refroidissement on essore le solide cristallisé, reprend les cristaux dans 30 ml d'EtOH et fait barboter de la méthylamine gaz dans la solution. On concentre sous vide et chromatographie le résidu sur silice H en éluant par le mélange DCM/MeOH/$H_2O$ (100/2/0,2 ; v/v/v). On obtient 0,18 g du produit attendu, F = 180°C.

EXEMPLE 25

Acide 2-{1-[4-(2,6-acétamidohexanoylamino)napht-1-yl]-5-(2,6-diméthoxy phényl)pyrazol-3-ylcarbonylamino}adamantane-2-carboxylique.
(I : R = $-NHCO(CH_2)_5NHCOMe$ ; T = Me ; AA(OH) = 2-carboxyadamant-2-yl).
A une solution, refroidie à 5°C, de 0,4 g du composé obtenu à la Préparation 4.16, 0,2 ml de triéthylamine dans 5 ml de MeCN, on ajoute 0,12 ml de chloroformiate d'isobutyle et laisse 2 heures sous agitation à TA. D'autre part, on laisse 2 heures sous agitation à TA un mélange de 0,21 g d'acide 2-aminoadamantane-2-carboxylique, 2 ml de bis(triméthylsilyl)acétamide dans 3 ml de MeCN. Puis on ajoute cette solution à la solution d'anhydride mixte préparée ci-dessus et laisse 16 jours sous agitation à TA. On ajoute ensuite 5 ml de MeOH et 1 ml d'eau au mélange réactionnel, laisse 30 minutes sous agitation et concentre sous vide. On reprend le résidu dans 100 ml de DCM, filtre un insoluble, lave le filtrat à l'eau, par une solution d'HCl 1N, sèche la phase organique sur $Na_2SO_4$ et évapore sous vide. On chromatographie le résidu sur silice H en éluant par le mélange DCM/MeOH/AcOH (100/8/1 ; v/v/v). On obtient 0,07 g du produit attendu après cristallisation dans l'éther, F = 190°C.

EXEMPLE 26

Acide 2-{1-[4-(2-aminoéthyl)napht-1-yl]-5-(2,6-diméthoxyphényl)pyrazol-3-ylcarbonylamino}adamantane-2-carboxylique.
(I: R = $-CH_2CH_2NH_2$ ; T = Me ; AA(OH) = 2-carboxyadamant-2-yl).

A) Acide 2-{1-[4-(cyanométhyl)napht-1-yl]-5-(2,6-diméthoxyphényl)pyrazol-3-ylcarbonylamino}adamantane-2-carboxylique.

On chauffe à 40°C pendant 1 heure un mélange de 5 g du composé obtenu à la Préparation 4.8, 50 ml de chlorure de thionyle dans 125 ml de DCM puis concentre sous vide. D'autre part, on chauffe à reflux pendant 1 heure un mélange de 3,38 g d'acide 2-aminoadamantane-2-carboxylique, 8,75 ml de bis(triméthylsilyl)acétamide dans 150 ml de MeCN. Après refroidissement à TA, on ajoute une solution du chlorure d'acide préparé ci-dessus dans 100 ml de DCM puis 1,6 ml de triéthylamine et laisse une nuit sous agitation à TA. On concentre sous vide, dissout le résidu dans l'EtOH et ajoute de l'éther jusqu'à cristallisation. Après essorage des cristaux (composé de l'EXEMPLE 14), on concentre sous vide le filtrat. On reprend le résidu dans l'EtOH, ajoute une solution d'HCl 3N jusqu'à pH = 1, essore le précipité formé et sèche. On chromatographie le précipité sur silice H en éluant par la mélange DCM/MeOH/AcOH (100/3/0,5 ; v/v/v). On sépare le composé de l'EXEMPLE 14 et ensuite on obtient 1,2 g du produit attendu.

B) Acide 2-{1-[4-(2-aminoéthyl)napht-1-yl]-5-(2,6-diméthoxyphényl)pyrazol-3-ylcarbonylamino}adamantane-2-carboxylique.

On hydrogène à TA et à pression atmosphérique un mélange de 1,2 g du composé obtenu à l'étape précédente, 0,12 g de nickel de Raney®, 60 ml de MeOH et 10 ml d'ammoniaque concentrée. On filtre le catalyseur et concentre sous vide en partie le filtrat. On essore les cristaux formés et sèche. On obtient 0,89 g du produit attendu, F = 250°C.

Spectre de RMN à 200 MHz dans DMSO.

1,4 à 2,2 ppm : m : 12H
2,5 ppm : m : 2H
3,0 à 3,6 ppm : m : 10H
6,4 ppm : d : 2H
6,9 ppm : s : 1H
7,1 ppm : t : 1H
7,2 à 7,6 ppm : m : 5H
8,1ppm : m : 1H

EXEMPLE 27

Chlorhydrate de l'acide 2-{5-(2,6-diméthoxyphényl)-1-[4-[N-méthyl-N-[3-(N',N'-diméthylamino)propyl]carbamoyl]napht-1-yl]pyrazol-3-ylcarbonylamino} adamantane-2-carboxylique.
(I: R = $-CON(Me)(CH_2)_3N(Me)_2$; T = Me ; AA(OH) = 2-carboxyadamant-2-yl).
On prépare ce composé selon le mode opératoire décrit à l'EXEMPLE 18, à partir de 0,32 g du composé obtenu à la Préparation 4.17, 2 ml de chlorure de thionyle, 5 ml de DCM puis 0,185 g de l'acide 2-aminoadamantane-2-carboxylique, 0,465 ml de bis(triméthylsilyl)acétamide, 10 ml de MeCN et 0,09 ml de triéthylamine. On dissout le produit brut dans un minimum d'EtOH, ajoute une solution saturée d'HCl dans l'éther et concentre sous vide. On obtient 0,1 g du produit attendu après cristallisation dans l'éther.

EXEMPLE 28

Chlorhydrate de l'acide 2-{5-(2,6-diméthoxyphényl)-1-[4-[N-méthyl-N-[2-(N',N'-diméthylamino)éthyl]carbamoyl]napht-1-yl]pyrazol-3-ylcarbonylamino}adamantane-2-carboxylique.
(I : R = $-CON(Me)(CH_2)_2N(Me)_2$; T = Me ; AA(OH) = 2-carboxyadamant-2-yl).
On prépare ce composé selon le mode opératoire décrit à l'EXEMPLE 18, à partir de 0,31 g du composé obtenu à la Préparation 4.18, 2 ml de chlorure de thionyle, 5 ml de DCM, puis 0,185 g d'acide 2-aminoadamantane-2-carboxylique, 0,465 ml de bis(triméthylsilyl)acétamide, 10 ml de MeCN et 0,09 ml de triéthylamine. On dissout le produit brut dans un minimum d'EtOH, ajoute une solution saturée d'HCl dans l'éther et concentre sous vide. On obtient 0,08 g du produit attendu après cristallisation dans l'éther.

EXEMPLE 29

Acide 2-{5-(2,6-diméthoxyphényl)-1-[1,3(2H)-dioxo-1*H*-benz[de]isoquinol-6-yl]pyrazol-3-ylcarbonylamino} adamantane-2-carboxylique

(I : 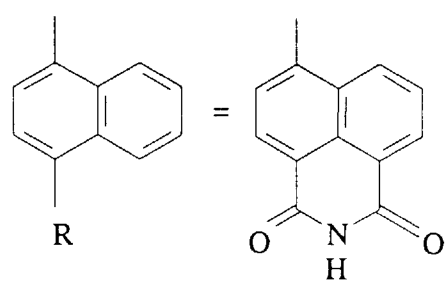 ; T = Me ; AA(OH) = 2-carboxyadamant-2-yl)

A une solution refroidie à 5°C de 1,4 g du composé obtenu à la Préparation 4.19, 0,28 ml de pyridine dans 20 ml de MeCN, on ajoute 0,43 ml de chloroformiate d'isobutyle et on laisse 1 heure sous agitation à TA. D'autre part, on chauffe à reflux pendant 1 heure un mélange de 0,64 g d'acide 2-aminoadamantane-2-carboxylique, 1,54 ml de bis(triméthylsilyl) acétamide dans 30 ml de MeCN. Après refroidissement à TA, on ajoute la solution d'anhydride mixte préparée ci-dessus et laisse trois jours sous agitation à TA et sous atmosphère d'azote. On acidifie le mélange réactionnel à pH = 2 par ajout d'HCl 1N et concentre sous vide. On reprend le résidu à l'eau, extrait à l'AcOEt, sèche sur $Na_2SO_4$ et évapore sous vide le solvant. On chromatographie le résidu sur silice H en éluant par le mélange DCM/ MeOH/AcOH (100/1/0,5 ; v/v/v). On reprend le produit obtenu dans du MeOH, on alcalinise par ajout de NaOH concentrée et concentre sous vide. On reprend le produit sous forme de sel de sodium dans du MeOH, acidifie à pH = 1 par ajout d'HCl 1N et essore le précipité formé. On obtient 0,17 g du produit attendu, F = 210°C.

EXEMPLE 30

Acide 2-{5-(2,6-diméthoxyphényl)-1-[2-acétamido-1,3(2*H*)-dioxo-1*H*-benz[de]isoquinol-6-yl]pyrazol-3-ylcarbonylamino}adamantane-2-carboxylique.

(I : 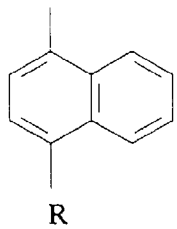 = 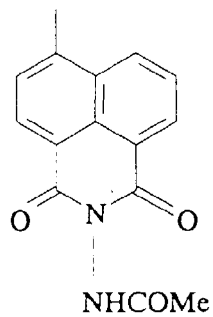 ; T = Me ; AA(OH) = 2-carboxyadamant-2-yl)

On prépare ce composé selon le mode opératoire décrit à l'EXEMPLE 29 à partir de 0,9 g du composé obtenu à la Préparation 4.21, 0,25 ml de triéthylamine, 0,25 ml de chloroformiate d'isobutyle et 15 ml de DCM puis 0,36 g d'acide 2-aminoadamantane-2-carboxylique, 0,89 ml de bis(triméthylsilyl)acétamide et 20 ml de MeCN. On chromatographie le produit brut sur silice H en éluant par le mélange DCM/MeOH/AcOH (100/3/0,5 ; v/v/v). On dissout le produit obtenu dans NaOH 1N, acidifie à pH = 3 par ajout d'HCl 1N, extrait à l'AcOEt, sèche sur $Na_2SO_4$ et évapore sous vide le solvant. On obtient 0,45 g du produit attendu après cristallisation dans l'éther, F = 292°C.

En procédant selon le mode opératoire décrit à l'EXEMPLE 7 (2ème procédé), à partir du composé obtenu à la Préparation 4.4, on prépare les composés selon l'invention rassemblés dans le TABLEAU 1 ci-après.

TABLEAU 1

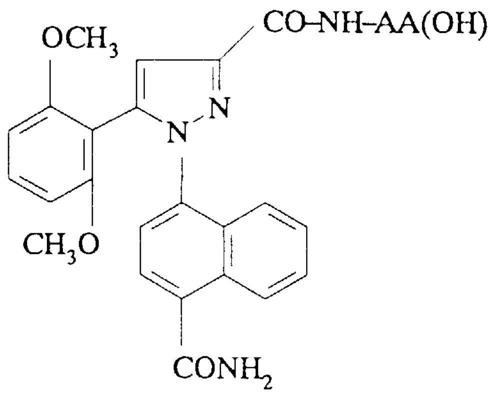

| EXEMPLES | -NH-AA(OH) |
|---|---|
| 31 | 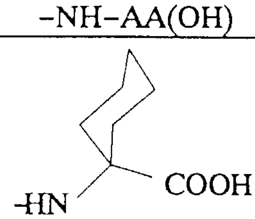 |
| 32 | 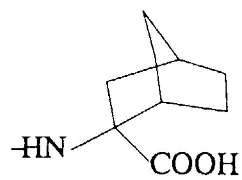 |
| 33 | 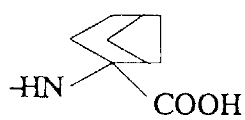 |
| 34 | 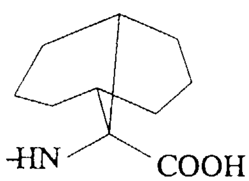 |

En procédant selon le mode opératoire décrit à l'EXEMPLE 10, à partir du composé obrenu à la Préparation 4.4, on prépare les composés selon l'invention rassemblés dans le tableau 2 ci-après.

TABLEAU 2

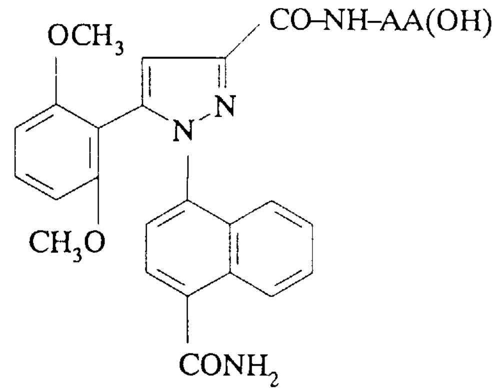

| EXEMPLES | –NH–AA(OH) |
|---|---|
| 35 | 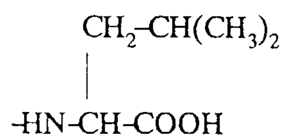 |
| 36 | 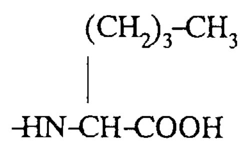 |
| 37 | 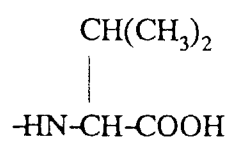 |
| 38 | 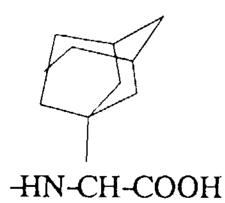 |
| 39 | 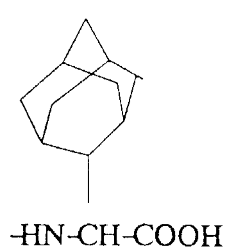 |

EXEMPLE 40

Chlorhydrate de l'acide 2-{5-(2,6-diméthoxyphényl)-1-[4-[N-[3-(N',N'-diméthylamino)propyl]carbamoyl]napht-1-yl]pyrazol-3-ylcarbonylamino}adamantane-2-carboxylique.

On prépare ce composé selon le mode opératoire décrit à l'exemple 18, à partir de 5,6g du composé obtenu à la préparation 4.11, 20ml de chlorure d'oxalyle et 20ml de DCM puis 3,4g d'acide 2-aminoadamantane-2-carboxylique, 8,4ml de MeCN et 1,6ml de triéthylamine. On obtient 1,3g du produit attendu sous forme de base après cristallisation dans le propan-2-ol et recristallisation dans le MeOH (F=242°C). On dissout 20mg du produit obtenu sous forme de base dans un minimum de MeOH, acidifie à pH=1 par ajout d'une solution saturée d'HCl dans l'éther et concentre sous vide. On obtient le chlorhydrate attendu après cristallisation dans le mélange acétone/pentane, F=200°C (déc.).

Spectre de RMN à 200 MHz dans DMSO

0,4 à 2,3 ppm : m : 14 H
2,6 ppm : se : 2 H
2,8 ppm : se : 6 H
3,15 ppm : se : 2 H
3,4 ppm : se : 2 H
3,55 ppm : s : 6 H
6,5 ppm : d : 2 H
6,9 ppm : s :1H
7,2 ppm : t : 1 H
7,3 à 8,3 ppm : m : 7 H
8,8 ppm : se : 1 H
10 à 13 ppm : 2se : 2 H

## Revendications

**1.** Composé de formule :

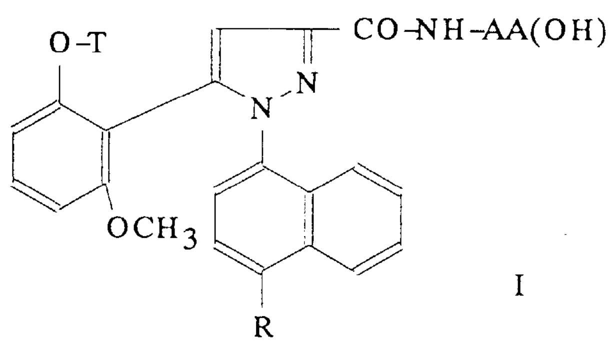

I

dans laquelle :

- R représente un groupe choisi parmi :
  $-CN$, $-C(NH_2) = N-OH$, $-C(NR_4R_5) = NR_6$,
  $-CONR_1R_2$, $-CON(R_7)(CH_2)_pNR_1R_2$,
  $-CON(R_7)(CH_2)_qCN$, $-CON(R_7)(CH_2)_qC(NR_{14}R_{15}) = N-R_{16}$,
  $-CH_2CN$, $-CH_2CONR_1R_2$, $CH_2CON(R_7)(CH_2)_pNR_1R_2$,
  $-CH_2CON(R_7)(CH_2)_qCN$, $-CH_2COOR_7$,
  $-O(CH_2)_nNR_1R_2$, $-O(CH_2)_nCONR_1R_2$,
  $-O(CH_2)_nCOOR_7$, $-O(CH_2)_nSO_2NR_1R_2$,
  $-N(R7)COR_3$, $-N(R_7)CO(CH_2)_nNR_1R_2$,
  $-N(R_7)CO(CH_2)_nNHCOR_3$, $-N(R_7)SO_2R_8$,
  $-N(R_7)CONR_9R_{10}$, $CH_2N(R_7)COR_3$,
  $-CH_2N(R_7)SO_2R_8$, $-CH_2CH_2NR_{11}R_{12}$,

$-CH_2CH_2N(R_7)COR_3$, $-CH_2CH_2N(R_7)SO_2R_8$,
$-SO_2NR_1R_2$, $-SO_2N(R_7)(CH_2)_nNR_1R_2$,
$-CH_2CON(R_7)(CH_2)_qC(NR_{14}R_{15}) = NR_{16}$
$-N(R_7)CO(CH_2)_qCN$, $-N(R_7)CO(CH_2)_qC(NR_{14}R_{15}) = NR_{16}$,
$-SO_2N(R_7)(CH_2)_qCN$, $SO_2N(R_7)(CH_2)_qC(NR_{14}R_{15}) = NR_{16}$ ;

- ou bien R, lié à l'atome de carbone en position 5 du radical naphtyle, forme un groupe $-CON(R_{13})CO-$
- p = 2 à 6 ;
- n = 1 à 6 ;
- q = 1 à 5 ;
- $R_1$ et $R_2$, représentent chacun indépendamment un hydrogène ou un $(C_1-C_4)$alkyle ; ou $R_1$ et $R_2$ ensemble avec l'atome d'azote auquel ils sont liés représentent un hétérocycle choisi parmi : la pyrrolidine, la pipéridine, la pipérazine substituée en position -4- par $R_7$, la morpholine ou la thiomorpholine ;
- $R_3$ représente l'hydrogène; un $(C_1-C_8)$alkyle ; un $(C_3-C_8)$cycloalkyle ; un phényle ; un pipéridyle ;
- $R_4$ et $R_5$ représentent chacun indépendamment un hydrogène ou un $(C_1-C_4)$alkyle ;
- $R_6$ représente un $(C_1-C_4)$alkyle ;
- $R_7$ représente un hydrogène ou un $(C_1-C_4)$alkyle ;
- $R_8$ représente un $(C_1-C_4)$alkyle ;
- $R_9$ et $R_{10}$ représentent chacun indépendamment un hydrogène ou un $(C_1-C_4)$alkyle ; $R_{10}$ peut de plus représenter un groupe $-(CH_2)_nNR_1R_2$ ;
- ou $R_9$ et $R_{10}$ ensemble avec l'atome d'azote auquel ils sont liés représentent un hétérocycle choisi parmi : la pyrrolidine, la pipéridine, la pipérazine substituée en position 4 par $R_7$, la morpholine ou la thiomorpholine;
- $R_{11}$ et $R_{12}$ représentent chacun indépendamment un hydrogène ou un $(C_1-C_4)$alkyle ; ou $R_{11}$ et $R_{12}$ ensemble avec l'atome d'azote auquel ils sont liés représentent la pyrrolidine ou la pipéridine ;
- $R_{13}$ représente un hydrogène ; un groupe $-(CH_2)_nNR_1R_2$ ; un groupe $-NHCOR_3$ ;
- $R_{14}$ et $R_{15}$ représentent chacun indépendamment un hydrogène ou un $(C_1-C_4)$alkyle ;
- $R_{16}$ représente un hydrogène ; $R_{16}$ peut de plus représenter un $(C_1-C_4)$alkyle lorsque $R_{14}$ représente un hydrogène et $R_{15}$ représente un $(C_1-C_4)$alkyle ;
- ou $R_{14}$ et $R_{16}$ ensemble représentent un groupe éthylène ou un groupe triméthylène et $R_{15}$ représente un hydrogène ou un $(C_1-C_4)$alkyle ;
- T représente l'hydrogène ; un $(C_1-C_4)$alkyle ; un allyle ; un $(C_3-C_8)$cycloalkyle ; un $(C_3-C_8)$cycloalkylméthyle ; un méthoxyéthyle ;
- le groupe -NH-AA(OH) représente le résidu d'un acide aminé :

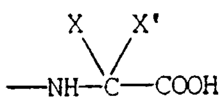

où X est l'hydrogène et X' est l'hydrogène, un $(C_1-C_5)$alkyle ou un radical carbocyclique non aromatique en $C_3-C_{15}$ ; ou bien X et X', ensemble avec l'atome de carbone auquel ils sont liés, forment un carbocycle non aromatique en $C_3-C_{15}$ ; et ses sels.

**2.** Composé selon la revendication 1 de formule I dans laquelle :

- R représente un groupe aminocarbonyle ; aminocarbonylméthyle ; acétamido ; N-(3-N',N'-diméthylaminopropyl)aminosulfonyle ; N-méthyl-N-(3-N',N'-diméthylaminopropyl)aminosulfonyle carbamoylméthyloxy ; N-(3-N',N'-diméthylaminopropyl)aminocarbonyle ; N-(2-N',N'-diméthylaminoéthyl)aminocarbonyle ; N-méthyl-N-(3-N',N'-diméthylaminopropyl)aminocarbonyle ; N-méthyl-N-(2-N',N'-diméthylaminoéthyl)aminocarbonyle ; N-méthylN-[2-$N^1$-méthyl-$N^2$-méthylamidino)éthyl]carbamoyle;
- T représente un groupe méthyle ou cyclopropylméthyle ; et
- le groupe -NH-AA-(OH) représente le résidu de l'acide 2-aminoadamantane-2-carboxylique ou (S)α-aminocyclohexaneacétique et ses sels.

**3.** Composé selon la revendication 1, de formule :

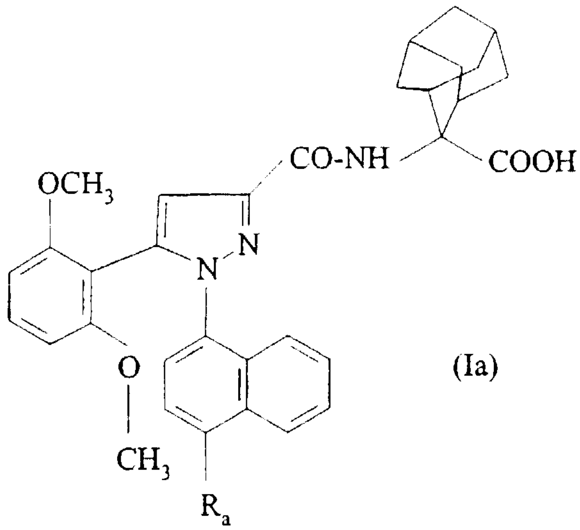

(Ia)

dans laquelle

- $R_a$ représente un groupe N-(3-N',N'-diméthylaminopropyl)aminocarbonyle ou N-(2-N',N'-diméthylaminoéthyl) aminocarbonyle ; et ses sels.

**4.** Procédé pour la préparation d'un composé selon la revendication 1 de formule I et de ses sels, caractérisé en ce que :

1) l'on traite un dérivé fonctionnel d'un acide 1-naphtylpyrazole-3-carboxylique de formule :

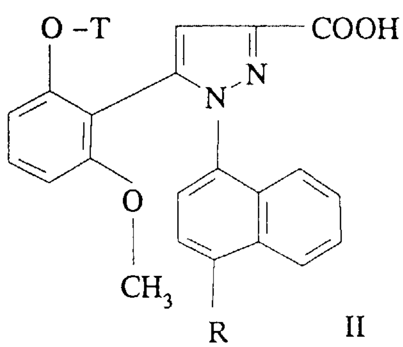

II

ou

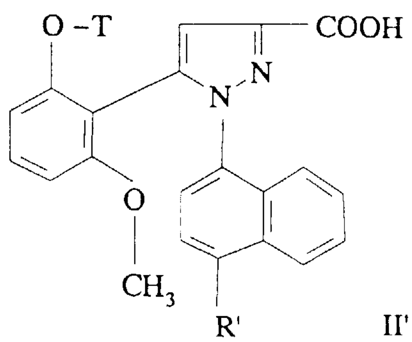

II'

dans laquelle T et R ont les significations données ci-dessus pour le composé de formule I et R' représente un précurseur de R choisi parmi les groupes nitro, amino, hydroxy, sulfo, chlorosulfonyle, carboxy, avec un acide aminé, éventuellement protégé par les groupements protecteurs habituels en synthèse peptidique, de formule :

H-HN-AA(OH) III.

dans laquelle -NH-AA(OH) est tel que défini ci-dessus pour le composé de formule I ;
2) le cas échéant, on soumet le composé ainsi obtenu de formule:

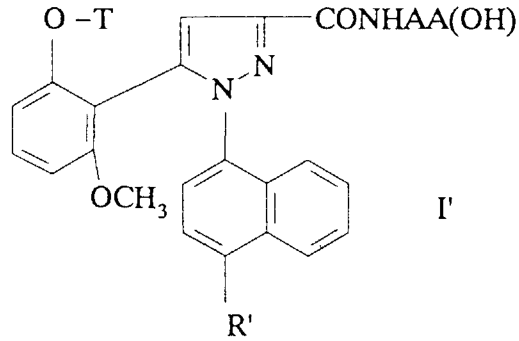

I'

à un traitement ultérieur approprié pour transformer le substituant R', précurseur de R, en le substituant R ;

3) éventuellement, on déprotège le composé ainsi obtenu à l'étape 1) ou à l'étape 2) pour conduire à l'acide libre de formule I correspondant ;

4) le cas échéant, on prépare un sel du composé I ainsi obtenu.

**5.** Acide de formule :

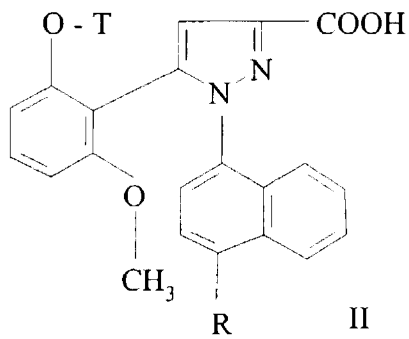

II

ou

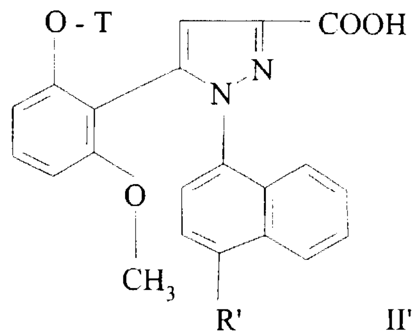

II'

dans laquelle T et R ont les définitions données dans la revendication 1 pour les composés I et R' représente un précurseur de R choisi parmi les groupes nitro, amino, hydroxy, sulfo, chlorosulfonyle, ainsi que le chlorure ou l'ester alkylique en $C_1$-$C_4$ dudit acide, ou l'anhydride mixte dudit acide avec le chloroformiate d'isobutyle ou d'éthyle.

**6.** Un composé de formule :

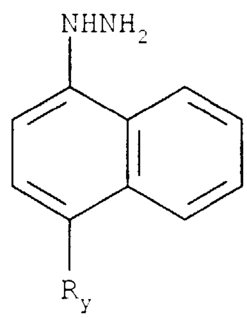

3'

dans laquelle :

- $R_y$ représente un groupe cyano ou carboxyméthyle et ses sels.

**7.** Composition pharmaceutique contenant en tant que principe actif un composé de formule I selon l'une quelconque

des revendications 1 à 3 ou un de ses sels éventuels pharmaceutiquement acceptables.

**8.** Composition pharmaceutique selon la revendication 7 sous forme d'unité de dosage.

**9.** Composition pharmaceutique selon la revendication 8, caractérisée en ce qu'elle contient de 0,5 à 250 mg de principe actif en mélange avec au moins un excipient pharmaceutique.

**Patentansprüche**

**1.** Verbindung der Formel:

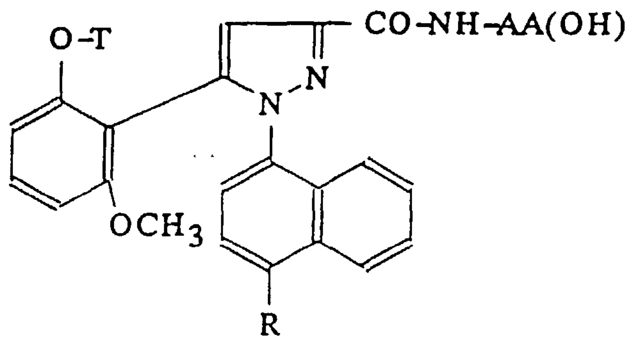

(I),

worin:

- R eine Gruppe darstellt, ausgewählt aus:
  -CN, $-C(NH_2) = N-OH$, $-C(NR_4R_5) = NR_6$,
  $-CONR_1R_2$, $-CON(R_7)(CH_2)_pNR_1R_2$,
  $-CON(R_7)(CH_2)_qCN$, $-CON(R_7)(CH_2)_qC(NR_{14}R_{15}) = N-R_{16}$,
  $-CH_2CN$, $-CH_2CONR_1R_2$, $-CH_2CON(R_7)(CH_2)_pNR_1R_2$,
  $-CH_2CON(R_7)(CH_2)_qCN$, $-CH_2COOR_7$,
  $-O(CH_2)_nNR_1R_2$, $-O(CH_2)_nCONR_1R_2$,
  $-O(CH_2)_nCOOR_7$, $-O(CH_2)_nSO_2NR_1R_2$,
  $-N(R7)COR_3$, $-N(R_7)CO(CH_2)_nNR_1R_2$,
  $-N(R_7)CO(CH_2)_nNHCOR_3$, $-N(R_7)SO_2R_8$,
  $-N(R_7)CONR_9R_{10}$, $-CH_2N(R_7)COR_3$,
  $-CH_2N(R_7)SO_2R_8$, $-CH_2CH_2NR_{11}R_{12}$,
  $-CH_2CH_2N(R_7)COR_3$, $-CH_2CH_2N(R_7)SO_2R_8$,
  $-SO_2NR_1R_2$, $-SO_2N(R_7)(CH_2)_nNR_1R_2$,
  $CH_2CON(R_7)(CH_2)_qC(NR_{14}R_{15}) = NR_{16}$
  $N(R_7)CO(CH_2)_qCN$, $-N(R_7)CO(CH_2)_qC(NR_{14}R_{15}) = NR_{16}$,
  $-SO_2N(R_7)(CH_2)_qCN$, $-SO_2N(R_7)(CH_2)_qC(NR_{14}R_{15}) = NR_{16}$;
- oder auch R, gebunden an das Kohlenstoffatom in Stellung 5 des Naphthylrests, eine Gruppe $-CON(R_{13})CO-$ bildet;
- p = 2 bis 6;
- n =1 bis 6;
- q = 1 bis 5;
- $R_1$, und $R_2$ jeweils unabhängig einen Wasserstoff oder ein $(C_1-C_4)$Alkyl darstellen; oder $R_1$, und $R_2$ gemeinsam mit dem Stickstoffatom, an das sie gebunden sind, einen Heterocyclus bilden, ausgewählt aus: Pyrrolidin, Piperidin, Piperazin substituiert in Stellung -4- durch $R_7$, Morpholin oder Thiomorpholin;
- $R_3$ Wasserstoff, ein $(C_1-C_8)$Alkyl, ein $(C_3-C_8)$Cycloalkyl, ein Phenyl, ein Piperidyl ist;
- $R_4$ und $R_5$ jeweils unabhängig einen Wasserstoff oder ein $(C_1-C_4)$Alkyl bedeuten;
- $R_6$ ein $(C_1-C_4)$Alkyl darstellt;
- $R_7$ ein Wasserstoff oder ein $(C_1-C_4)$Alkyl ist;
- $R_8$ ein $(C_1-C_4)$Alkyl bedeutet;
- $R_9$ und $R_{10}$ jeweils unabhängig einen Wasserstoff oder ein $(C_1-C_4)$Alkyl darstellen; wobei $R_{10}$ außerdem eine

Gruppe $-(CH_2)_nNR_1R_2$ sein kann;

- oder $R_9$ und $R_{10}$ gemeinsam mit dem Stickstoffatom, an das sie gebunden sind, einen Heterocyclus bilden, ausgewählt aus Pyrrolidin, Piperidin, Piperazin substituiert in Stellung 4 durch $R_7$, Morpholin oder Thiomorpholin;
- $R_{11}$ und $R_{12}$ jeweils unabhängig einen Wasserstoff oder ein $(C_1-C_4)$Alkyl darstellen; oder $R_{11}$ und $R_{12}$ gemeinsam mit dem Stickstoffatom, an das sie gebunden sind, Pyrrolidin oder Piperidin bedeuten;
- $R_{13}$ ein Wasserstoff, eine Gruppe $-(CH_2)_nNR_1R_2$, eine Gruppe $NHCOR_3$ ist;
- $R_{14}$ und $R_{15}$ jeweils unabhängig einen Wasserstoff oder ein $(C_1-C_4)$Alkyl darstellen;
- $R_{16}$ einen Wasserstoff bedeutet; wobei $R_{16}$ außerdem ein $(C_1-C_4)$Alkyl sein kann, wenn $R_{14}$ einen Wasserstoff darstellt, und $R_{15}$ ein $(C_1-C_4)$Alkyl ist;
- oder $R_{14}$ und $R_{16}$ gemeinsam eine Ethylen-Gruppe oder eine Trimethylen-Gruppe bedeuten, und $R_{15}$ einen Wasserstoff oder ein $(C_1-C_4)$Alkyl darstellt;
- T Wasserstoff, ein $(C_1-C_4)$Alkyl, ein Allyl, ein $(C_3-C_8)$Cycloalkyl, ein $(C_3-C_8)$Cycloalkylmethyl, ein Methoxyethyl ist;
- die Gruppe -NH-AA(OH) den Aminosäurerest bedeutet:

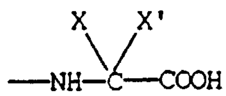

worin X Wasserstoff darstellt, und X' Wasserstoff, ein $(C_1-C_5)$Alkyl oder ein nicht aromatischer $C_3$-$C_{15}$-Carbocydusrest

ist; oder auch X und X' gemeinsam mit dem Kohlenstoffatom, an das sie gebunden sind, einen nicht aromatischen $C_3$-$C_{15}$-Carbocyclus bilden;

und ihre Salze.

**2.** Verbindung nach Anspruch 1 der Formel (I), worin:

- R eine Gruppe Aminocarbonyl, Aminocarbonylmethyl, Acetamido, N-(3-N',N'-Dimethylaminopropyl)-aminosulfonyl, N-Methyl-N-(3-N',N'-dimethylaminopropyl)-aminosulfonyl, Carbamoylmethyloxy, N-(3-N',N'-Dimethylaminopropyl)-aminocarbonyl; N-(2-N',N'-Dimethylaminoethyl)-aminocarbonyl, N-Methyl-N-(3-N',N'-dimethylaminopropyl)-aminocarbonyl, N-Methyl-N-(2-N',N'-dimethylaminoethyl)-aminocarbonyl, N-Methyl-N-[2-$N^1$-methyl-$N^2$-methylamidino)-ethyl]-carbamoyl bedeutet;
- T eine Methyl- oder Cyclopropylmethyl-Gruppe darstellt; und
- die Gruppe -NH-AA-(OH) der Rest von 2-Aminoadamantan-2-carbon- oder (S)α-Aminocyclohexanessigsäure ist;

und ihre Salze.

**3.** Verbindung der Formel:

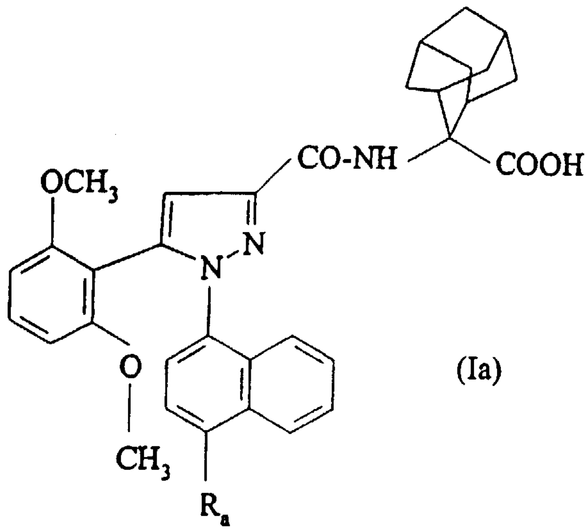

(Ia)

worin:

- $R_a$ eine Gruppe N-(3-N',N'-Dimethylaminopropyl)-aminocarbonyl oder N-(2-N',N'-Dimethylaminoethyl)-aminocarbonyl bedeutet;

und ihre Salze.

**4.** Verfahren zur Herstellung einer Verbindung nach Anspruch 1 der Formel (I) und ihrer Salze, dadurch gekennzeichnet, daß:

1) ein funktionelles Derivat von 1-Naphthylpyrazol-3-carbonsäure der Formel:

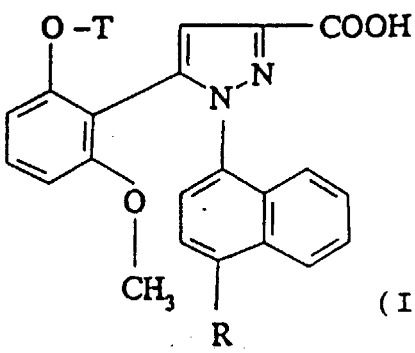

(II)

oder

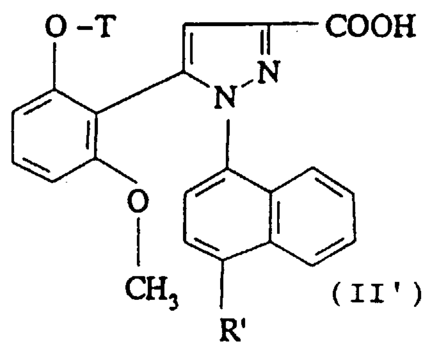

(II'),

worin T und R die oben für die Verbindung der Formel (I) angegebenen Bedeutungen haben, und R' einen Vorläufer von R darstellt, ausgewählt aus den Gruppen Nitro, Amino, Hydroxy, Sulfo, Chlorsulfonyl, Carboxy; mit einer Aminosäure, gegebenenfalls geschützt durch in der Peptidsynthese übliche Schutzgruppen der Formel:

H-HN-AA(OH) (III),

worin -NH-AA(OH) wie oben für die Verbindung der Formel (I) definiert ist; behandelt wird;
2) gegebenenfalls die so erhaltene Verbindung der Formel:

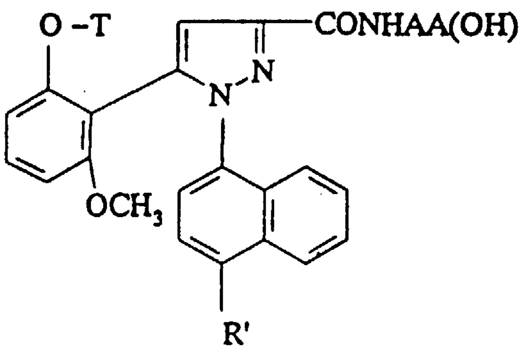

(I'),

einer geeigneten nachfolgenden Behandlung unterworfen wird, um den Substituenten R', einen Vorläufer von R, in den Substituenten R zu transformieren;

3) gegebenenfalls die so in Schritt 1) oder in Schritt 2) erhaltene Verbindung entschützt wird, um die entsprechende freie Säure der Formel (I) zu ergeben;

4) gegebenenfalls ein Salze der so erhaltenen Verbindung (I) hergestellt wird.

**5.** Säure der Formel:

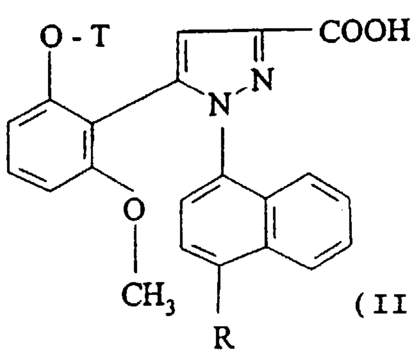

(II)

oder

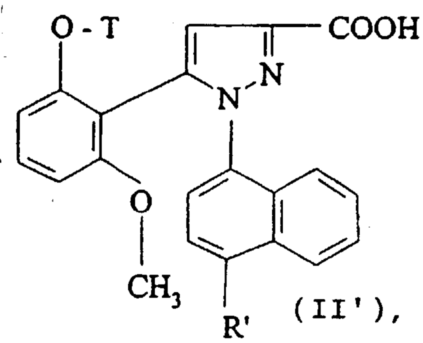

(II'),

worin T und R die in Anspruch 1 für die Verbindungen (I) angegebenen Bedeutungen haben, und R' einen Vorläufer von R darstellt, ausgewählt aus den Gruppen Nitro, Amino, Hydroxy, Sulfo, Chlorsulfonyl, wie das Chorid oder der $C_1$-$C_4$-Alkylester der genannten Säure, oder das gemischte Anhydrid der genannten Säure mit Isobutyl- oder Ethylchloroformiat.

**6.** Verbindung der Formel:

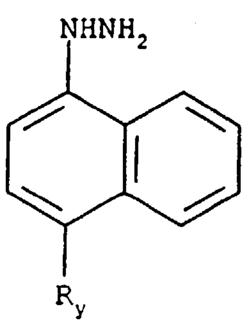

(3'),

worin:

- $R_y$ eine Cyano- oder Carboxymethyl-Gruppe bedeutet; und ihre Salze.

7. Pharmazeutische Zusammensetzung, welche als aktiven Bestandteil eine Verbindung der Formel (I) nach einem der Ansprüche 1 bis 3 oder eines ihrer gegebenenfalls pharmazeutisch annehmbaren Salze enthält.

8. Pharmazeutische Zusammensetzung nach Anspruch 7 in Einheitsdosierungsform.

9. Pharmazeutische Zusammensetzung nach Anspruch 8, dadurch gekennzeichnet, daß sie 0,5 bis 250 mg aktiven Bestandteil in Mischung mit zumindest einem pharmazeutischen Exzipienten enthält.

## Claims

1. A compound of formula:

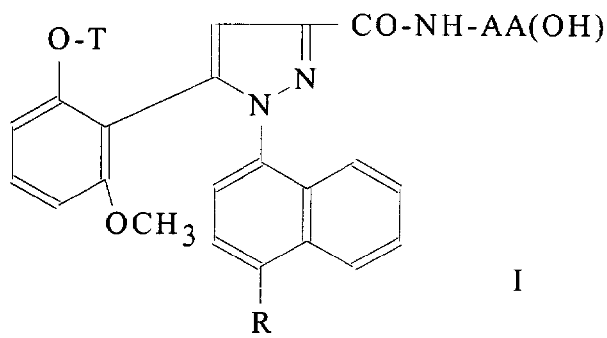

I

in which :

- R represents a group selected from :
  -CN, $-C(NH_2)$ N-OH, $-C(NR_4R_5) = NR_6$,
  $-CONR_1R_2$, $CON(R_7)(CH_2)_pNR_1R_2$,
  $-CON(R_7)(CH_2)_qCN$, $-CON(R_7)(CH_2)_qC(NR_{14}R_{15}) = N-R_{16}$,
  $-CH_2CN$, $-CH_2CONR_1R_2$, $-CH_2CON(R_7)(CH_2)_pNR_1R_2$,
  $CH_2CON(R_7)(CH_2)_qCN$, $-CH_2COOR_7$,
  $O(CH_2)_nNR_1R_2$, $-O(CH_2)_nCONR_1R_2$,
  $-O(CH_2)_nCOOR_7$, $-O(CH_2)_nSO_2NR_1R_2$,
  -N(R7)$COR_3$, $-N(R_7)CO(CH_2)_nNR_1R_2$,
  $-N(R_7)CO(CH_2)_nNHCOR_3$, $-N(R_7)SO_2R_8$,
  $-N(R_7)CONR_9R_{10}$, $-CH_2N(R_7)COR_3$,
  $-CH_2N(R_7)SO_2R_8$, $-CH_2CH_2NR_{11}R_{12}$,
  $-CH_2CH_2N(R_7)COR_3$, $-CH_2CH_2N(R_7)SO_2R_8$,
  $-SO_2NR_1R_2$, $-SO_2N(R_7)(CH_2)_nNR_1R_2$,
  $-CH_2CON(R_7)(CH_2)_qC(NR_{14}R_{15})= NR_{16}$
  $-N(R_7)CO(CH_2)_qCN$, $-N(R_7)CO(CH_2)_qC(NR_{14}R_{15}) = NR_{16}$,
  $SO_2N(R_7)(CH_2)_qCN$ $SO_2N(R_7)(CH_2)_qC(NR_{14}R_{15}) = NR_{16}$ ;
- or even R, bound to the carbon atom in position 5 of the naphthyl radical, forms a $-CON(R_{13})CO-$ group
- p = 2 to 6 ;
- n = 1 to 6 ;
- q = 1 to 5 ;
- $R_1$ and $R_2$ each independently represent a hydrogen or a $(C_1-C_4)$alkyl ; or $R_1$ and $R_2$ together with the nitrogen atom to which they are bound represent a heterocycle selected from : pyrrolidine, piperidine, piperazine substituted in position -4- with $R_7$, morpholine, or thiomorpholine;
- $R_3$ represents hydrogen ; a $(C_1-C_8)$alkyl ; a $(C_3-C_8)$cycloalkyl ; a phenyl ; or a piperidyl ;
- $R_4$ and $R_5$ each independently represent a hydrogen or a $(C_1-C_4)$alkyl ;
- $R_6$ represents a $(C_1-C_4)$alkyl ;
- $R_7$ represents a hydrogen or a $(C_1-C_4)$alkyl ;
- $R_8$ represents a $(C_1-C_4)$alkyl ;
- $R_9$ and $R_{10}$ each independently represent a hydrogen or a $(C_1-C_4)$alkyl ; $R_{10}$ can also represent a -

$(CH_2)_nNR_1R_2$ group ;

- or $R_9$ and $R_{10}$ together with the nitrogen atom to which they are bound represent a heterocycle selected from : pyrrolidine, piperidine, piperazine substituted in position 4 with $R_7$, morpholine or thiomorpholine :
- $R_{11}$ and $R_{12}$ each independently represent a hydrogen or a $(C_1-C_4)$alkyl ; or $R_{11}$ and $R_{12}$ together with the nitrogen atom to which they are bound represent pyrrolidine or piperidine ;
- $R_{13}$ represents a hydrogen ; a $-(CH_2)_nNR_1R_2$ group ; or an $-NHCOR_3$ group ;
- $R_{14}$ and $R_{15}$ each independently represent a hydrogen or a $(C_1-C_4)$alkyl ;
- $R_{16}$ represents a hydrogen ; $R_{16}$ can also represent a $(C_1-C_4)$alkyl when $R_{14}$ represents a hydrogen and $R_{15}$ represents a $(C_1-C_4)$alkyl ;
- or $R_{14}$ and $R_{16}$ together represent an ethylene group or a trimethylene group, and $R_{15}$ represents a hydrogen or a $(C_1-C_4)$alkyl ;
- T represents hydrogen ; a $(C_1-C_4)$alkyl ; an allyl ; a $(C_3-C_8)$cycloalkyl ; a $(C_3-C_8)$cycloalkylmethyl ; or methoxyethyl ;
- the -NH-AA(OH) group represents the amino acid residue :

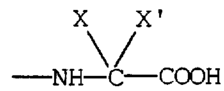

wherein X is hydrogen and X' is hydrogen, a $(C_1-C_5)$alkyl or a nonaromatic $C_3-C_{15}$ carbocyclic radical ; or even X and X', together with the carbon atom to which they are bound, form a non-aromatic $C_3-C_{15}$ carbocycle; and its salts.

**2.** The compound according to claim 1 of formula 1 in which :

- R represents an aminocarbonyl ; aminocarbonylmethyl ; acetamido ; N-(3-N',N'-dimethylaminopropyl) aminosulphonyl ; N-methyl-N-(3-N',N'-dimethylaminopropyl)aminosulphonyl ; carbamoylmethyloxy ; N-(3-N', N'-dimethylaminopropyl)aminocarbonyl ; N-(2-N',N'-dimethylaminoethyl)aminocarbonyl ; N-methyl-N-(3-N', N'-dimethylaminopropyl)aminocarbonyl; N-methyl-N-(2-N',N'dimethylaminoethyl)aminocarbonyl ; or N-methyl-N-[2-$N^1$-methyl-$N^2$-methylamidino)ethyl]carbamoyl group ;
- T represents a methyl or cyclopropylmethyl group ; and
- the -NH-AA-(OH) group represents a 2-aminoadamantane-2-carboxylic or (S)α-aminocyclohexaneacetic acid residue ; and its salts.

**3.** The compound according to claim 1, of formula :

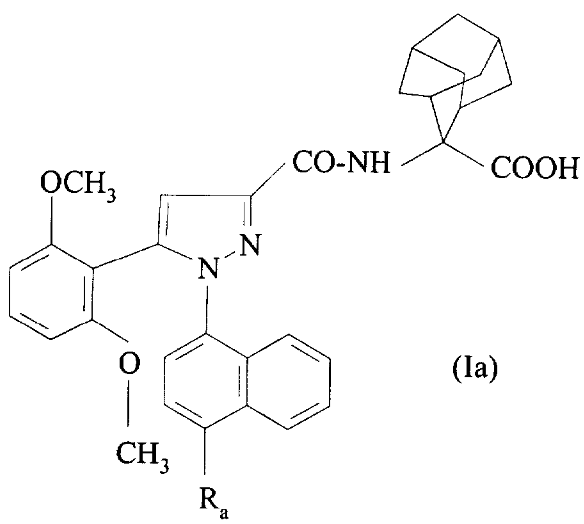

(Ia)

in which

- $R_a$ represents an N-(3-N',N'-dimethylaminopropyl)aminocarbonyl or N-(2-N',N'-dimethylaminoethyl)aminocarbonyl group ;

and its salts.

**4.** A method for the preparation of a compound according to claim 1 of formula I and of its salts, characterised in that :

1) a functional derivative of a 1-naphthylpyrazole-3-carboxylic acid of formula:

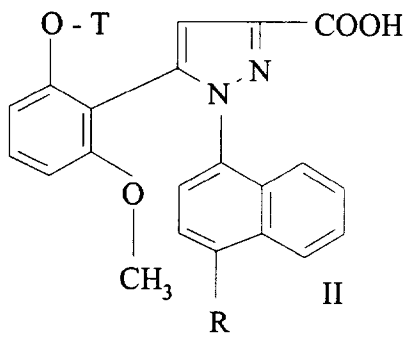

II

or

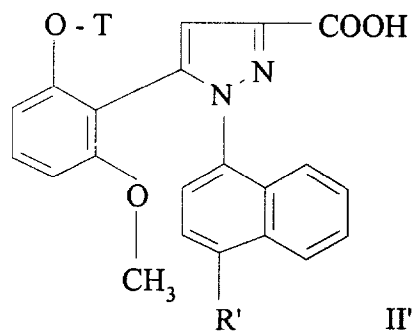

II'

in which T and R have the meanings given above for the compound of formula I and R' represents a precursor of R selected from nitro, amino, hydroxy, sulpho, chlorosulphonyl, and carboxy groups, is treated with an amino acid, optionally protected with protecting groups usual in peptide synthesis, of formula :

H-HN-AA(OH) III

in which -NH-AA(OH) is as defined above for the compound of formula I ;
2) if necessary, the resulting compound of formula :

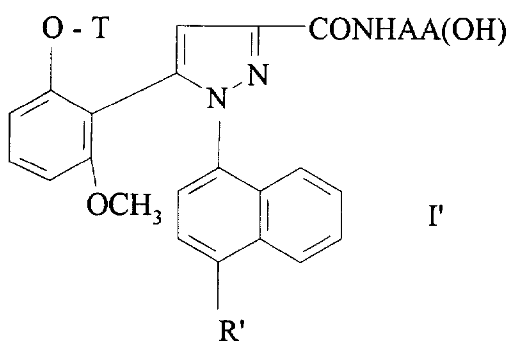

I'

is subjected to a further treatment which is suitable for converting the $R^1$ substituent, precursor of R, into the R substituent ;
3) optionally, the compound thus obtained in step 1) or in step 2) is deprotected to lead to the corresponding free acid of formula I ;
4) if necessary, a salt of compound I thus obtained is prepared.

**5.** An acid of formula :

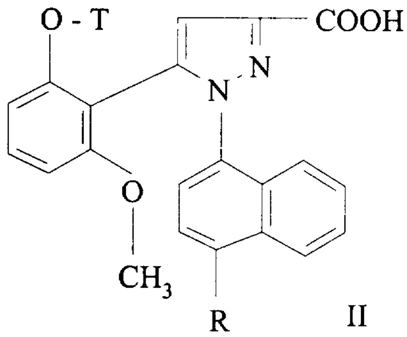

II

or

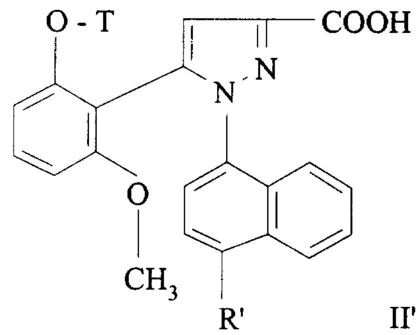

II'

in which T and R have the definitions given in claims 1 for compounds I and R' represents a precursor of R selected from nitro, amino, hydroxy, sulpho, and chlorosulphonyl groups, as well as chloride or $C_1$-$C_4$ alkyl ester of said acid, or mixed anhydride of said acid with ethyl or isobutyl chloroformate.

**6.** A compound of formula :

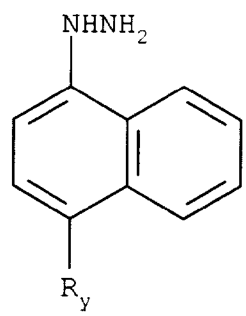

3'

in which :

- $R_y$ represents a cyano or carboxymethyl group ; and its salts.

**7.** A pharmaceutical composition containing as active principle a compound of formula I according to any one of claims 1 to 3, or one of its optional pharmaceutically acceptable salts.

**8.** The pharmaceutical composition according to claims 7 in the form of a unit dose.

**9.** The pharmaceutical composition according to claim 8, characterised in that it contains 0.5 to 250 mg of active principle in a mixture with at least one pharmaceutical excipient.